# EUROPÄISCHE PATENTSCHRIFT

(11) **EP 2 705 550 B1**
(45) Veröffentlichungstag und Bekanntmachung des Hinweises auf die Patenterteilung: **05.07.2017**
(21) Anmeldenummer: 12714970.6
(22) Anmeldetag: 12.04.2012
(51) Int. Cl.: H01L 51/54, C09B 19/00, C09B 21/00, C09B 57/00

(54) **VERBINDUNGEN FÜR ELEKTRONISCHE VORRICHTUNGEN**
COMPOUNDS FOR ELECTRONIC DEVICES
COMPOSÉS POUR DISPOSITIFS ÉLECTRONIQUES

(30) Priorität: 05.05.2011 EP 11003705
(43) Veröffentlichungstag der Anmeldung: 12.03.2014
(73) Patentinhaber: Merck Patent GmbH, 64293 Darmstadt (DE)
(72) Erfinder: PARHAM, Amir, Hossain, 65929 Frankfurt am Main (DE); BUESING, Arne, 65929 Frankfurt am Main (DE); PFLUMM, Christof, 64291 Darmstadt (DE); MUJICA-FERNAUD, Teresa, 64283 Darmstadt (DE); STOESSEL, Philipp, 60487 Frankfurt am Main (DE); EBERLE, Thomas, 76829 Landau (DE)
(86) Internationale Anmeldenummer: PCT/EP2012/001600
(87) Internationale Veröffentlichungsnummer: WO 2012/149999

(56) Entgegenhaltungen:
- WO-A1-2010/001982
- KR-A-020110 111 692
- KR-A-020110 113 469
- US-A1- 2008 018 238
- US-A1- 2010 171 417

## Beschreibung

Gegenstand der Erfindung ist eine Verbindung der Formel (I), die Verwendung dieser Verbindung in einer elektronischen Vorrichtung, sowie eine elektronische Vorrichtung, enthaltend eine oder mehrere Verbindungen der Formel (I). Weiterhin betrifft die Erfindung die Herstellung der Verbindung der Formel (I) sowie eine Formulierung enthaltend eine oder mehrere Verbindungen der Formel (I).

Die Entwicklung von neuartigen funktionellen Verbindungen zur Verwendung in elektronischen Vorrichtungen ist aktuell Gegenstand intensiver Forschung. Ziel ist hierbei die Entwicklung und Untersuchung von Verbindungen, welche bislang noch nicht in elektronischen Vorrichtungen eingesetzt wurden, sowie die Entwicklung von Verbindungen, welche ein verbessertes Eigenschaftsprofil der Vorrichtungen ermöglichen.

Unter dem Begriff elektronische Vorrichtung werden gemäß der vorliegenden Erfindung unter anderem organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs) verstanden.

Der Aufbau der oben genannten organischen Elektrolumineszenzvorrichtungen (OLEDs) ist dem Fachmann bekannt und unter anderem in US 4539507, US 5151629, EP 0676461 und WO 1998/27136 beschrieben.

Betreffend die Leistungsdaten der organischen Elektrolumineszenzvorrichtungen sind, insbesondere in Hinblick auf eine breite kommerzielle Verwendung, noch weitere Verbesserungen erforderlich. Von besonderer Bedeutung sind in diesem Zusammenhang die Lebensdauer, die Effizienz und die Betriebsspannung der organischen Elektrolumineszenzvorrichtungen sowie die realisierten Farbwerte. Insbesondere bei blau emittierenden Elektrolumineszenzvorrichtungen besteht Verbesserungspotential bezüglich der Lebensdauer der Vorrichtungen. Zudem ist es wünschenswert, dass die Verbindungen zur Verwendung als Funktionsmaterialien in elektronischen Vorrichtungen eine hohe thermische Stabilität und eine hohe Glasübergangstemperatur aufweisen und sich unzersetzt sublimieren lassen.

Auf dem Gebiet der elektronischen Vorrichtungen enthaltend organische Materialien besteht Bedarf an Matrixmaterialien, insbesondere an Matrixmaterialien für phosphoreszierende Emitter, die gleichzeitig zu guter Effizienz, hoher Lebensdauer und geringer Betriebsspannung der elektronischen Vorrichtungen führen. Gerade die Eigenschaften der Matrixmaterialien sind häufig limitierend für die Lebensdauer und die Effizienz der organischen Elektrolumineszenzvorrichtung. Bei Matrixmaterialien für phosphoreszierende Emitter ist es wünschenswert, dass diese ein hochliegendes T₁-Niveau (Triplettniveau) aufweisen. Besonders relevant ist dies bei Matrixmaterialien für blau emittierende phosphoreszierende Emitter.

Weiterhin ist die Bereitstellung neuer Elektronentransportmaterialien wünschenswert, da gerade auch die Eigenschaften des Elektronentransportmaterials einen wesentlichen Einfluss auf die oben genannten Eigenschaften der organischen Elektrolumineszenzvorrichtung ausüben. Insbesondere besteht Bedarf an Elektronentransportmaterialien, welche gleichzeitig zu guter Effizienz, hoher Lebensdauer und geringer Betriebsspannung führen.

Gemäß dem Stand der Technik werden häufig Carbazolderivate, z. B. Bis(carbazolyl)biphenyl, als Matrixmaterialien für phosphoreszierende Emitter verwendet. Ebenfalls werden in dieser Funktion Ketone (WO 2004/093207), Phosphinoxide, Sulfone (WO 2005/003253), Triazinverbindungen wie Triazinylspirobifluoren (vgl. WO 2010/015306) sowie Metallkomplexe, beispielsweise BAIq oder Bis[2-(2-benzothiazol)phenolat]-zink(II), verwendet.

In den Anmeldungen WO 2006/067976 und WO 2008/123189 werden Carbazolderivate, welche mit elektronenarmen Heterocyclen wie beispielsweise Triazin derivatisiert sind, zur Verwendung in elektronischen Vorrichtungen offenbart. Es besteht jedoch weiterhin Bedarf an neuen Verbindungen zur Verwendung als Funktionsmaterialien für elektronische Vorrichtungen. Insbesondere besteht Bedarf an Verbindungen zur Verwendung als Matrixmaterialien oder als Elektronentransportmaterialien in organischen Elektrolumineszenzvorrichtungen. Nochmals insbesondere besteht Bedarf an Verbindungen, mit denen eine Verbesserung der Leistungsdaten der elektronischen Vorrichtung erreicht werden kann.

Außerdem werden in den Anmeldungen US 2010/0171417, US 2011/0108820 und US 2008/0018238 Dihydroacridinderivate als Matrixmaterialien oder als Ladungstransportmaterialien in organischen Elektrolumineszenzvorrichtungen offenbart.

Der vorliegenden Erfindung liegt somit die technische Aufgabe zu Grunde, Verbindungen bereitzustellen, welche sich zur Verwendung in elektronischen Vorrichtungen wie beispielsweise OLEDs eignen, und welche insbesondere als Matrixmaterialien für phosphoreszierende Emitter und/oder als Elektronentransportmaterialien eingesetzt werden können.

Im Rahmen der vorliegenden Erfindung wurde nun gefunden, dass sich Verbindungen der unten angegebenen Formel (I) ausgezeichnet zur Verwendung in elektronischen Vorrichtungen eignen, besonders als Matrixmaterialien für phosphoreszierende Emitter und als Elektronentransportmaterialien. Diese Verbindungen weisen kennzeichnenderweise eine elektronenarme Gruppe A auf, welche über einen aromatischen Sechsring als Abstandsgruppe an ein Dihydroacridinderivat gebunden ist.

Gegenstand der Erfindung ist somit eine Verbindung einer Formel (I) 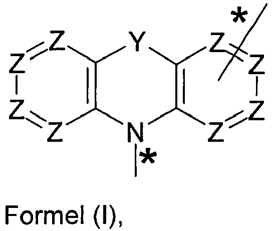 wobei über mindestens eine der mit * gekennzeichneten Bindungen eine Gruppe der Formel (II) gebunden ist 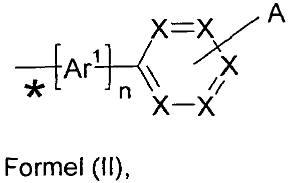 wobei * in Formel (II) wiederum die Bindung zur Einheit gemäß Formel (I) kennzeichnet, und
wobei in Formel (I) für den Fall, dass an der mit * gekennzeichneten Bindung keine Gruppe der Formel (II) gebunden ist, dort ein Rest R² gebunden ist,
und wobei für die auftretenden Symbole und Indices gilt:
- A: ist bei jedem Auftreten gleich oder verschieden
- eine Heteroarylgruppe mit 5 bis 20 aromatischen Ringatomen, welche mit einem oder mehreren Resten R¹ substituiert sein kann und welche mindestens einen heteroaromatischen Fünfring mit zwei oder mehr Heteroatomen ausgewählt aus N, O und S enthält oder mindestens einen heteroaromatischen Sechsring mit einem oder mehr Heteroatomen ausgewählt aus N, O und S enthält, oder
- eine Ketogruppe, welche mit einem oder mehreren Resten R¹ substituiert sein kann, oder
- eine Phosphoroxidgruppe, welche mit einem oder mehreren Resten R¹ substituiert sein kann, oder
- eine Schwefeloxidgruppe, welche mit einem oder mehreren Resten R¹ substituiert sein kann;
- Y: ist eine divalente Gruppe ausgewählt aus C(R²)₂, Si(R²)₂, NR² und O;
- X: ist bei jedem Auftreten gleich oder verschieden CR¹ oder N, bzw. ist C mit daran gebundener Gruppe A;
- Z: ist bei jedem Auftreten gleich oder verschieden CR² oder N;
- Ar¹: ist ein aromatisches oder heteroaromatisches Ringsystem mit 5 bis 30 aromatischen Ringatomen, welches mit einem oder mehreren Resten R¹ substituiert sein kann;
- R¹: ist bei jedem Auftreten gleich oder verschieden H, D, F, Cl, Br, I, B(OR³)₂, CHO, C(=O)R³, CR³=C(R³)₂, CN, C(=O)OR³, C(=O)N(R³)₂, Si(R³)₃, NO₂, P(=O)(R³)₂, OSO₂R³, OR³, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R³ substituiert sein kann, oder eine Aryloxygruppe mit 6 bis 30 aromatischen Ringatomen, die durch einen oder mehrere Reste R³ substituiert sein kann, wobei zwei oder mehr Reste R¹ miteinander verknüpft sein können und einen Ring bilden können;
- R²: ist bei jedem Auftreten gleich oder verschieden H, D, F, Cl, Br, I, B(OR³)₂, CHO, C(=O)R³, CR³=C(R³)², CN, C(=O)OR³, C(=O)N(R³)₂, Si(R³)₃, N(R³)₂, NO₂, P(=O)(R³)₂, OSO₂R³, OR³, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 60 aromatischen Ringatomen, die durch einen oder mehrere Reste R³ substituiert sein kann, wobei zwei oder mehr Reste R² miteinander verknüpft sein können und einen Ring bilden können;
- R³: ist bei jedem Auftreten gleich oder verschieden H, D, F, Cl, Br, I, B(OR⁴)₂, CHO, C(=O)R⁴, CR⁴=C(R⁴)₂, CN, C(=O)OR⁴, C(=O)N(R⁴)₂, Si(R⁴)₃, N(R⁴)₂, NO₂, P(=O)(R⁴)₂, OSO₂R⁴, OR⁴, S(=O)R⁴, S(=O)₂R⁴, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=S, C=Se, C=NR⁴, -C(=O)O-, -C(=O)NR⁴-, NR⁴, P(=O)(R⁴), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R⁴ substituiert sein kann, oder eine Aryloxy- oder Heteroaryloxygruppe mit 5 bis 60 aromatischen Ringatomen, die durch einen oder mehrere Reste R⁴ substituiert sein kann, wobei zwei oder mehr Reste R³ miteinander verknüpft sein können und einen Ring bilden können;
- R⁴: ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁴ miteinander verknüpft sein und einen Ring bilden;
- n: ist bei jedem Auftreten gleich oder verschieden 0, 1, 2 oder 3;
wobei Reste R² als Bestandteile von Gruppen Z keine an das Ringsystem von Formel (I) ankondensierten Ringe bilden dürfen; und
wobei weiterhin die Gruppe A in meta- oder in ortho-Position an den aromatischen Sechsring gebunden ist, wenn die Gruppe der Formel (II) an das Stickstoffatom in Formel (I) gebunden ist;
und wobei weiterhin die folgenden Verbindungen ausgenommen sind:

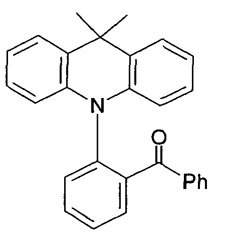

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung weitere Bevorzugungen angegeben, beispielsweise bezüglich der Zahl der aromatischen Ringatome oder der enthaltenden Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen. Es soll an dieser Stelle darauf hingewiesen werden, dass gemäß dieser Definition unter einer Heteroarylgruppe insbesondere keine Gruppe verstanden wird, bei der ein oder mehrere nichtkonjugierte (nicht heteroaromatische) Ringe, wie beispielsweise ein Piperidinring, mit aromatischen oder heteroaromatischen Ringen kondensiert sind.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter einer Ketogruppe wird im Rahmen der vorliegenden Erfindung eine Gruppe der folgenden Formel (K) verstanden: 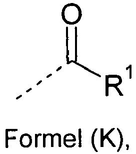 wobei R¹ wie oben definiert ist und die gestrichelte Bindung die Bindung an die Einheit von Formel (II) darstellt. Bevorzugt stellt R¹ in Formel (K) ein aromatisches Ringsystem mit 6 bis 30 aromatischen Ringatomen, welches mit einem oder mehreren Resten R³ substituiert sein kann, oder eine Alkylgruppe mit 1 bis 20 C-Atomen dar, bei der eine oder mehrere CH₂-Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und bei der ein oder mehrere H-Atome durch D, F, Cl, Br, I, CN oder NO₂ ersetzt sein können. Besonders bevorzugt stellt R¹ in Formel (K) ein aromatisches Ringsystem mit 6 bis 24 aromatischen Ringatomen dar, welches mit einem oder mehreren Resten R³ substituiert sein kann.

Unter einer Phosphoroxidgruppe wird im Rahmen der vorliegenden Erfindung eine Gruppe der folgenden Formel (P) verstanden: 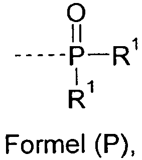 wobei R¹ wie oben definiert ist und die gestrichelte Bindung die Bindung an die Einheit von Formel (II) darstellt. Bevorzugt stellt R¹ in Formel (P) bei jedem Auftreten gleich oder verschieden ein aromatisches Ringsystem mit 6 bis 30 aromatischen Ringatomen dar, welches mit einem oder mehreren Resten R³ substituiert sein kann, oder eine Alkylgruppe mit 1 bis 20 C-Atomen dar, bei der eine oder mehrere CH₂-Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und bei der ein oder mehrere H-Atome durch D, F, Cl, Br, I, CN oder NO₂ ersetzt sein können. Besonders bevorzugt stellt R¹ in Formel (P) ein aromatisches Ringsystem mit 6 bis 24 aromatischen Ringatomen dar, welches mit einem oder mehreren Resten R³ substituiert sein kann.

Unter einer Schwefeloxidgruppe wird im Rahmen der vorliegenden Erfindung eine Gruppe der folgenden Formel (S) verstanden: 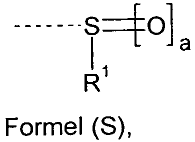 wobei R¹ wie oben definiert ist, der Index a gleich 1 oder 2 sein kann und die gestrichelte Bindung die Bindung an die Einheit von Formel (II) darstellt. Bevorzugt stellt R¹ in Formel (S) ein aromatisches Ringsystem mit 6 bis 30 aromatischen Ringatomen dar, welches mit einem oder mehreren Resten R³ substituiert sein kann, oder eine Alkylgruppe mit 1 bis 20 C-Atomen dar, bei der eine oder mehrere CH₂-Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und bei der ein oder mehrere H-Atome durch D, F, Cl, Br, I, CN oder NO₂ ersetzt sein können. Besonders bevorzugt stellt R¹ in Formel (S) ein aromatisches Ringsystem mit 6 bis 24 aromatischen Ringatomen dar, welches mit einem oder mehreren Resten R³ substituiert sein kann.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung verknüpft sind. Dies wird durch das folgende Schema verdeutlicht:

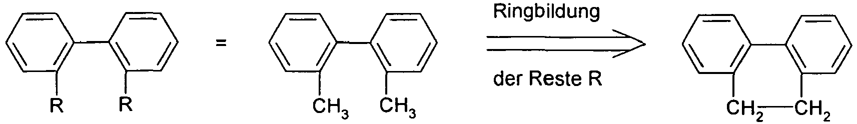

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

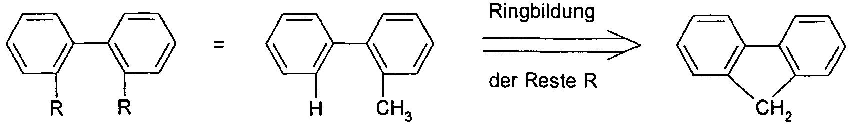

Unter der Formulierung, dass Reste R² als Bestandteile von Gruppen Z keine an das Ringsystem von Formel (I) ankondensierten Ringe bilden dürfen, wird im Rahmen der vorliegenden Erfindung insbesondere verstanden, dass das Ringsystem von Formel (I) nicht durch ankondensierte Heterocyclen, wie beispielsweise Pyrrol-, Indol- und Piperidinringe erweitert sein darf.

Gemäß einer bevorzugten Ausführungsform umfasst die erfindungsgemäße Verbindung zusätzlich zu der in Formel (I) gezeigten Arylaminogruppe keine weitere Arylaminogruppe. Besonders bevorzugt umfasst die erfindungsgemäße Verbindung zusätzlich zu der in Formel (I) gezeigten Aminogruppe keine weitere Aminogruppe.

Gemäß einer weiteren bevorzugten Ausführungsform hat n einen Wert von 0 oder 1, besonders bevorzugt von 0.

Weiterhin ist es bevorzugt, dass die Gruppe Ar¹ ein aromatisches Ringsystem mit 6 bis 18 aromatischen Ringatomen oder ein heteroaromatisches Ringsystem mit 5 bis 18 aromatischen Ringatomen darstellt, welche jeweils mit einem oder mehreren Resten R¹ substituiert sein können. Besonders bevorzugt stellt die Gruppe Ar¹ eine Arylengruppe mit 6 bis 14 aromatischen Ringatomen dar, welche mit einem oder mehreren Resten R¹ substituiert sein kann, ganz besonders bevorzugt Phenylen oder Naphthylen, welche jeweils mit einem oder mehreren Resten R¹ substituiert sein können.

Weiterhin ist es bevorzugt, dass die Gruppe Y gleich C(R²)₂.

Weiterhin ist es bevorzugt, dass keine, eine, zwei oder drei Gruppen X pro Gruppe der Formel (II) gleich N sind, wobei nicht mehr als zwei benachbarte Gruppen X gleichzeitig gleich N sind. Besonders bevorzugt ist nicht mehr als eine Gruppe X pro Gruppe der Formel (II) gleich N, ganz besonders bevorzugt ist keine Gruppe X gleich N.

Weiterhin ist es bevorzugt, dass keine, eine, zwei oder drei Gruppen Z pro aromatischem Sechsring gleich N sind, wobei nicht mehr als zwei benachbarte Gruppen Z gleichzeitig gleich N sind. Besonders bevorzugt ist nicht mehr als eine Gruppe Z pro aromatischem Sechsring gleich N, ganz besonders bevorzugt ist keine Gruppe Z gleich N.

Bevorzugt ist weiterhin R¹ bei jedem Auftreten gleich oder verschieden ausgewählt aus H, D, F, CN, Si(R³)₃ oder einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, wobei die oben genannten Gruppen jeweils mit einem oder mehreren Resten R³ substituiert sein können und wobei in den oben genannten Gruppen eine oder mehrere CH₂-Gruppen durch -C≡C-, -R³C=CR³-, Si(R³)₂, C=O, C=NR³, -NR³-, -O-, -S-, -C(=O)O- oder -C(=O)NR³- ersetzt sein können, oder ein aromatisches Ringsystem mit 6 bis 24 aromatischen Ringatomen, welches mit einem oder mehreren Resten R³ substituiert sein kann, wobei zwei oder mehr Reste R¹ miteinander verknüpft sein können und einen Ring bilden können.

Bevorzugt sind weiterhin diejenigen Gruppen R¹, welche nicht an eine Gruppe A binden, gleich H.

Gemäß einer bevorzugten Ausführungsform sind eine oder mehrere der Gruppen R¹ in Formel (II), welche Bestandteil einer Gruppe X sind oder an eine Gruppe Ar¹ binden, zusätzlich unter Ausbildung eines Rings an einen benachbarten Ring gebunden.

R² ist bevorzugt bei jedem Auftreten gleich oder verschieden ausgewählt aus H, D, F, CN, Si(R³)₃, N(R³)₂ oder einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, wobei die oben genannten Gruppen jeweils mit einem oder mehreren Resten R³ substituiert sein können und wobei in den oben genannten Gruppen eine oder mehrere CH₂-Gruppen durch -C≡C-, -R³C=CR³-, Si(R³)₂, C=O, C=NR³, -NR³-, -O-, -S-, -C(=O)O- oder -C(=O)NR³- ersetzt sein können, oder ein aromatisches oder heteroaromatisches Ringsystem mit 5 bis 24 aromatischen Ringatomen, das jeweils mit einem oder mehreren Resten R³ substituiert sein kann, wobei zwei oder mehr Reste R² miteinander verknüpft sein können und einen Ring bilden können.

R³ ist bevorzugt bei jedem Auftreten gleich oder verschieden ausgewählt aus H, D, F, CN, Si(R⁴)₃, N(R⁴)₂ oder einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, wobei die oben genannten Gruppen jeweils mit einem oder mehreren Resten R⁴ substituiert sein können und wobei in den oben genannten Gruppen eine oder mehrere CH₂-Gruppen durch -C≡C-, -R⁴C=CR⁴-, Si(R⁴)₂, C=O, C=NR⁴, -NR⁴-, -O-, -S-, -C(=O)O- oder -C(=O)NR⁴- ersetzt sein können, oder ein aromatisches oder heteroaromatisches Ringsystem mit 5 bis 20 aromatischen Ringatomen, das jeweils mit einem oder mehreren Resten R⁴ substituiert sein kann, wobei zwei oder mehr Reste R³ miteinander verknüpft sein können und einen Ring bilden können.

In einer bevorzugten Ausführungsform weist die Gruppe der Formel (II) eine Struktur der folgenden Formel (II-1) auf: 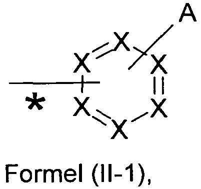 wobei A, R¹ und X wie oben definiert sind. Bevorzugt ist in diesem Fall die Gruppe A in meta- oder in ortho-Position am aromatischen Sechsring gebunden.

Besonders bevorzugte Ausführungsformen der Gruppen der Formel (II-1) stellen die folgenden Formeln (II-1-1) und (II-1-2) dar: 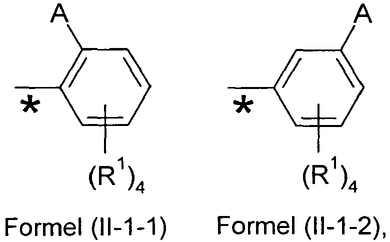 wobei A und R¹ wie oben definiert sind.

In einer weiteren bevorzugten Ausführungsform weist die Gruppe der Formel (II) eine Struktur der folgenden Formel (II-2) auf: 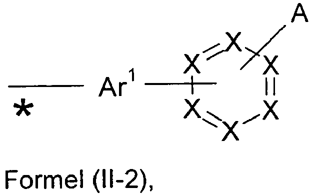 wobei A, Ar¹, R¹ und X wie oben definiert sind. Bevorzugt ist in diesem Fall die Gruppe A in meta- oder in ortho-Position am aromatischen Sechsring gebunden.

Besonders bevorzugte Ausführungsformen der Gruppen der Formel (II-2) stellen die folgenden Formeln (II-2-1) und (II-2-2) dar: 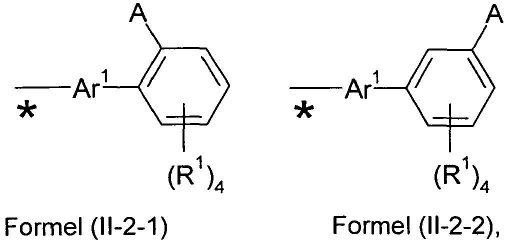 wobei A, Ar¹ und R¹ wie oben definiert sind.

In einer weiteren bevorzugten Ausführungsform weist die Gruppe der Formel (II) eine Struktur der folgenden Formel (II-3) auf: 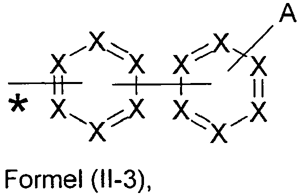 wobei A, R¹ und X wie oben definiert sind und wobei eine oder mehrere Gruppen R¹, welche an einen der Sechsringe binden, zusätzlich unter Ausbildung eines Rings an einen benachbarten Ring gebunden sein können. Bevorzugt ist in Formel (II-3) die Gruppe A in meta- oder in ortho-Position am aromatischen Sechsring gebunden.

Besonders bevorzugte Ausführungsformen der Gruppen der Formel (II-3) stellen die folgenden Formeln (II-3-1) bis (II-3-6) dar: 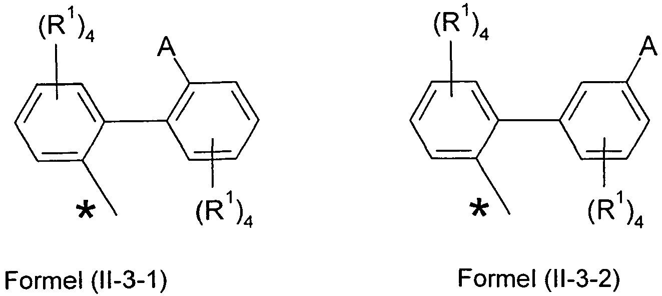 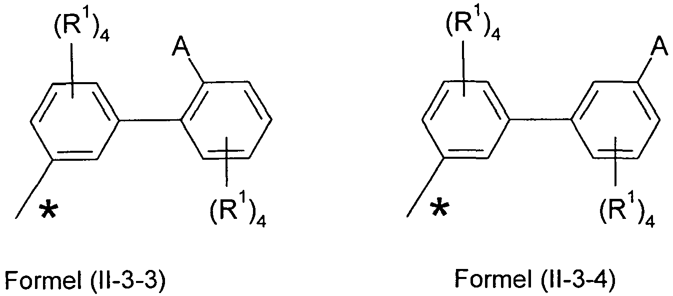 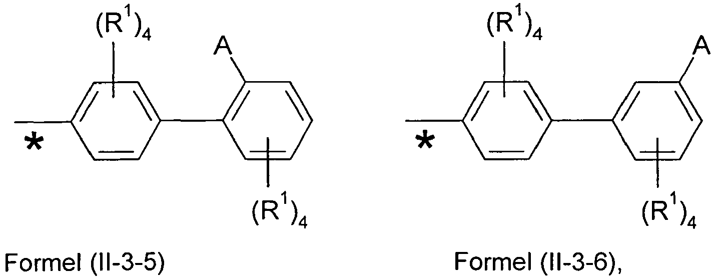 wobei A und R¹ wie oben definiert sind.

In einer weiteren bevorzugten Ausführungsform weist die Gruppe der Formel (II) eine Struktur der folgenden Formel (II-4) auf: 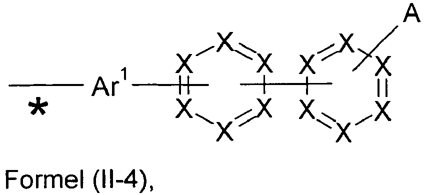 wobei A, Ar¹, R¹ und X wie oben definiert sind und wobei eine oder mehrere Gruppen R¹, welche an einen der Sechsringe binden, zusätzlich unter Ausbildung eines Rings an einen benachbarten Ring gebunden sein können. Bevorzugt ist in Formel (II-4) die Gruppe A in meta- oder in ortho-Position am aromatischen Sechsring gebunden.

Besonders bevorzugte Ausführungsformen der Gruppen der Formel (II-4) stellen die folgenden Formeln (II-4-1) bis (II-4-6) dar: 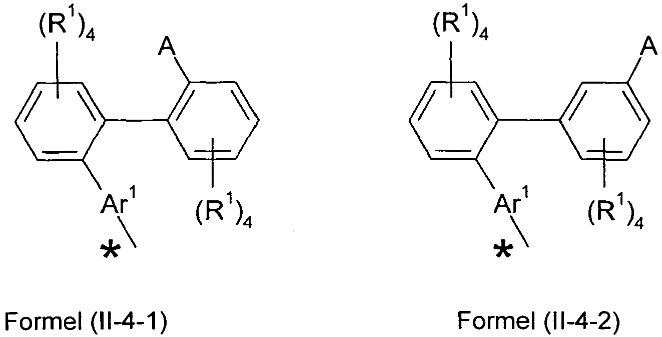 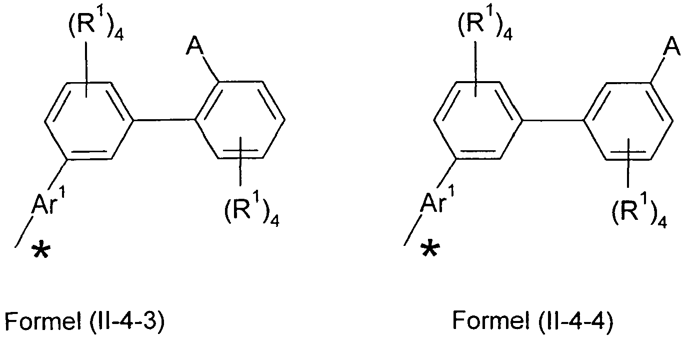 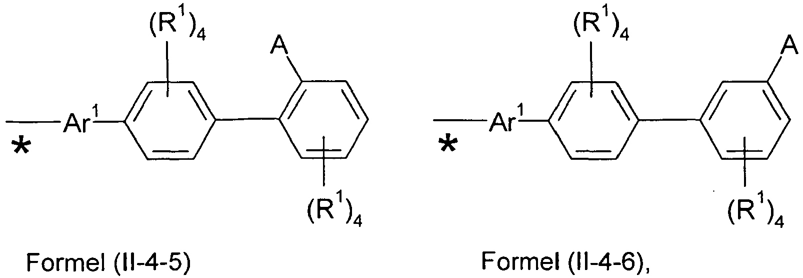 wobei A und Ar¹ und R¹ wie oben definiert sind.

In einer weiteren bevorzugten Ausführungsform weist die Gruppe der Formel (II) eine Struktur der folgenden Formel (II-5) auf: 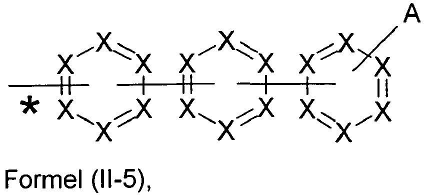 wobei A, R¹ und X wie oben definiert sind und wobei eine oder mehrere Gruppen R¹, welche an einen der Sechsringe binden, zusätzlich unter Ausbildung eines Rings an einen benachbarten Ring gebunden sein können. Bevorzugt ist in Formel (II-5) die Gruppe A in meta- oder in ortho-Position am aromatischen Sechsring gebunden.

Besonders bevorzugte Ausführungsformen der Gruppen der Formel (II-5) stellen die folgenden Formeln (II-5-1) bis (II-5-18) dar: 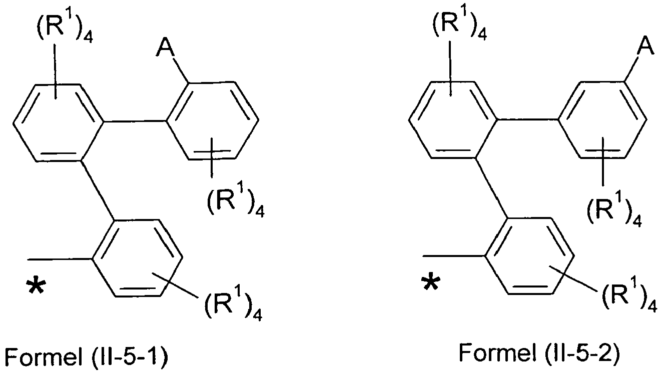 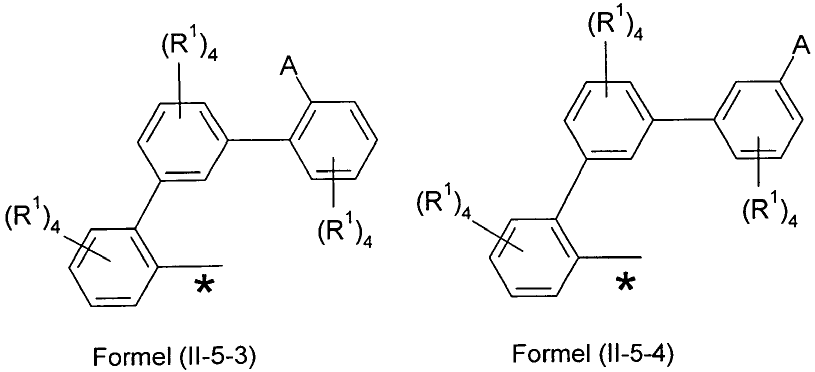 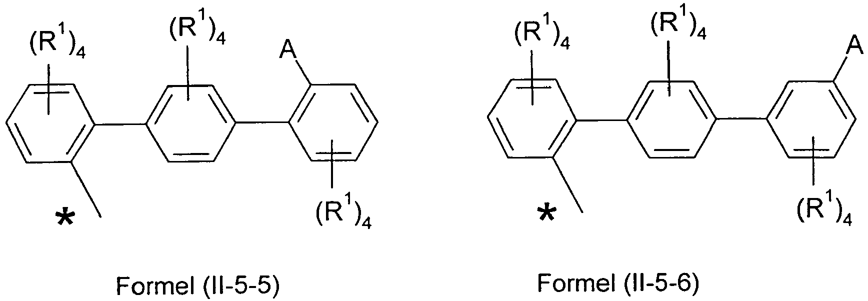 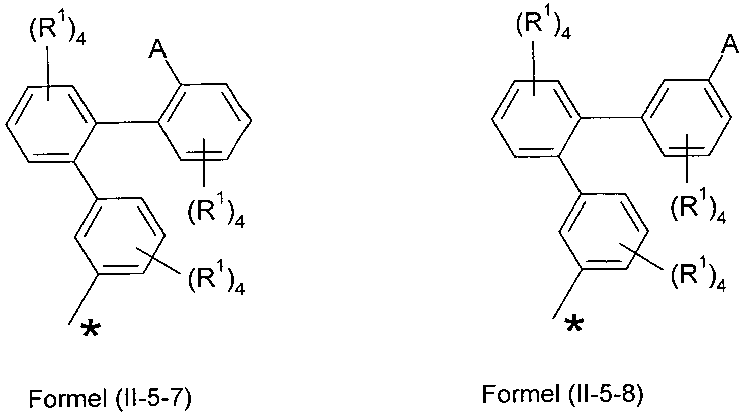 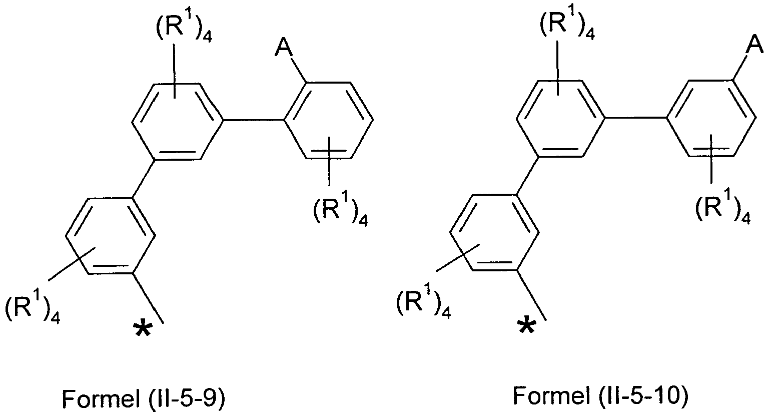 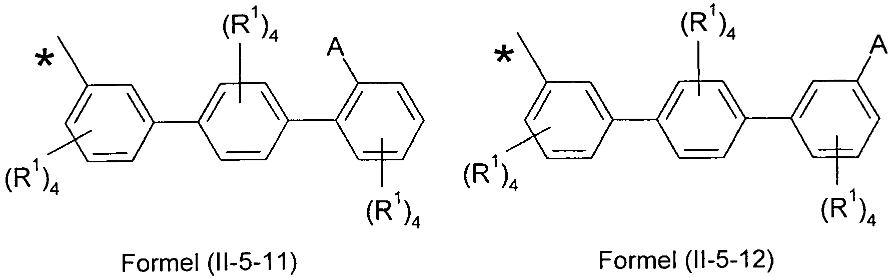 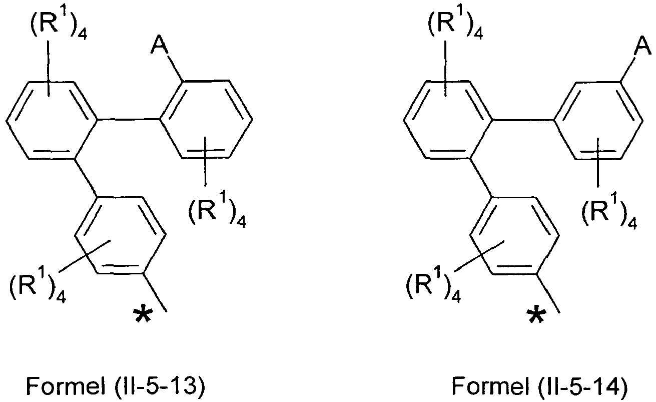 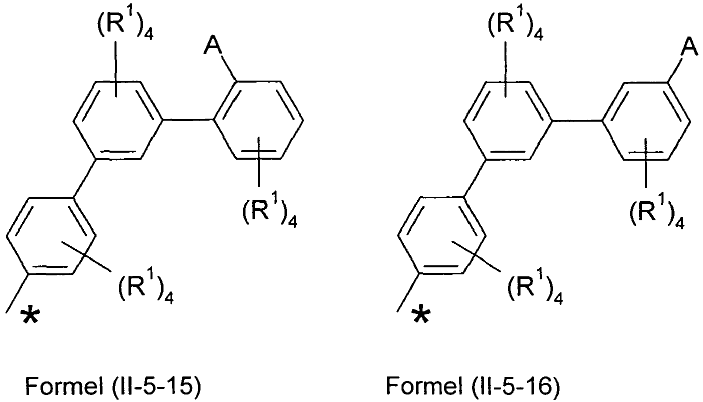 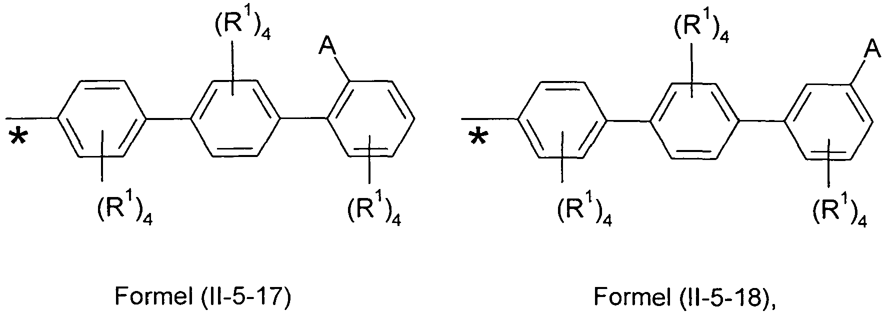 wobei A und R¹ wie oben definiert sind.

Die Gruppe A stellt bevorzugt eine Gruppe der oben definierten Formeln (K), (P) oder (S) oder eine Gruppe der Formel (A-1) bis (A-9) dar: 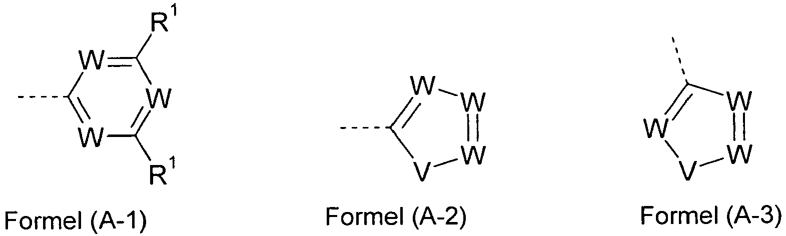 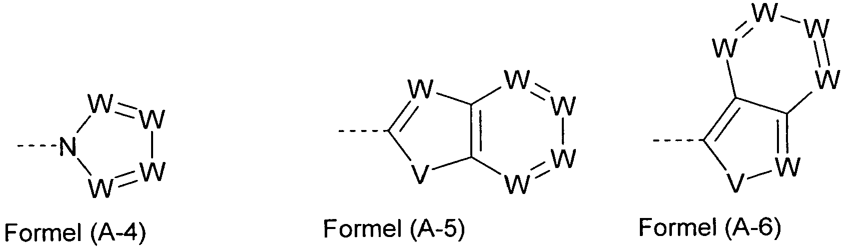 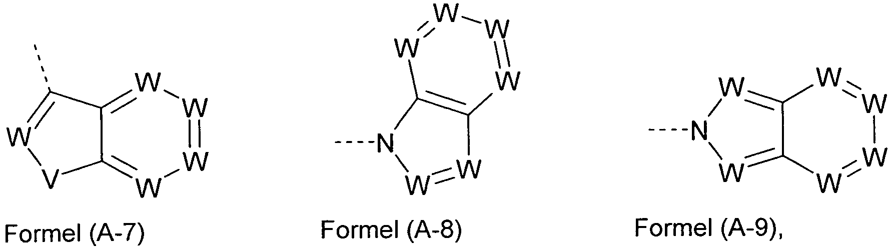 wobei die gestrichelte Bindung die Bindung an die Einheit gemäß Formel (II) darstellt, R¹ wie oben definiert ist und
- W: bei jedem Auftreten gleich oder verschieden CR¹ oder N darstellt, und
- V: NR¹, O oder S darstellt, und
wobei mindestens eine Gruppe W gleich N ist.

Weiterhin gilt für die erfindungsgemäße Verbindung, dass diese bevorzugt eine Struktur gemäß einer der folgenden Formeln (I-1) bis (I-10) aufweist: 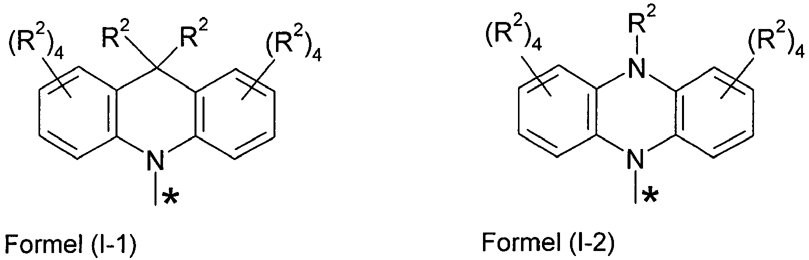 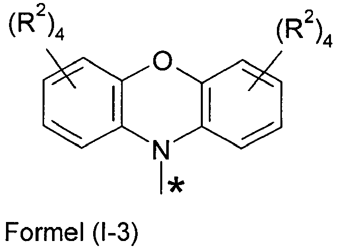 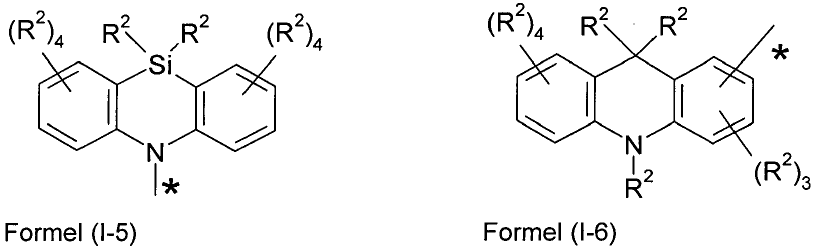 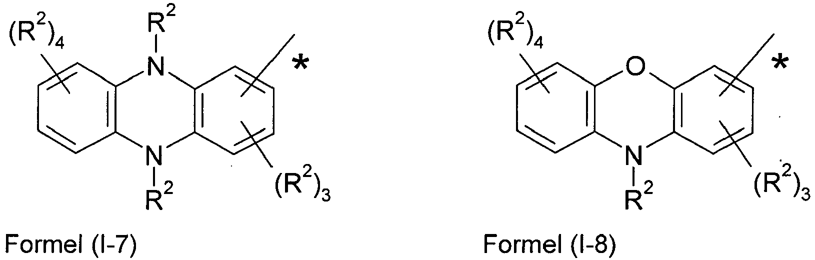 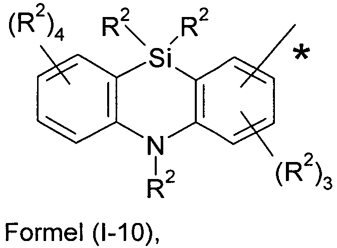 wobei über die mit * gekennzeichnete Bindung eine Gruppe der Formel (II) gebunden ist, bevorzugt in einer der oben angegebenen bevorzugten Ausführungsformen, und wobei weiterhin R² wie oben definiert ist.

Besonders bevorzugt gilt für die Einheiten der Formel (I-6) bis (I-10), dass die Gruppe der Formel (II) in para-Position zur Gruppe NR² gebunden ist.

Bevorzugt ist weiterhin die Kombination aller oben genannten bevorzugten Ausführungsformen der einzelnen variablen Gruppen.

Bevorzugt sind weiterhin die in der folgenden Tabelle gezeigten Ausführungsformen von erfindungsgemäßen Verbindungen, welche eine Struktur gemäß Formel (I-1) bis (I-10) aufweisen, welche als Gruppe der Formel (II) eine Gruppe der Formel (II-1), (II-2), (II-3), (II-4) oder (II-5) aufweisen, und welche als Gruppe A eine Einheit gemäß Formel (P), (K), (S) oder (A-1) bis (A-9) aufweisen, wobei A in meta- oder in ortho-Position gebunden ist.

| | Verbindung gemäß Formel | Gruppe der Formel (II) | Gruppe A |
|---|---|---|---|
| (I-1-1) | (I-1) | (II-1) | (P) |
| (I-1-2) | (I-1) | (II-1) | (K) |
| (I-1-3) | (I-1) | (II-1) | (S) |
| (I-1-4) | (I-1) | (II-1) | (A-1) |
| (I-1-5) | (I-1) | (II-1) | (A-2) |
| (I-1-6) | (I-1) | (II-1) | (A-3) |
| (I-1-7) | (I-1) | (II-1) | (A-4) |
| (I-1-8) | (I-1) | (II-1) | (A-5) |
| (I-1-9) | (I-1) | (II-1) | (A-6) |
| (I-1-10) | (I-1) | (II-1) | (A-7) |
| (I-1-11) | (I-1) | (II-1) | (A-8) |
| (I-1-12) | (I-1) | (II-1) | (A-9) |
| (I-1-13) | (I-1) | (II-2) | (P) |
| (I-1-14) | (I-1) | (II-2) | (K) |
| (I-1-15) | (I-1) | (II-2) | (S) |
| (I-1-16) | (I-1) | (II-2) | (A-1) |
| (I-1-17) | (I-1) | (II-2) | (A-2) |
| (I-1-18) | (I-1) | (II-2) | (A-3) |
| (I-1-19) | (I-1) | (II-2) | (A-4) |
| (I-1-20) | (I-1) | (II-2) | (A-5) |
| (I-1-21) | (I-1) | (II-2) | (A-6) |
| (I-1-22) | (I-1) | (II-2) | (A-7) |
| (I-1-23) | (I-1) | (II-2) | (A-8) |
| (I-1-24) | (I-1) | (II-2) | (A-9) |
| (I-1-25) | (I-1) | (II-3) | (P) |
| (I-1-26) | (I-1) | (II-3) | (K) |
| (I-1-27) | (I-1) | (II-3) | (S) |
| (I-1-28) | (I-1) | (II-3) | (A-1) |
| (I-1-29) | (I-1) | (II-3) | (A-2) |
| (I-1-30) | (I-1) | (II-3) | (A-3) |
| (I-1-31) | (I-1) | (II-3) | (A-4) |
| (I-1-32) | (I-1) | (II-3) | (A-5) |
| (I-1-33) | (I-1) | (II-3) | (A-6) |
| (I-1-34) | (I-1) | (II-3) | (A-7) |
| (I-1-35) | (I-1) | (II-3) | (A-8) |
| (I-1-36) | (I-1) | (II-3) | (A-9) |
| (I-1-37) | (I-1) | (II-4) | (P) |
| (I-1-38) | (I-1) | (II-4) | (K) |
| (I-1-39) | (I-1) | (II-4) | (S) |
| (I-1-40) | (I-1) | (II-4) | (A-1) |
| (I-1-41) | (I-1) | (II-4) | (A-2) |
| (I-1-42) | (I-1) | (II-4) | (A-3) |
| (I-1-43) | (I-1) | (II-4) | (A-4) |
| (I-1-44) | (I-1) | (II-4) | (A-5) |
| (I-1-45) | (I-1) | (II-4) | (A-6) |
| (I-1-46) | (I-1) | (II-4) | (A-7) |
| (I-1-47) | (I-1) | (II-4) | (A-8) |
| (I-1-48) | (I-1) | (II-4) | (A-9) |
| (I-1-49) | (I-1) | (II-5) | (P) |
| (I-1-50) | (I-1) | (II-5) | (K) |
| (I-1-51) | (I-1) | (II-5) | (S) |
| (I-1-52) | (I-1) | (II-5) | (A-1) |
| (I-1-53) | (I-1) | (II-5) | (A-2) |
| (I-1-54) | (I-1) | (II-5) | (A-3) |
| (I-1-55) | (I-1) | (II-5) | (A-4) |
| (I-1-56) | (I-1) | (II-5) | (A-5) |
| (I-1-57) | (I-1) | (II-5) | (A-6) |
| (I-1-58) | (I-1) | (II-5) | (A-7) |
| (I-1-59) | (I-1) | (II-5) | (A-8) |
| (I-1-60) | (I-1) | (II-5) | (A-9) |
| (I-2-1) | (I-2) | (II-1) | (P) |
| (I-2-2) | (I-2) | (II-1) | (K) |
| (I-2-3) | (I-2) | (II-1) | (S) |
| (I-2-4) | (I-2) | (II-1) | (A-1) |
| (I-2-5) | (I-2) | (II-1) | (A-2) |
| (I-2-6) | (I**-**2) | (II-1) | (A-3) |
| (I-2-7) | (I-2) | (II-1) | (A-4) |
| (I-2-8) | (I-2) | (II-1) | (A-5) |
| (I-2-9) | (I-2) | (II-1) | (A-6) |
| (I-2-10) | (I-2) | (II-1) | (A-7) |
| (I-2-11) | (I-2) | (II-1) | (A-8) |
| (I-2-12) | (I-2) | (II-1) | (A-9) |
| (I-2-13) | (I-2) | (II-2) | (P) |
| (I-2-14) | (I-2) | (II-2) | (K) |
| (I-2-15) | (I-2) | (II-2) | (S) |
| (I-2-16) | (I-2) | (II-2) | (A-1) |
| (I-2-17) | (I-2) | (II-2) | (A-2) |
| (I-2-18) | (I-2) | (II-2) | (A-3) |
| (I-2-19) | (I-2) | (II-2) | (A-4) |
| (I-2-20) | (I-2) | (II-2) | (A-5) |
| (I-2-21) | (I-2) | (II-2) | (A-6) |
| (I-2-22) | (I-2) | (II-2) | (A-7) |
| (I-2-23) | (I-2) | (II-2) | (A-8) |
| (I-2-24) | (I-2) | (II-2) | (A-9) |
| (I-2-25) | (I-2) | (II-3) | (P) |
| (I-2-26) | (I-2) | (II-3) | (K) |
| (I-2-27) | (I-2) | (II-3) | (S) |
| (I-2-28) | (I-2) | (II-3) | (A-1) |
| (I-2-29) | (I-2) | (II-3) | (A-2) |
| (I-2-30) | (I-2) | (II-3) | (A-3) |
| (I-2-31) | (I-2) | (II-3) | (A-4) |
| (I-2-32) | (I-2) | (II-3) | (A-5) |
| (I-2-33) | (I-2) | (II-3) | (A-6) |
| (I-2-34) | (I-2) | (II-3) | (A-7) |
| (I-2-35) | (I-2) | (II-3) | (A-8) |
| (I-2-36) | (I-2) | (II-3) | (A-9) |
| (I-2-37) | (I-2) | (II-4) | (P) |
| (I-2-38) | (I-2) | (II-4) | (K) |
| (I-2-39) | (I-2) | (II-4) | (S) |
| (I-2-40) | (I-2) | (II-4) | (A-1) |
| (I-2-41) | (I-2) | (II-4) | (A-2) |
| (I-2-42) | (I-2) | (II-4) | (A-3) |
| (I-2-43) | (I-2) | (II-4) | (A-4) |
| (I-2-44) | (I-2) | (II-4) | (A-5) |
| (I-2-45) | (I-2) | (II-4) | (A-6) |
| (I-2-46) | (I-2) | (II-4) | (A-7) |
| (I-2-47) | (I-2) | (II-4) | (A-8) |
| (I-2-48) | (I-2) | (II-4) | (A-9) |
| (I-2-49) | (I-2) | (II-5) | (P) |
| (I-2-50) | (I-2) | (II-5) | (K) |
| (I-2-51) | (I-2) | (II-5) | (S) |
| (I-2-52) | (I-2) | (II-5) | (A-1) |
| (I-2-53) | (I-2) | (II-5) | (A-2) |
| (I-2-54) | (I-2) | (II-5) | (A-3) |
| (I-2-55) | (I-2) | (II-5) | (A-4) |
| (I-2-56) | (I-2) | (II-5) | (A-5) |
| (I-2-57) | (I-2) | (II-5) | (A-6) |
| (I-2-58) | (I-2) | (II-5) | (A-7) |
| (I-2-59) | (I-2) | (II-5) | (A-8) |
| (I-2-60) | (I-2) | (II-5) | (A-9) |
| (I-3-1) | (I-3) | (II-1) | (P) |
| (I-3-2) | (I-3) | (II-1) | (K) |
| (I-3-3) | (I-3) | (II-1) | (S) |
| (I-3-4) | (I-3) | (II-1) | (A-1) |
| (I-3-5) | (I-3) | (II-1) | (A-2) |
| (I-3-6) | (I-3) | (II-1) | (A-3) |
| (I-3-7) | (I-3) | (II-1) | (A-4) |
| (I-3-8) | (I-3) | (II-1) | (A-5) |
| (I-3-9) | (I-3) | (II-1) | (A-6) |
| (I-3-10) | (I-3) | (II-1) | (A-7) |
| (I-3-11) | (I-3) | (II-1) | (A-8) |
| (I-3-12) | (I-3) | (II-1) | (A-9) |
| (I-3-13) | (I-3) | (II-2) | (P) |
| (I-3-14) | (I-3) | (II-2) | (K) |
| (I-3-15) | (I-3) | (II-2) | (S) |
| (I-3-16) | (I-3) | (II-2) | (A-1) |
| (I-3-17) | (I-3) | (II-2) | (A-2) |
| (I-3-18) | (I-3) | (II-2) | (A-3) |
| (I-3-19) | (I-3) | (II-2) | (A-4) |
| (I-3-20) | (I-3) | (II-2) | (A-5) |
| (I-3-21) | (I-3) | (II-2) | (A-6) |
| (I-3-22) | (I-3) | (II-2) | (A-7) |
| (I-3-23) | (I-3) | (II-2) | (A-8) |
| (I-3-24) | (I-3) | (II-2) | (A-9) |
| (I-3-25) | (I-3) | (II-3) | (P) |
| (I-3-26) | (I-3) | (II-3) | (K) |
| (I-3-27) | (I-3) | (II-3) | (S) |
| (I-3-28) | (I-3) | (II-3) | (A-1) |
| (I-3-29) | (I-3) | (II-3) | (A-2) |
| (I-3-30) | (I-3) | (II-3) | (A-3) |
| (I-3-31) | (I-3) | (II-3) | (A-4) |
| (I-3-32) | (I-3) | (II-3) | (A-5) |
| (I-3-33) | (I-3) | (II-3) | (A-6) |
| (I-3-34) | (I-3) | (II-3) | (A-7) |
| (I-3-35) | (I-3) | (II-3) | (A-8) |
| (I-3-36) | (I-3) | (II-3) | (A-9) |
| (I-3-37) | (I-3) | (II-4) | (P) |
| (I-3-38) | (I-3) | (II-4) | (K) |
| (I-3-39) | (I-3) | (II-4) | (S) |
| (I-3-40) | (I-3) | (II-4) | (A-1) |
| (I-3-41) | (I-3) | (II-4) | (A-2) |
| (I-3-42) | (I-3) | (II-4) | (A-3) |
| (I-3-43) | (I-3) | (II-4) | (A-4) |
| (I-3-44) | (I-3) | (II-4) | (A-5) |
| (I-3-45) | (I-3) | (II-4) | (A-6) |
| (I-3-46) | (I-3) | (II-4) | (A-7) |
| (I-3-47) | (I-3) | (II-4) | (A-8) |
| (I-3-48) | (I-3) | (II-4) | (A-9) |
| (I-3-49) | (I-3) | (II-5) | (P) |
| (I-3-50) | (I-3) | (II-5) | (K) |
| (I-3-51) | (I-3) | (II-5) | (S) |
| (I-3-52) | (I-3) | (II-5) | (A-1) |
| (I-3-53) | (I-3) | (II-5) | (A-2) |
| (I-3-54) | (I-3) | (II-5) | (A-3) |
| (I-3-55) | (I-3) | (II-5) | (A-4) |
| (I-3-56) | (I-3) | (II-5) | (A-5) |
| (I-3-57) | (I-3) | (II-5) | (A-6) |
| (I-3-58) | (I-3) | (II-5) | (A-7) |
| (I-3-59) | (I-3) | (II-5) | (A-8) |
| (I-3-60) | (I-3) | (II-5) | (A-9) |
| (I-4-1)* | (I-4) | (II-1) | (P) |
| (I-4-2)* | (I-4) | (II-1) | (K) |
| (I-4-3)* | (I-4) | (II-1) | (S) |
| (I-4-4)* | (I-4) | (II-1) | (A-1) |
| (I-4-5)* | (I-4) | (II-1) | (A-2) |
| (I-4-6)* | (I-4) | (II-1) | (A-3) |
| (I-4-7)* | (I-4) | (II-1) | (A-4) |
| (I-4-8)* | (I-4) | (II-1) | (A-5) |
| (I-4-9)* | (I-4) | (II-1) | (A-6) |
| (I-4-10)* | (I-4) | (II-1) | (A-7) |
| (I-4-11)* | (I-4) | (II-1) | (A-8) |
| (I-4-12)* | (I-4) | (II-1) | (A-9) |
| (I-4-13)* | (I-4) | (II-2) | (P) |
| (I-4-14)* | (I-4) | (II-2) | (K) |
| (I-4-15)* | (I-4) | (II-2) | (S) |
| (I-4-16)* | (I-4) | (II-2) | (A-1) |
| (I-4-17)* | (I-4) | (II-2) | (A-2) |
| (I-4-18)* | (I-4) | (II-2) | (A-3) |
| (I-4-19)* | (I-4) | (II-2) | (A-4) |
| (I-4-20)* | (I-4) | (II-2) | (A-5) |
| (I-4-21)* | (I-4) | (II-2) | (A-6) |
| (I-4-22)* | (I-4) | (II-2) | (A-7) |
| (I-4-23)* | (I-4) | (II-2) | (A-8) |
| (I-4-24)* | (I-4) | (II-2) | (A-9) |
| (I-4-25)* | (I-4) | (II-3) | (P) |
| (I-4-26)* | (I-4) | (II-3) | (K) |
| (I-4-27)* | (I-4) | (II-3) | (S) |
| (I-4-28)* | (I-4) | (II-3) | (A-1) |
| (I-4-29)* | (1-4) | (II-3) | (A-2) |
| (I-4-30)* | (1-4) | (II-3) | (A-3) |
| (I-4-31)* | (I-4) | (II-3) | (A-4) |
| (I-4-32)* | (I-4) | (II-3) | (A-5) |
| (I-4-33)* | (I-4) | (II-3) | (A-6) |
| (I-4-34)* | (I-4) | (II-3) | (A-7) |
| (I-4-35)* | (I-4) | (II-3) | (A-8) |
| (I-4-36)* | (I-4) | (II-3) | (A-9) |
| (I-4-37)* | (I-4) | (II-4) | (P) |
| (I-4-38)* | (I-4) | (II-4) | (K) |
| (I-4-39)* | (1-4) | (II-4) | (S) |
| (I-4-40)* | (I-4) | (II-4) | (A-1) |
| (I-4-41)* | (I-4) | (II-4) | (A-2) |
| (I-4-42)* | (I-4) | (II-4) | (A-3) |
| (I-4-43)* | (I-4) | (II-4) | (A-4) |
| (I-4-44)* | (I-4) | (II-4) | (A-5) |
| (I-4-45)* | (I-4) | (II-4) | (A-6) |
| (I-4-46)* | (I-4) | (II-4) | (A-7) |
| (I-4-47)* | (I-4) | (II-4) | (A-8) |
| (I-4-48)* | (1-4) | (II-4) | (A-9) |
| (I-4-49)* | (1-4) | (II-5) | (P) |
| (I-4-50)* | (1-4) | (II-5) | (K) |
| (I-4-51)* | (I-4) | (II-5) | (S) |
| (I-4-52)* | (I-4) | (II-5) | (A-1) |
| (I-4-53)* | (I-4) | (II-5) | (A-2) |
| (I-4-54)* | (I-4) | (II-5) | (A-3) |
| (I-4-55)* | (I-4) | (II-5) | (A-4) |
| (I-4-56)* | (I-4) | (II-5) | (A-5) |
| (I-4-57)* | (I-4) | (II-5) | (A-6) |
| (I-4-58)* | (I-4) | (II-5) | (A-7) |
| (I-4-59)* | (I-4) | (II-5) | (A-8) |
| (I-4-60)* | (I-4) | (II-5) | (A-9) |
| (I-5-1) | (I-5) | (II-1) | (P) |
| (I-5-2) | (I-5) | (II-1) | (K) |
| (I-5-3) | (I-5) | (II-1) | (S) |
| (I-5-4) | (I-5) | (II-1) | (A-1) |
| (I-5-5) | (I-5) | (II-1) | (A-2) |
| (I-5-6) | (I-5) | (II-1) | (A-3) |
| (I-5-7) | (I-5) | (II-1) | (A-4) |
| (I-5-8) | (I-5) | (II-1) | (A-5) |
| (I-5-9) | (I-5) | (II-1) | (A-6) |
| (I-5-10) | (I-5) | (II-1) | (A-7) |
| (I-5-11) | (I-5) | (II-1) | (A-8) |
| (I-5-12) | (I-5) | (II-1) | (A-9) |
| (I-5-13) | (I-5) | (II-2) | (P) |
| (I-5-14) | (I-5) | (II-2) | (K) |
| (I-5-15) | (I-5) | (II-2) | (S) |
| (I-5-16) | (I-5) | (II-2) | (A-1) |
| (I-5-17) | (I-5) | (II-2) | (A-2) |
| (I-5-18) | (I-5) | (II-2) | (A-3) |
| (I-5-19) | (I-5) | (II-2) | (A-4) |
| (I-5-20) | (I-5) | (II-2) | (A-5) |
| (I-5-21) | (I-5) | (II-2) | (A-6) |
| (I-5-22) | (I-5) | (II-2) | (A-7) |
| (I-5-23) | (I-5) | (II-2) | (A-8) |
| (I-5-24) | (I-5) | (II-2) | (A-9) |
| (I-5-25) | (I-5) | (II-3) | (P) |
| (I-5-26) | (I-5) | (II-3) | (K) |
| (I-5-27) | (I-5) | (II-3) | (S) |
| (I-5-28) | (I-5) | (II-3) | (A-1) |
| (I-5-29) | (I-5) | (II-3) | (A-2) |
| (I-5-30) | (I-5) | (II-3) | (A-3) |
| (I-5-31) | (I-5) | (II-3) | (A-4) |
| (I-5-32) | (I-5) | (II-3) | (A-5) |
| (I-5-33) | (I-5) | (II-3) | (A-6) |
| (I-5-34) | (I-5) | (II-3) | (A-7) |
| (I-5-35) | (I-5) | (II-3) | (A-8) |
| (I-5-36) | (I-5) | (II-3) | (A-9) |
| (I-5-37) | (I-5) | (II-4) | (P) |
| (I-5-38) | (I-5) | (II-4) | (K) |
| (I-5-39) | (I-5) | (II-4) | (S) |
| (I-5-40) | (I-5) | (II-4) | (A-1) |
| (I-5-41) | (I-5) | (II-4) | (A-2) |
| (I-5-42) | (I-5) | (II-4) | (A-3) |
| (I-5-43) | (I-5) | (II-4) | (A-4) |
| (I-5-44) | (I-5) | (II-4) | (A-5) |
| (I-5-45) | (I-5) | (II-4) | (A-6) |
| (I-5-46) | (I-5) | (II-4) | (A-7) |
| (I-5-47) | (I-5) | (II-4) | (A-8) |
| (I-5-48) | (I-5) | (II-4) | (A-9) |
| (I-5-49) | (I-5) | (II-5) | (P) |
| (I-5-50) | (I-5) | (II-5) | (K) |
| (I-5-51) | (I-5) | (II-5) | (S) |
| (I-5-52) | (I-5) | (II-5) | (A-1) |
| (I-5-53) | (I-5) | (II-5) | (A-2) |
| (I-5-54) | (I-5) | (II-5) | (A-3) |
| (I-5-55) | (I-5) | (II-5) | (A-4) |
| (I-5-56) | (I-5) | (II-5) | (A-5) |
| (I-5-57) | (I-5) | (II-5) | (A-6) |
| (I-5-58) | (I-5) . | (II-5) | (A-7) |
| (I-5-59) | (I-5) | (II-5) | (A-8) |
| (I-5-60) | (I-5) | (II-5) | (A-9) |
| (I-6-1) | (I-6) | (II-1) | (P) |
| (I-6-2) | (I-6) | (II-1) | (K) |
| (I-6-3) | (I-6) | (II-1) | (S) |
| (I-6-4) | (I-6) | (II-1) | (A-1) |
| (I-6-5) | (I-6) | (II-1) | (A-2) |
| (I-6-6) | (I-6) | (II-1) | (A-3) |
| (I-6-7) | (I-6) | (II-1) | (A-4) |
| (I-6-8) | (I-6) | (II-1) | (A-5) |
| (I-6-9) | (I-6) | (II-1) | (A-6) |
| (I-6-10) | (I-6) | (II-1) | (A-7) |
| (I-6-11) | (I-6) | (II-1) | (A-8) |
| (I-6-12) | (I-6) | (II-1) | (A-9) |
| (I-6-13) | (I-6) | (II-2) | (P) |
| (I-6-14) | (I-6) | (II-2) | (K) |
| (I-6-15) | (I-6) | (II-2) | (S) |
| (I-6-16) | (I-6) | (II-2) | (A-1) |
| (I-6-17) | (I-6) | (II-2) | (A-2) |
| (I-6-18) | (I-6) | (II-2) | (A-3) |
| (I-6-19) | (I-6) | (II-2) | (A-4) |
| (I-6-20) | (I-6) | (II-2) | (A-5) |
| (I-6-21) | (I-6) | (II-2) | (A-6) |
| (I-6-22) | (I-6) | (II-2) | (A-7) |
| (I-6-23) | (I-6) | (II-2) | (A-8) |
| (I-6-24) | (I-6) | (II-2) | (A-9) |
| (I-6-25) | (I-6) | (II-3) | (P) |
| (I-6-26) | (I-6) | (II-3) | (K) |
| (I-6-27) | (I-6) | (II-3) | (S) |
| (I-6-28) | (I-6) | (II-3) | (A-1) |
| (I-6-29) | (I-6) | (II-3) | (A-2) |
| (I-6-30) | (I-6) | (II-3) | (A-3) |
| (I-6-31) | (I-6) | (II-3) | (A-4) |
| (I-6-32) | (I-6) | (II-3) | (A-5) |
| (I-6-33) | (I-6) | (II-3) | (A-6) |
| (I-6-34) | (I-6) | (II-3) | (A-7) |
| (I-6-35) | (I-6) | (II-3) | (A-8) |
| (I-6-36) | (I-6) | (II-3) | (A-9) |
| (I-6-37) | (I-6) | (II-4) | (P) |
| (I-6-38) | (I-6) | (II-4) | (K) |
| (I-6-39) | (I-6) | (II-4) | (S) |
| (I-6-40) | (I-6) | (II-4) | (A-1) |
| (I-6-41) | (I-6) | (II-4) | (A-2) |
| (I-6-42) | (I-6) | (II-4) | (A-3) |
| (I-6-43) | (I-6) | (II-4) | (A-4) |
| (I-6-44) | (I-6) | (II-4) | (A-5) |
| (I-6-45) | (I-6) | (II-4) | (A-6) |
| (I-6-46) | (I-6) | (II-4) | (A-7) |
| (I-6-47) | (I-6) | (II-4) | (A-8) |
| (I-6-48) | (I-6) | (II-4) | (A-9) |
| (I-6-49) | (I-6) | (II-5) | (P) |
| (I-6-50) | (I-6) | (II-5) | (K) |
| (I-6-51) | (I-6) | (II-5) | (S) |
| (I-6-52) | (I-6) | (II-5) | (A-1) |
| (I-6-53) | (I-6) | (II-5) | (A-2) |
| (I-6-54) | (I-6) | (II-5) | (A-3) |
| (I-6-55) | (I-6) | (II-5) | (A-4) |
| (I-6-56) | (I-6) | (II-5) | (A-5) |
| (I-6-57) | (I-6) | (II-5) | (A-6) |
| (I-6-58) | (I-6) | (II-5) | (A-7) |
| (I-6-59) | (I-6) | (II-5) | (A-8) |
| (I-6-60) | (I-6) | (II-5) | (A-9) |
| (I-7-1) | (I-7) | (II-1) | (P) |
| (I-7-2) | (I-7) | (II-1) | (K) |
| (I-7-3) | (I-7) | (II-1) | (S) |
| (I-7-4) | (I-7) | (II-1) | (A-1) |
| (I-7-5) | (I-7) | (II-1) | (A-2) |
| (I-7-6) | (I-7) | (II-1) | (A-3) |
| (I-7-7) | (I-7) | (II-1) | (A-4) |
| (I-7-8) | (I-7) | (II-1) | (A-5) |
| (I-7-9) | (I-7) | (II-1) | (A-6) |
| (I-7-10) | (I-7) | (II-1) | (A-7) |
| (I-7-11) | (I-7) | (II-1) | (A-8) |
| (I-7-12) | (I-7) | (II-1) | (A-9) |
| (I-7-13) | (I-7) | (II-2) | (P) |
| (I-7-14) | (I-7) | (II-2) | (K) |
| (I-7-15) | (I-7) | (II-2) | (S) |
| (I-7-16) | (I-7) | (II-2) | (A-1) |
| (I-7-17) | (I-7) | (II-2) | (A-2) |
| (I-7-18) | (I-7) | (II-2) | (A-3) |
| (I-7-19) | (I-7) | (II-2) | (A-4) |
| (I-7-20) | (I-7) | (II-2) | (A-5) |
| (I-7-21) | (I-7) | (II-2) | (A-6) |
| (I-7-22) | (I-7) | (II-2) | (A-7) |
| (I-7-23) | (I-7) | (II-2) | (A-8) |
| (I-7-24) | (I-7) | (II-2) | (A-9) |
| (I-7-25) | (I-7) | (II-3) | (P) |
| (I-7-26) | (I-7) | (II-3) | (K) |
| (I-7-27) | (I-7) | (II-3) | (S) |
| (I-7-28) | (I-7) | (II-3) | (A-1) |
| (I-7-29) | (I-7) | (II-3) | (A-2) |
| (I-7-30) | (I-7) | (II-3) | (A-3) |
| (I-7-31) | (I-7) | (II-3) | (A-4) |
| (I-7-32) | (I-7) | (II-3) | (A-5) |
| (I-7-33) | (I-7) | (II-3) | (A-6) |
| (I-7-34) | (I-7) | (II-3) | (A-7) |
| (I-7-35) | (I-7) | (II-3) | (A-8) |
| (I-7-36) | (I-7) | (II-3) | (A-9) |
| (I-7-37) | (I-7) | (II-4) | (P) |
| (I-7-38) | (I-7) | (II-4) | (K) |
| (I-7-39) | (I-7) | (II-4) | (S) |
| (I-7-40) | (I-7) | (II-4) | (A-1) |
| (I-7-41) | (I-7) | (II-4) | (A-2) |
| (I-7-42) | (I-7) | (II-4) | (A-3) |
| (I-7-43) | (I-7) | (II-4) | (A-4) |
| (I-7-44) | (I-7) | (II-4) | (A-5) |
| (I-7-45) | (I-7) | (II-4) | (A-6) |
| (I-7-46) | (I-7) | (II-4) | (A-7) |
| (I-7-47) | (I-7) | (II-4) | (A-8) |
| (I-7-48) | (I-7) | (II-4) | (A-9) |
| (I-7-49) | (I-7) | (II-5) | (P) |
| (I-7-50) | (I-7) | (II-5) | (K) |
| (I-7-51) | (I-7) | (II-5) | (S) |
| (I-7-52) | (I-7) | (II-5) | (A-1) |
| (I-7-53) | (I-7) | (II-5) | (A-2) |
| (I-7-54) | (I-7) | (II-5) | (A-3) |
| (I-7-55) | (I-7) | (II-5) | (A-4) |
| (I-7-56) | (I-7) | (II-5) | (A-5) |
| (I-7-57) | (I-7) | (II-5) | (A-6) |
| (I-7-58) | (I-7) | (II-5) | (A-7) |
| (I-7-59) | (I-7) | (II-5) | (A-8) |
| (I-7-60) | (I-7) | (II-5) | (A-9) |
| (I-8-1) | (I-8) | (II-1) | (P) |
| (I-8-2) | (I-8) | (II-1) | (K) |
| (I-8-3) | (I-8) | (II-1) | (S) |
| (I-8-4) | (I-8) | (II-1) | (A-1) |
| (I-8-5) | (I-8) | (II-1) | (A-2) |
| (I-8-6) | (I-8) | (II-1) | (A-3) |
| (I-8-7) | (I-8) | (II-1) | (A-4) |
| (I-8-8) | (I-8) | (II-1) | (A-5) |
| (I-8-9) | (I-8) | (II-1) | (A-6) |
| (I-8-10) | (I-8) | (II-1) | (A-7) |
| (I-8-11) | (I-8) | (II-1) | (A-8) |
| (I-8-12) | (I-8) | (II-1) | (A-9) |
| (I-8-13) | (I-8) | (II-2) | (P) |
| (I-8-14) | (I-8) | (II-2) | (K) |
| (I-8-15) | (I-8) | (II-2) | (S) |
| (I-8-16) | (I-8) | (II-2) | (A-1) |
| (I-8-17) | (I-8) | (II-2) | (A-2) |
| (I-8-18) | (I-8) | (II-2) | (A-3) |
| (I-8-19) | (I-8) | (II-2) | (A-4) |
| (I-8-20) | (I-8) | (II-2) | (A-5) |
| (I-8-21) | (I-8) | (II-2) | (A-6) |
| (I-8-22) | (I-8) | (II-2) | (A-7) |
| (I-8-23) | (I-8) | (II-2) | (A-8) |
| (I-8-24) | (I-8) | (II-2) | (A-9) |
| (I-8-25) | (I-8) | (II-3) | (P) |
| (I-8-26) | (I-8) | (II-3) | (K) |
| (I-8-27) | (I-8) | (II-3) | (S) |
| (I-8-28) | (I-8) | (II-3) | (A-1) |
| (I-8-29) | (I-8) | (II-3) | (A-2) |
| (I-8-30) | (I-8) | (II-3) | (A-3) |
| (I-8-31) | (I-8) | (II-3) | (A-4) |
| (I-8-32) | (I-8) | (II-3) | (A-5) |
| (I-8-33) | (I-8) | (II-3) | (A-6) |
| (I-8-34) | (I-8) | (II-3) | (A-7) |
| (I-8-35) | (I-8) | (II-3) | (A-8) |
| (I-8-36) | (I-8) | (II-3) | (A-9) |
| (I-8-37) | (I-8) | (II-4) | (P) |
| (I-8-38) | (I-8) | (II-4) | (K) |
| (I-8-39) | (I-8) | (II-4) | (S) |
| (I-8-40) | (I-8) | (II-4) | (A-1) |
| (I-8-41) | (I-8) | (II-4) | (A-2) |
| (I-8-42) | (I-8) | (II-4) | (A-3) |
| (I-8-43) | (I-8) | (II-4) | (A-4) |
| (I-8-44) | (I-8) | (II-4) | (A-5) |
| (I-8-45) | (I-8) | (II-4) | (A-6) |
| (I-8-46) | (I-8) | (II-4) | (A-7) |
| (I-8-47) | (I-8) | (II-4) | (A-8) |
| (I-8-48) | (I-8) | (II-4) | (A-9) |
| (I-8-49) | (I-8) | (II-5) | (P) |
| (I-8-50) | (I-8) | (II-5) | (K) |
| (I-8-51) | (I-8) | (II-5) | (S) |
| (I-8-52) | (I-8) | (II-5) | (A-1) |
| (I-8-53) | (I-8) | (II-5) | (A-2) |
| (I-8-54) | (I-8) | (II-5) | (A-3) |
| (I-8-55) | (I-8) | (II-5) | (A-4) |
| (I-8-56) | (I-8) | (II-5) | (A-5) |
| (I-8-57) | (I-8) | (II-5) | (A-6) |
| (I-8-58) | (I-8) | (II-5) | (A-7) |
| (I-8-59) | (I-8) | (II-5) | (A-8) |
| (I-8-60) | (I-8) | (II-5) | (A-9) |
| (I-9-1)* | (I-9) | (II-1) | (P) |
| (I-9-2)* | (I-9) | (II-1) | (K) |
| (I-9-3)* | (I-9) | (II-1) | (S) |
| (I-9-4)* | (I-9) | (II-1) | (A-1) |
| (I-9-5)* | (I-9) | (II-1) | (A-2) |
| (I-9-6)* | (I-9) | (II-1) | (A-3) |
| (I-9-7)* | (I-9) | (II-1) | (A-4) |
| (I-9-8)* | (I-9) | (II-1) | (A-5) |
| (I-9-9)* | (I-9) | (II-1) | (A-6) |
| (I-9-10)* | (I-9) | (II-1) | (A-7) |
| (I-9-11)* | (I-9) | (II-1) | (A-8) |
| (I-9-12)* | (I-9) | (II-1) | (A-9) |
| (I-9-13)* | (I-9) | (II-2) | (P) |
| (I-9-14)* | (I-9) | (II-2) | (K) |
| (I-9-15)* | (I-9) | (II-2) | (S) |
| (I-9-16)* | (I-9) | (II-2) | (A-1) |
| (I-9-17)* | (I-9) | (II-2) | (A-2) |
| (I-9-18)* | (I-9) | (II-2) | (A-3) |
| (I-9-19)* | (I-9) | (II-2) | (A-4) |
| (I-9-20)* | (I-9) | (II-2) | (A-5) |
| (I-9-21)* | (I-9) | (II-2) | (A-6) |
| (I-9-22)* | (I-9) | (II-2) | (A-7) |
| (I-9-23)* | (I-9) | (II-2) | (A-8) |
| (I-9-24)* | (I-9) | (II-2) | (A-9) |
| (I-9-25)* | (I-9) | (II-3) | (P) |
| (I-9-26)* | (I-9) | (II-3) | (K) |
| (I-9-27)* | (I-9) | (II-3) | (S) |
| (I-9-28)* | (I-9) | (II-3) | (A-1) |
| (I-9-29)* | (I-9) | (II-3) | (A-2) |
| (I-9-30)* | (1-9) | (II-3) | (A-3) |
| (I-9-31)* | (I-9) | (II-3) | (A-4) |
| (I-9-32)* | (I-9) | (II-3) | (A-5) |
| (I-9-33)* | (I-9) | (II-3) | (A-6) |
| (I-9-34)* | (I-9) | (II-3) | (A-7) |
| (I-9-35)* | (I-9) | (II-3) | (A-8) |
| (I-9-36)* | (I-9) | (II-3) | (A-9) |
| (I-9-37)* | (I-9) | (II-4) | (P) |
| (I-9-38)* | (1-9) | (II-4) | (K) |
| (I-9-39)* | (1-9) | (II-4) | (S) |
| (I-9-40)* | (1-9) | (II-4) | (A-1) |
| (I-9-41)* | (I-9) | (II-4) | (A-2) |
| (I-9-42)* | (I-9) | (II-4) | (A-3) |
| (I-9-43)* | (I-9) | (II-4) | (A-4) |
| (I-9-44)* | (I-9) | (II-4) | (A-5) |
| (I-9-45)* | (I-9) | (II-4) | (A-6) |
| (I-9-46)* | (I-9) | (II-4) | (A-7) |
| (I-9-47)* | (I-9) | (II-4) | (A-8) |
| (I-9-48)* | (I-9) | (II-4) | (A-9) |
| (I-9-49)* | (I-9) | (II-5) | (P) |
| (I-9-50)* | (I-9) | (II-5) | (K) |
| (I-9-51)* | (I-9) | (II-5) | (S) |
| (I-9-52)* | (I-9) | (II-5) | (A-1) |
| (I-9-53)* | (I-9) | (II-5) | (A-2) |
| (I-9-54)* | (I-9) | (II-5) | (A-3) |
| (I-9-55)* | (I-9) | (II-5) | (A-4) |
| (I-9-56)* | (I-9) | (II-5) | (A-5) |
| (I-9-57)* | (I-9) | (II-5) | (A-6) |
| (I-9-58)* | (I-9) | (II-5) | (A-7) |
| (I-9-59)* | (1-9) | (II-5) | (A-8) |
| (I-9-60)* | (I-9) | (II-5) | (A-9) |
| (I-10-1) | (I-10) | (II-1) | (P) |
| (I-10-2) | (I-10) | (II-1) | (K) |
| (I-10-3) | (I-10) | (II-1) | (S) |
| (I-10-4) | (I-10) | (II-1) | (A-1) |
| (I-10-5) | (I-10) | (II-1) | (A-2) |
| (I-10-6) | (I-10) | (II-1) | (A-3) |
| (I-10-7) | (I-10) | (II-1) | (A-4) |
| (I-10-8) | (I-10) | (II-1) | (A-5) |
| (I-10-9) | (I-10) | (II-1) | (A-6) |
| (I-10-10) | (I-10) | (II-1) | (A-7) |
| (I-10-11) | (I-10) | (II-1) | (A-8) |
| (I-10-12) | (I-10) | (II-1) | (A-9) |
| (I-10-13) | (I-10) | (II-2) | (P) |
| (I-10-14) | (I-10) | (II-2) | (K) |
| (I-10-15) | (I-10) | (II-2) | (S) |
| (I-10-16) | (I-10) | (II-2) | (A-1) |
| (I-10-17) | (I-10) | (II-2) | (A-2) |
| (I-10-18) | (I-10) | (II-2) | (A-3) |
| (I-10-19) | (I-10) | (II-2) | (A-4) |
| (I-10-20) | (I-10) | (II-2) | (A-5) |
| (I-10-21) | (I-10) | (II-2) | (A-6) |
| (I-10-22) | (I-10) | (II-2) | (A-7) |
| (I-10-23) | (I-10) | (II-2) | (A-8) |
| (I-10-24) | (I-10) | (II-2) | (A-9) |
| (I-10-25) | (I-10) | (II-3) | (P) |
| (I-10-26) | (I-10) | (II-3) | (K) |
| (I-10-27) | (I-10) | (II-3) | (S) |
| (I-10-28) | (I-10) | (II-3) | (A-1) |
| (I-10-29) | (I-10) | (II-3) | (A-2) |
| (I-10-30) | (I-10) | (II-3) | (A-3) |
| (I-10-31) | (I-10) | (II-3) | (A-4) |
| (I-10-32) | (I-10) | (II-3) | (A-5) |
| (I-10-33) | (I-10) | (II-3) | (A-6) |
| (I-10-34) | (I-10) | (II-3) | (A-7) |
| (I-10-35) | (I-10) | (II-3) | (A-8) |
| (I-10-36) | (I-10) | (II-3) | (A-9) |
| (I-10-37) | (I-10) | (II-4) | (P) |
| (I-10-38) | (I-10) | (II-4) | (K) |
| (I-10-39) | (I-10) | (II-4) | (S) |
| (I-10-40) | (I-10) | (II-4) | (A-1) |
| (I-10-41) | (I-10) | (II-4) | (A-2) |
| (I-10-42) | (I-10) | (II-4) | (A-3) |
| (I-10-43) | (I-10) | (II-4) | (A-4) |
| (I-10-44) | (I-10) | (II-4) | (A-5) |
| (I-10-45) | (I-10) | (II-4) | (A-6) |
| (I-10-46) | (I-10) | (II-4) | (A-7) |
| (I-10-47) | (I-10) | (II-4) | (A-8) |
| (I-10-48) | (I-10) | (II-4) | (A-9) |
| (I-10-49) | (I-10) | (II-5) | (P) |
| (I-10-50) | (I-10) | (II-5) | (K) |
| (I-10-51) | (I-10) | (II-5) | (S) |
| (I-10-52) | (I-10) | (II-5) | (A-1) |
| (I-10-53) | (I-10) | (II-5) | (A-2) |
| (I-10-54) | (I-10) | (II-5) | (A-3) |
| (I-10-55) | (I-10) | (II-5) | (A-4) |
| (I-10-56) | (I-10) | (II-5) | (A-5) |
| (I-10-57) | (I-10) | (II-5) | (A-6) |
| (I-10-58) | (1-10) | (II-5) | (A-7) |
| (I-10-59) | (I-10) | (II-5) | (A-8) |
| (I-10-60) | (1-10) | (II-5) | (A-9) |

| | | | |
|---|---|---|---|
| **Nicht gemäß der Erfindung* | | | |

wobei die auftretenden Symbole wie oben definiert sind und bevorzugt in den oben angegebenen bevorzugten Ausführungsformen vorliegen.

Beispiele für erfindungsgemäße Verbindungen sind in der folgenden Tabelle aufgeführt.

| | |
|---|---|
| 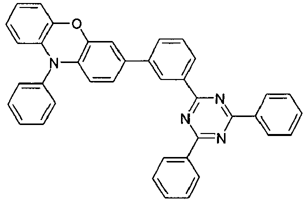 | 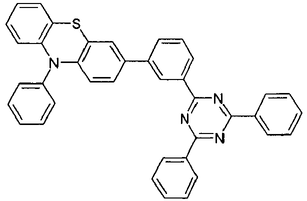 |
| 1 | 2* |
| 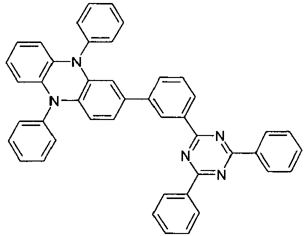 | 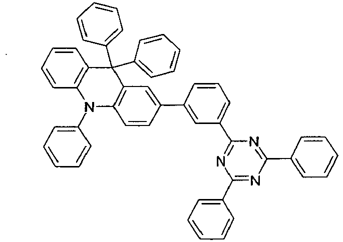 |
| 3 | 4 |
| 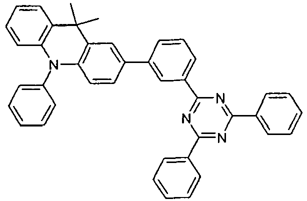 | 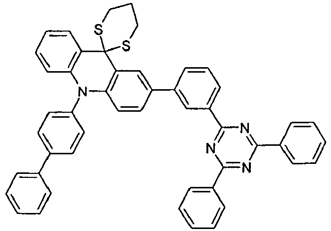 |
| 5 | 6 |
| 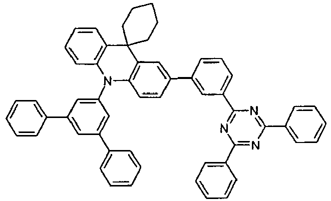 | 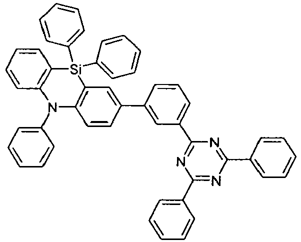 |
| 7 | 8 |
| 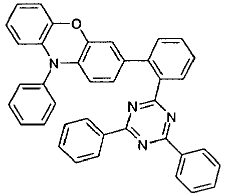 | 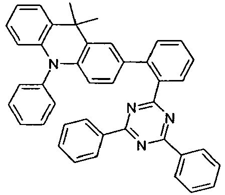 |
| 9 | 10 |
| 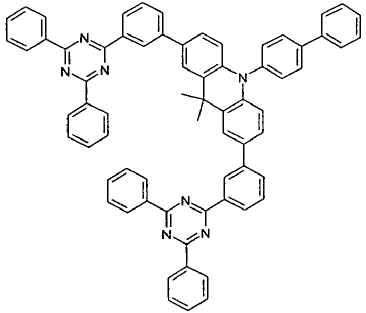 | 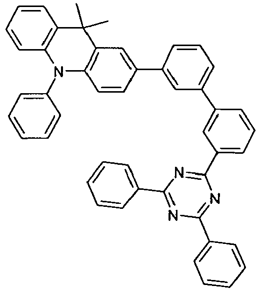 |
| 11 | 12 |
| 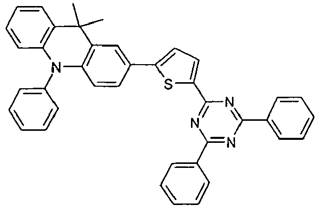 | 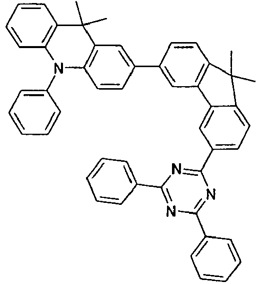 |
| 13 | 14 |
| 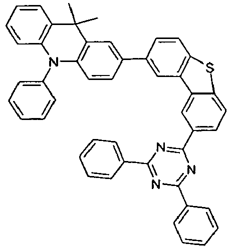 | 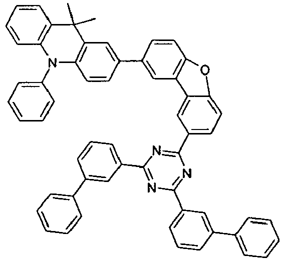 |
| 15 | 16 |
| 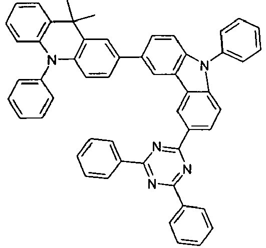 | 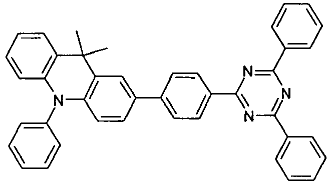 |
| 17 | 18 |
| 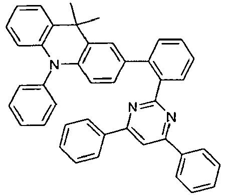 | 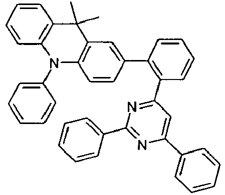 |
| 19 | 20 |
| 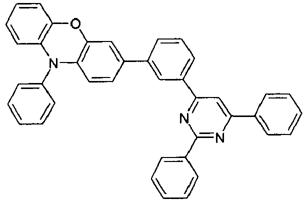 | 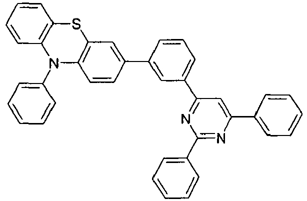 |
| 21 | 22* |
| 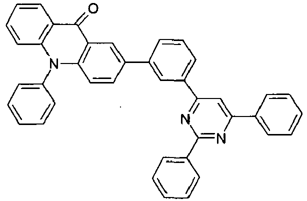 | 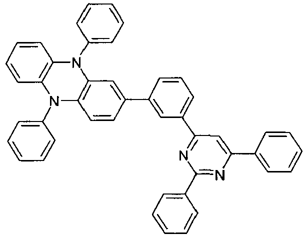 |
| 23* | 24 |
| 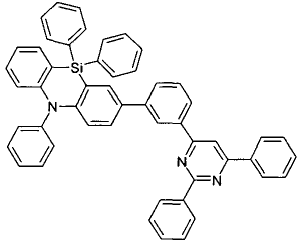 | 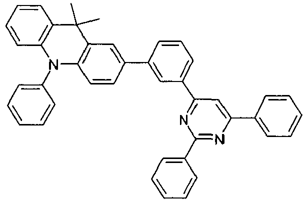 |
| 25 | 26 |
| 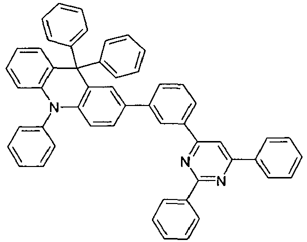 | 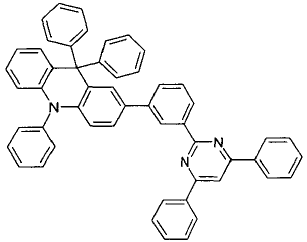 |
| 27 | 28 |
| 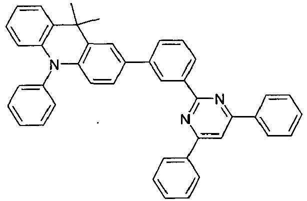 | 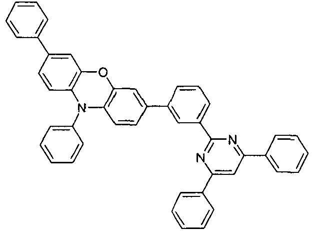 |
| 29 | 30 |
| 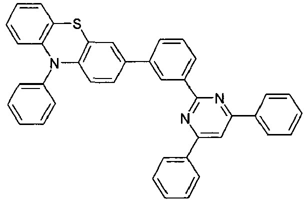 | 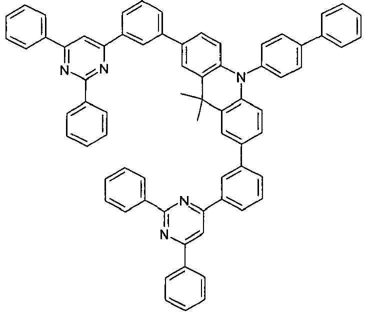 |
| 31* | 32 |
| 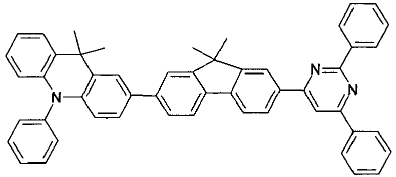 | 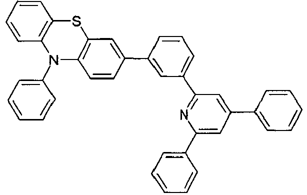 |
| 33 | 34* |
| 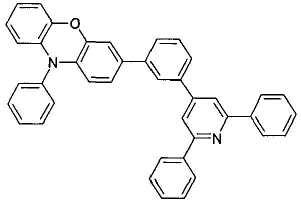 | 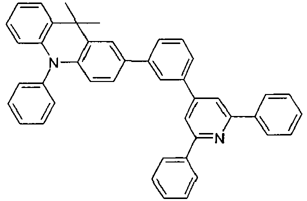 |
| 35 | 36 |
| 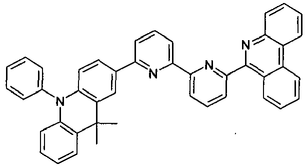 | 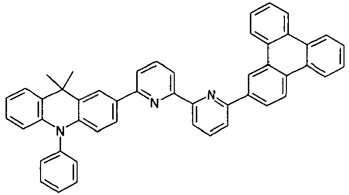 |
| 37 | 38 |
| 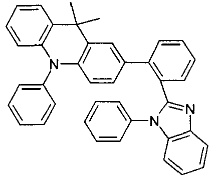 | 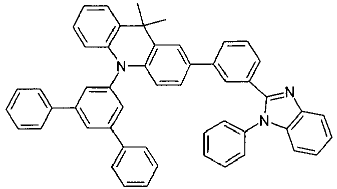 |
| 39 | 40 |
| 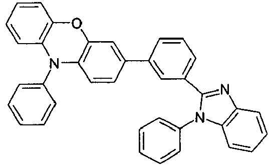 | 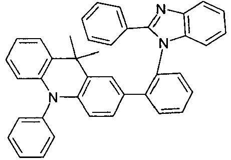 |
| 41 | 42 |
| 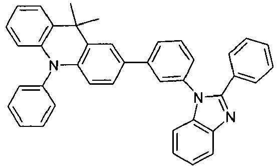 | 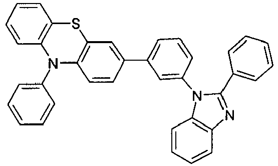 |
| 43 | 44* |
| 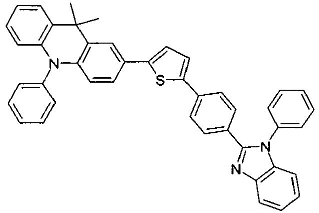 | 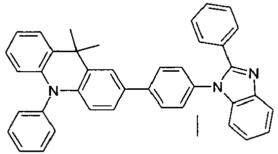 |
| 45 | 46 |
| 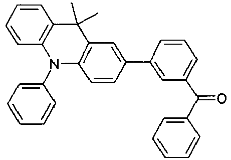 | 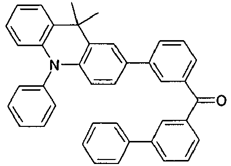 |
| 47 | 48 |
| 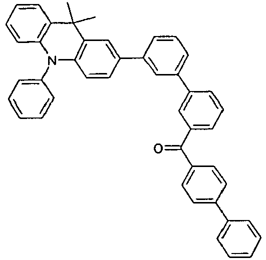 | 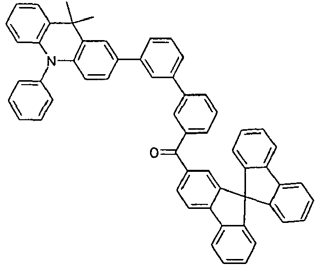 |
| 49 | 50 |
| 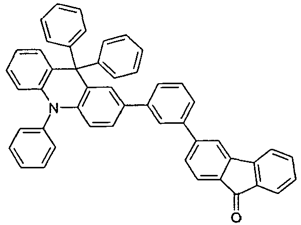 | 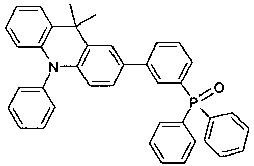 |
| 51 | 52 |
| 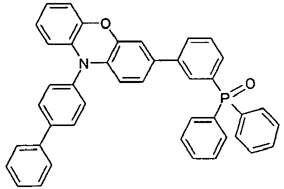 | 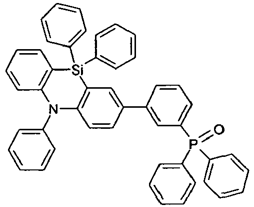 |
| 53 | 54 |
| 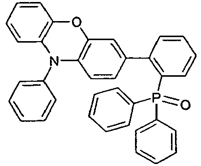 | 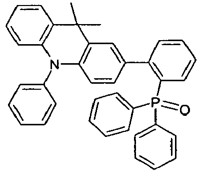 |
| 55 | 56 |
| 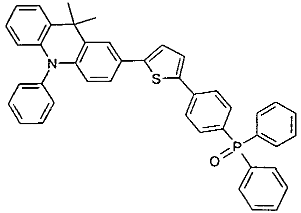 | 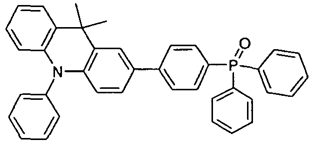 |
| 57 | 58 |
| 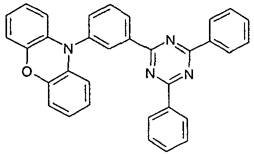 | 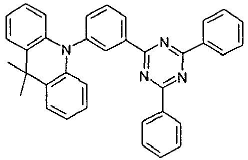 |
| 59 | 60 |
| 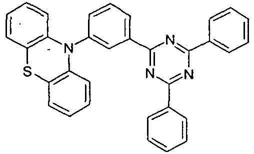 | 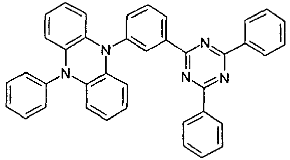 |
| 61* | 62 |
| 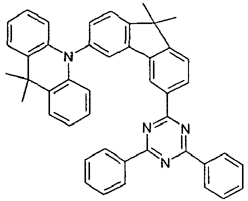 | 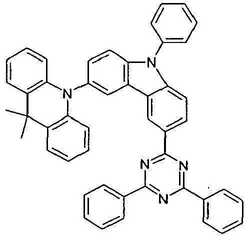 |
| 63 | 64 |
| 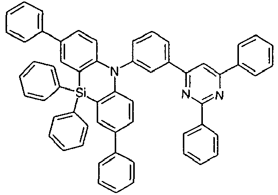 | 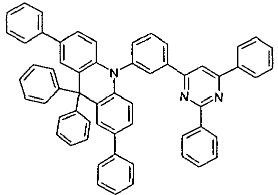 |
| 65 | 66 |
| 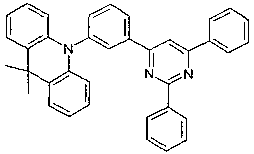 | 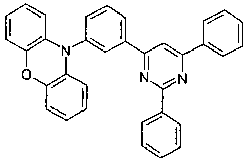 |
| 67 | 68 |
| 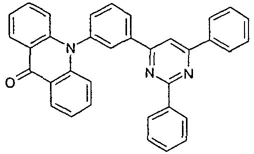 | 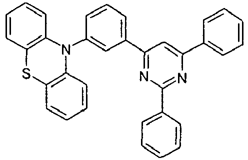 |
| 69^{*} | 70* |
| 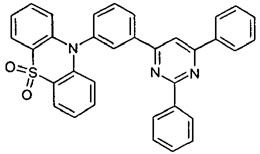 | 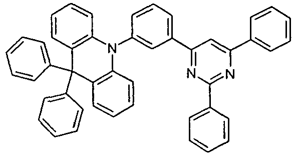 |
| 71* | 72 |
| 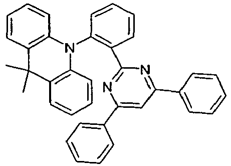 | 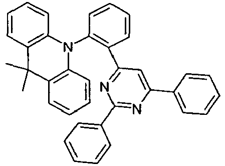 |
| 73 | 74 |
| 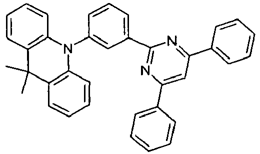 | 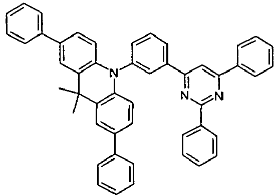 |
| 75 | 76 |
| 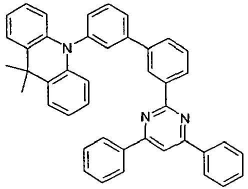 | 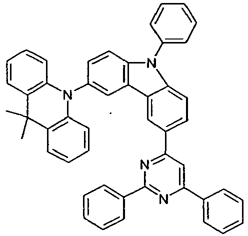 |
| 77 | 78 |
| 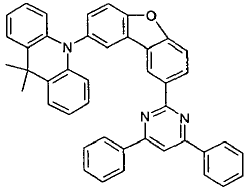 | 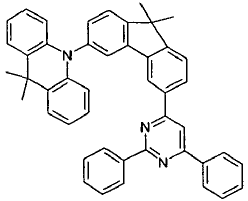 |
| 79 | 80 |
| 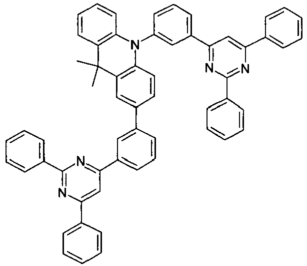 | 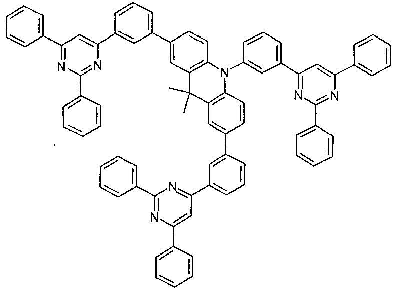 |
| 81 | 82 |
| 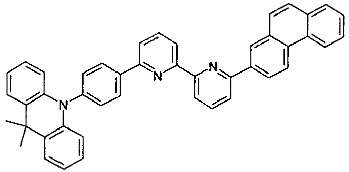 | 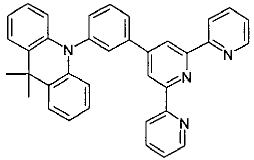 |
| 83 | 84 |
| 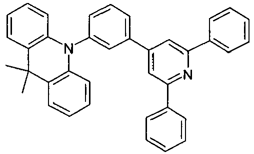 | 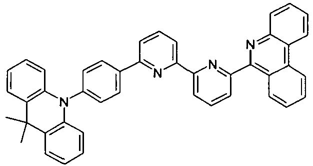 |
| 85 | 86 |
| 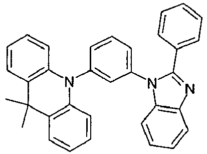 | 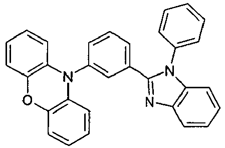 |
| 87 | 88 |
| 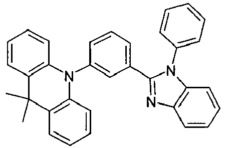 | 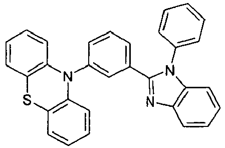 |
| 89 | 90* |
| 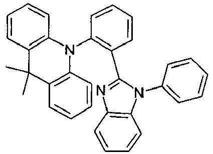 | 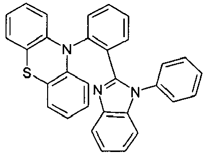 |
| 91 | 92* |
| 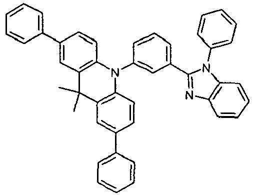 | 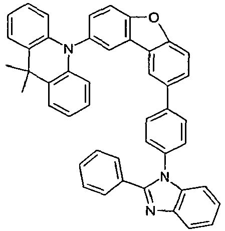 |
| 93 | 94 |
| 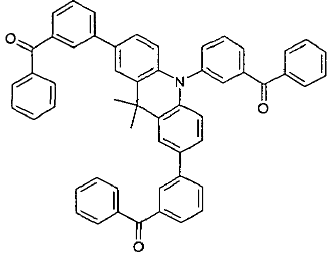 | 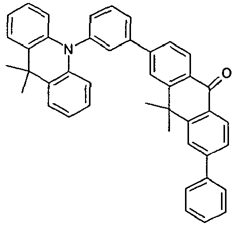 |
| 95 | 96 |
| 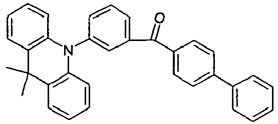 | 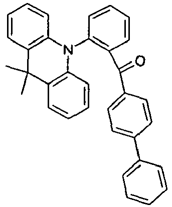 |
| 97 | 98 |
| 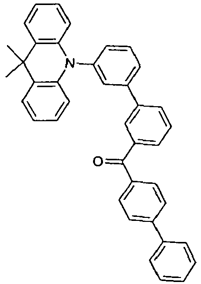 | 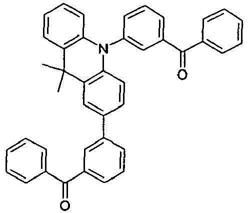 |
| 99 | 100 |
| 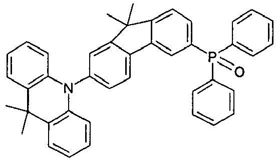 | 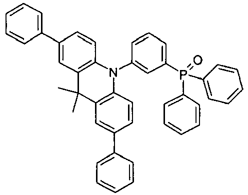 |
| 101 | 102 |
| 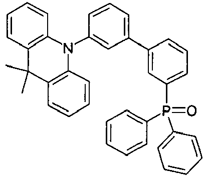 | 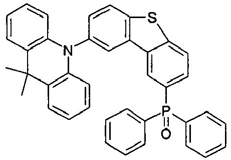 |
| 103 | 104 |
| 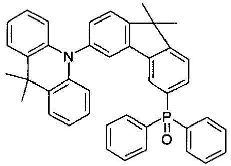 | 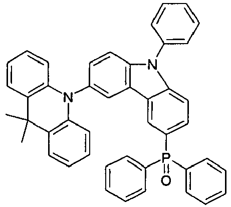 |
| 105 | 106 |
| 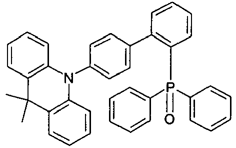 | |
| 107 | |

| | |
|---|---|
| **Nicht gemäß der Erfindung* | |

Die erfindungsgemäßen Verbindungen können durch bekannte organischchemische Syntheseverfahren hergestellt werden. Dazu zählen beispielsweise die Ullmann-Kupplung, die Hartwig-Buchwald-Kupplung, die Suzuki-Kupplung sowie Halogenierungsreaktionen.

Gemäß dem folgenden Schema 1 können erfindungsgemäße Verbindungen hergestellt werden, in denen die Gruppe A (gezeigt ist beispielhaft eine Sechsring-Heteroarylgruppe) an einen der aromatischen Ringe der Dihydroacridineinheit (Dibenzopiperidineinheit) gebunden ist.

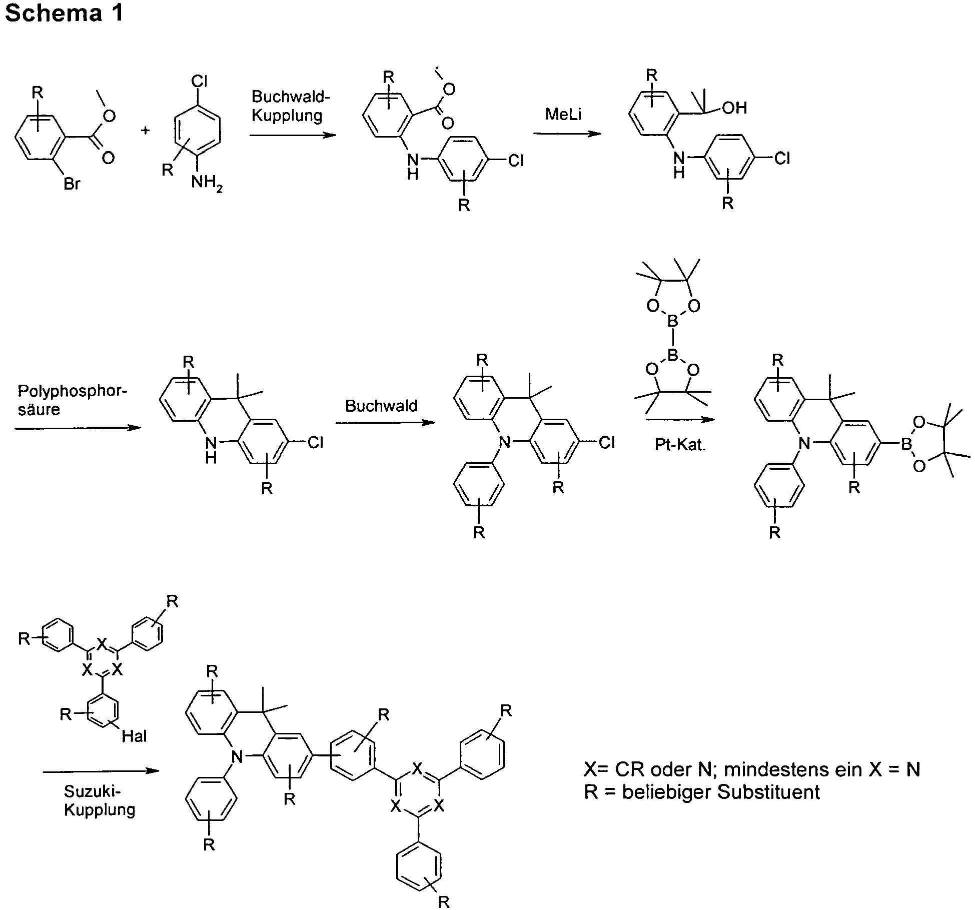

Hierzu wird zunächst über eine Buchwald-Kupplung mit anschließender Ringschlussreaktion die Dihydroacridineinheit synthetisiert. Vergleichbare Reaktionen wurden unter anderem in der Anmeldung WO 2010/083871 offenbart und näher beschrieben. Anschließend wird die freie NH-Funktion des Dihydroacridins in einer Buchwald-Kupplung aryliert. Dann wird eine Boronsäureester-Gruppe eingeführt und in einer Suzuki-Reaktion die Gruppe A, im vorliegenden Fall eine Sechsring-Heteroarylgruppe, gekuppelt. Weitere Funktionalisierungsschritte können sich anschließen. Alternativ zur Kupplung mit einer Sechsring-Heteroarylgruppe kann auch eine Kupplung mit einer anderen Gruppe A gemäß der Definition der vorliegenden Erfindung erfolgen, beispielsweise einer Fünfring-Heteroarylgruppe oder einer Arylketon- oder einer Arylphosphinoxid-Gruppe.

Das folgende Schema 2 zeigt die Synthese von erfindungsgemäßen Verbindungen, in denen die Gruppe A (gezeigt ist beispielhaft eine Sechsring-Heteroarylgruppe) an das Stickstoffatom der Dihydroacridineinheit gebunden ist.

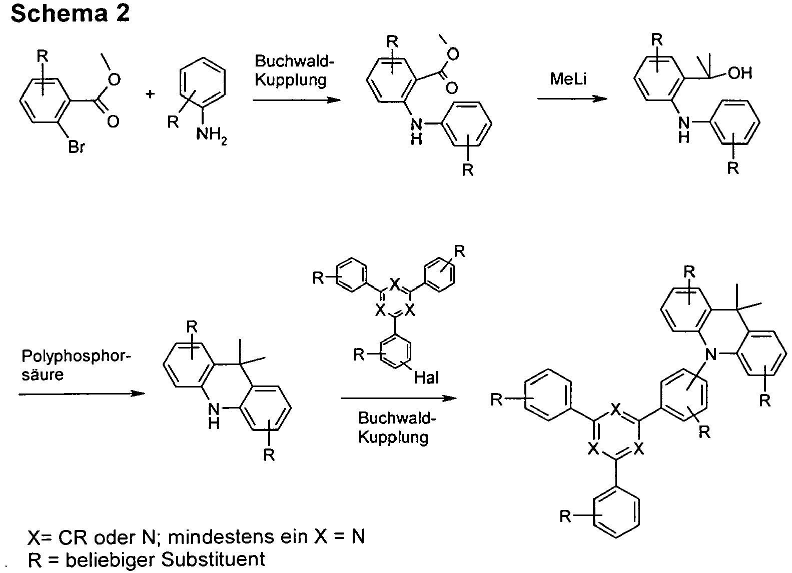

Hierzu wird im Anschluss an die Synthese des Dihydroacridin-Grundkörpers über eine Buchwald-Kupplung die Sechsring-Heteroarylgruppe in der entsprechenden Position des Dihydroacridins eingeführt. Weitere Funktionalisierungsschritte können sich anschließen. Alternativ zur Kupplung mit einer Sechsring-Heteroarylgruppe kann auch eine Kupplung mit einer anderen Gruppe A gemäß der Definition der vorliegenden Erfindung erfolgen, beispielsweise einer Fünfring-Heteroarylgruppe oder einer Arylketon- oder einer Arylphosphinoxid-Gruppe.

In den Schemata 1 und 2 wird die Synthese von erfindungsgemäßen Verbindungen gezeigt, in denen die Gruppe Y eine Methylenbrücke darstellt. Alternativ können jedoch durch Anwendung von dem Fachmann bekannten Syntheseverfahren auch andere Gruppen Y realisiert werden. Teilweise kann auch von kommerziell erhältlichen Intermediaten ausgegangen werden, wie in einigen Synthesebeispielen des experimentellen Teils gezeigt wird.

Weiterer Gegenstand der Erfindung ist zusammenfassend ein Verfahren zur Herstellung einer Verbindung gemäß Formel (I), dadurch gekennzeichnet, dass mindestens eine Gruppe umfassend eine Struktur gemäß Formel (II) in einer metallkatalysierten Kupplungsreaktion eingeführt wird.

Die oben beschriebenen erfindungsgemäßen Verbindungen, insbesondere Verbindungen, welche mit reaktiven Abgangsgruppen substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹, R² oder R³ substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein. Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Für die Verarbeitung der Verbindungen gemäß Formel (I) aus flüssiger Phase, beispielsweise durch Spin-Coating oder durch Druckverfahren, sind Formulierungen der Verbindungen erforderlich. Diese Formulierungen können beispielsweise Lösungen, Dispersionen oder Miniemulsionen sein.

Gegenstand der Erfindung ist daher weiterhin eine Formulierung, insbesondere eine Lösung, Dispersion oder Miniemulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in den Anmeldungen WO 2002/072714 und WO 2003/019694 und der darin zitierten Literatur beschrieben.

Die Verbindungen gemäß Formel (I) eignen sich für den Einsatz in elektronischen Vorrichtungen, insbesondere in organischen Elektrolumineszenzvorrichtungen (OLEDs). Die Verbindungen können, unter anderem abhängig von der Substitution, in unterschiedlichen Funktionen und/oder Schichten eingesetzt werden. Bevorzugt werden die Verbindungen als Hostmaterialien für phosphoreszierende Emitter oder als Elektronentransportmaterialien eingesetzt.

Ein weiterer Gegenstand der Erfindung ist die Verwendung der Verbindungen gemäß Formel (I) in elektronischen Vorrichtungen. Dabei sind die elektronischen Vorrichtungen bevorzugt ausgewählt aus der Gruppe bestehend aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und besonders bevorzugt ausgewählt aus organischen Elektrolumineszenzvorrichtungen (OLEDs).

Gegenstand der Erfindung ist weiterhin eine elektronische Vorrichtung, enthaltend Anode, Kathode sowie mindestens eine organische Schicht, wobei die organische Schicht mindestens eine Verbindung der Formel (I) enthält. Dabei ist die elektronische Vorrichtung bevorzugt ausgewählt aus den oben genannten Vorrichtungen und besonders bevorzugt eine organische Elektrolumineszenzvorrichtung (OLED).

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer), Auskopplungsschichten und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt. Die in den jeweiligen Schichten und Funktionen bevorzugt eingesetzten Verbindungen werden in späteren Abschnitten explizit offenbart.

Es ist erfindungsgemäß bevorzugt, wenn die Verbindung gemäß Formel (I) in einer elektronischen Vorrichtung enthaltend einen oder mehrere phosphoreszierende Dotanden eingesetzt wird. Dabei kann die Verbindung in unterschiedlichen Schichten, bevorzugt in einer Elektronentransportschicht, einer Lochtransportschicht, einer Lochinjektionsschicht oder in der emittierenden Schicht verwendet werden. Die Verbindung gemäß Formel (I) kann aber auch erfindungsgemäß in einer elektronischen Vorrichtung, enthaltend einen oder mehrere fluoreszierende Dotanden und keine phosphoreszierenden Dotanden, eingesetzt werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen. Weitere Beispiele für geeignete phosphoreszierende Dotanden können der in einem späteren Abschnitt folgenden Tabelle entnommen werden.

In einer bevorzugten Ausführungsform der vorliegenden Erfindung werden die Verbindungen der Formel (I) als Matrixmaterial in Kombination mit einem oder mehreren Dotanden, vorzugsweise phosphoreszierenden Dotanden, eingesetzt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%. Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß Formel (I) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Dotanden umfassen. Die Dotandverbindung bzw. die Dotandverbindungen zusammen haben erfindungsgemäß einen Anteil von 0.1 bis 50.0 Vol.-% an der Gesamtmischung und bevorzugt einen Anteil von 0.5 bis 20.0 Vol.-% an der Gesamtmischung. Entsprechend haben die Matrixkomponenten zusammen einen Anteil von 50.0 bis 99.9 Vol-% an der Gesamtmischung und bevorzugt einen Anteil von 80.0 bis 99.5 Vol.-% an der Gesamtmischung.

Besonders geeignete Matrixmaterialien, welche in Kombination mit den erfindungsgemäßen Verbindungen als Matrixkomponenten eines Mixed-Matrix-Systems eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, Indenocarbazolderivate, z. B. gemäß WO 2010/136109 und WO 2011/000455, oder verbrückte Carbazole, z. B gemäß WO 2011/088877.9 und WO 2011/128017.

Bevorzugte phosphoreszierende Dotanden zur Verwendung in Mixed-Matrix-Systemen enthaltend die erfindungsgemäßen Verbindungen sind die in einer folgenden Tabelle aufgeführten phosphoreszierenden Dotanden.

In einer weiteren bevorzugten Ausführungsform der Erfindung wird die Verbindung gemäß Formel (I) als Elektronentransportmaterial in einer Elektronentransport- oder Elektroneninjektionsschicht eingesetzt. Dabei kann die emittierende Schicht fluoreszierende und/oder phosphoreszierende Emitter enthalten. Wenn die Verbindung als Elektronentransportmaterial eingesetzt wird, kann es bevorzugt sein, wenn sie dotiert ist, beispielsweise mit Alkalimetallkomplexen, wie z. B. Liq (Lithiumhydroxychinolinat). Auch eignet sich die Kombination der erfindungsgemäßen Verbindung in einer Elektronentransportschicht mit einer Elektroneninjektionsschicht. Als Materialien für die Elektroneninjektionsschicht eignen sich beispielsweise Alkali- oder Erdalkalifluoride, wie z. B. LiF.

In nochmals einer weiteren bevorzugten Ausführungsform der Erfindung wird die Verbindung gemäß Formel (I) als Lochblockiermaterial in einer Lochblockierschicht eingesetzt. Unter einer Lochblockierschicht wird eine Schicht verstanden, die auf Kathodenseite direkt an eine emittierende Schicht angrenzt.

Die erfindungsgemäße organische Elektrolumineszenzvorrichtung kann auch mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren, wobei die verschiedenen Farben in dieser Ausführungsform der Erfindung zusammen weißes Licht ergeben. Insbesondere bevorzugt sind Dreischichtsysteme, also Systeme mit drei emittierenden Schichten, wobei eine oder mehrere dieser Schichten eine Verbindung gemäß Formel (I) enthalten kann und wobei die drei Schichten blaue, grüne und orangefarbene oder rote Emission zeigen (für den prinzipiellen Aufbau siehe z. B. WO 2005/011013). Ebenso eignen sich in solchen Systemen für weiße Emission Emitter, welche breitbandige Emissionsbanden aufweisen und dadurch weiße Emission zeigen. Alternativ und/oder zusätzlich können die erfindungsgemäßen Verbindungen in solchen Systemen auch in einer Lochtransportschicht oder Elektronentransportschicht oder in einer anderen Schicht vorhanden sein.

Im Folgenden werden die in den erfindungsgemäßen elektronischen Vorrichtungen bevorzugt eingesetzten weiteren Funktionsmaterialien aufgeführt.

Die in der folgenden Tabelle aufgeführten Verbindungen stellen besonders geeignete phosphoreszierende Dotanden dar.

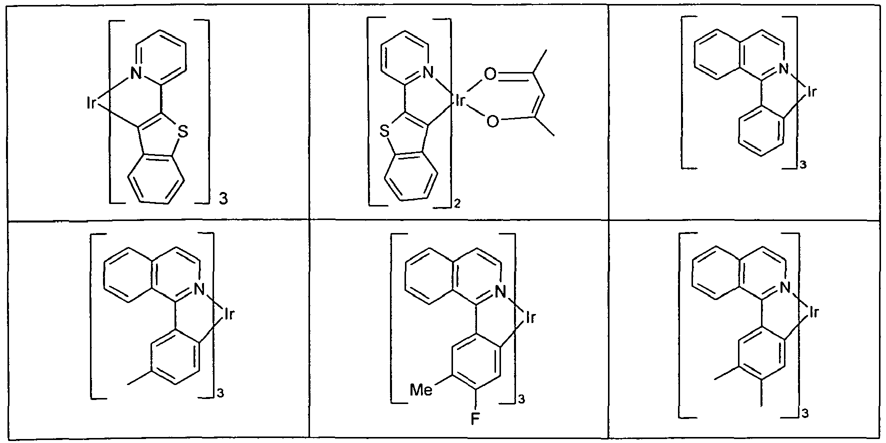

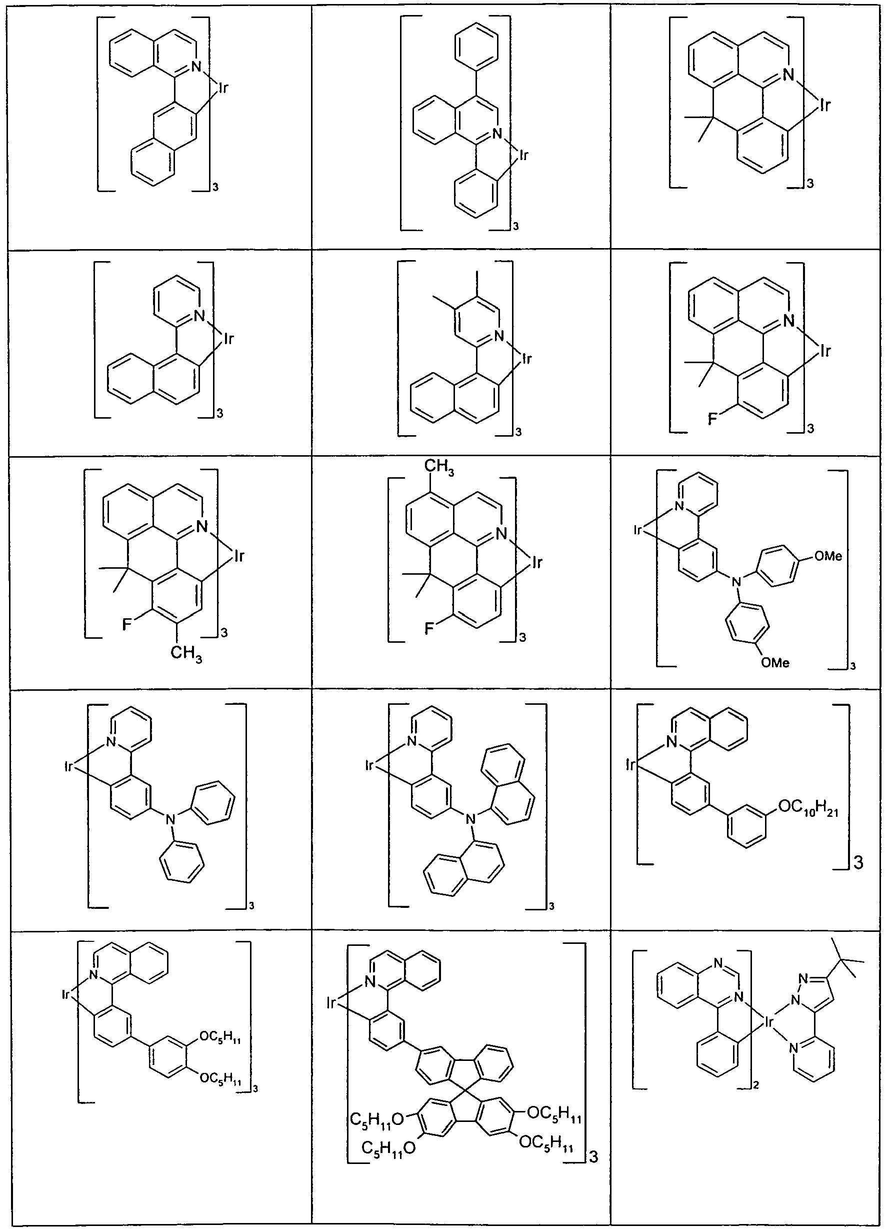

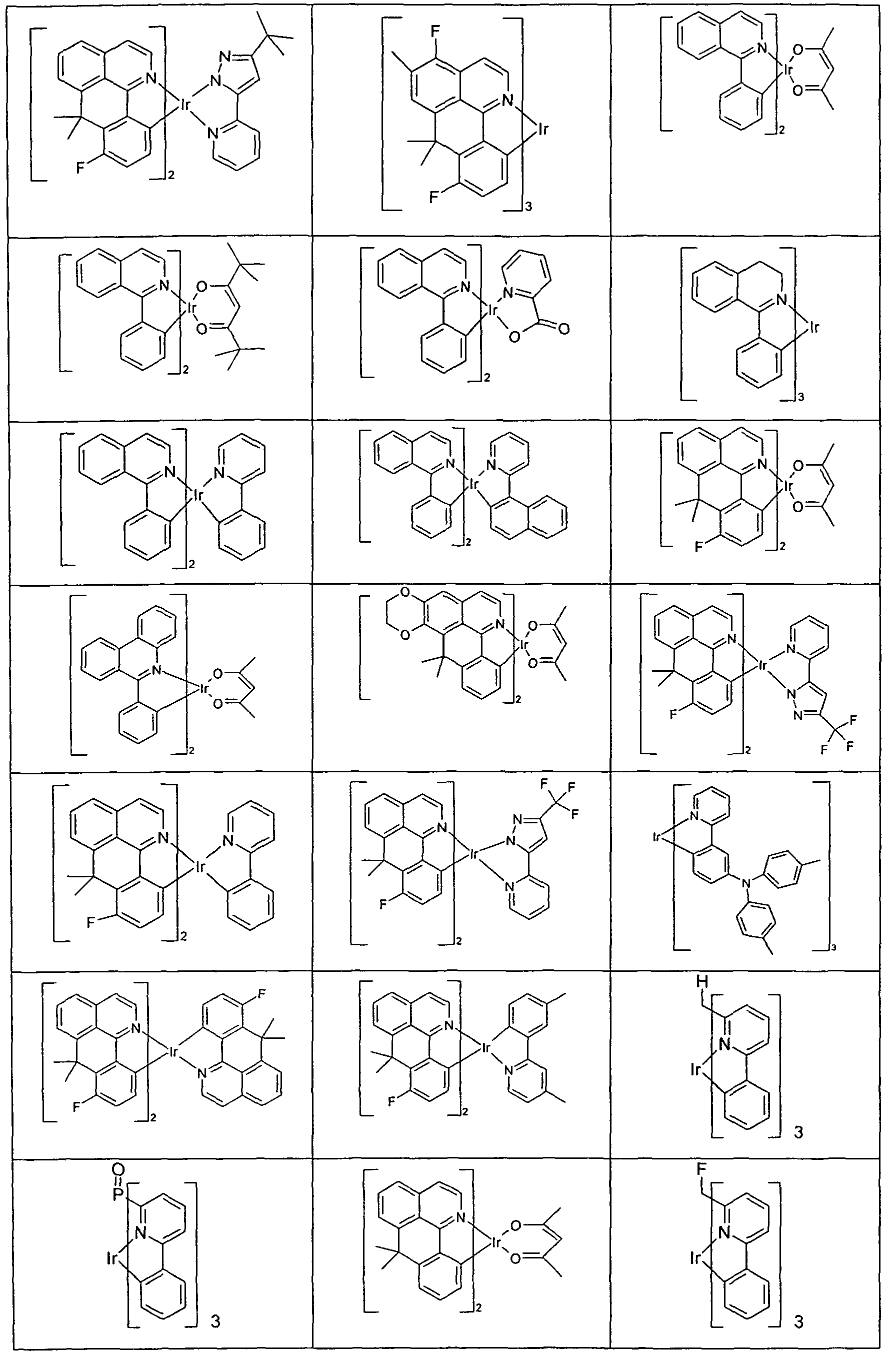

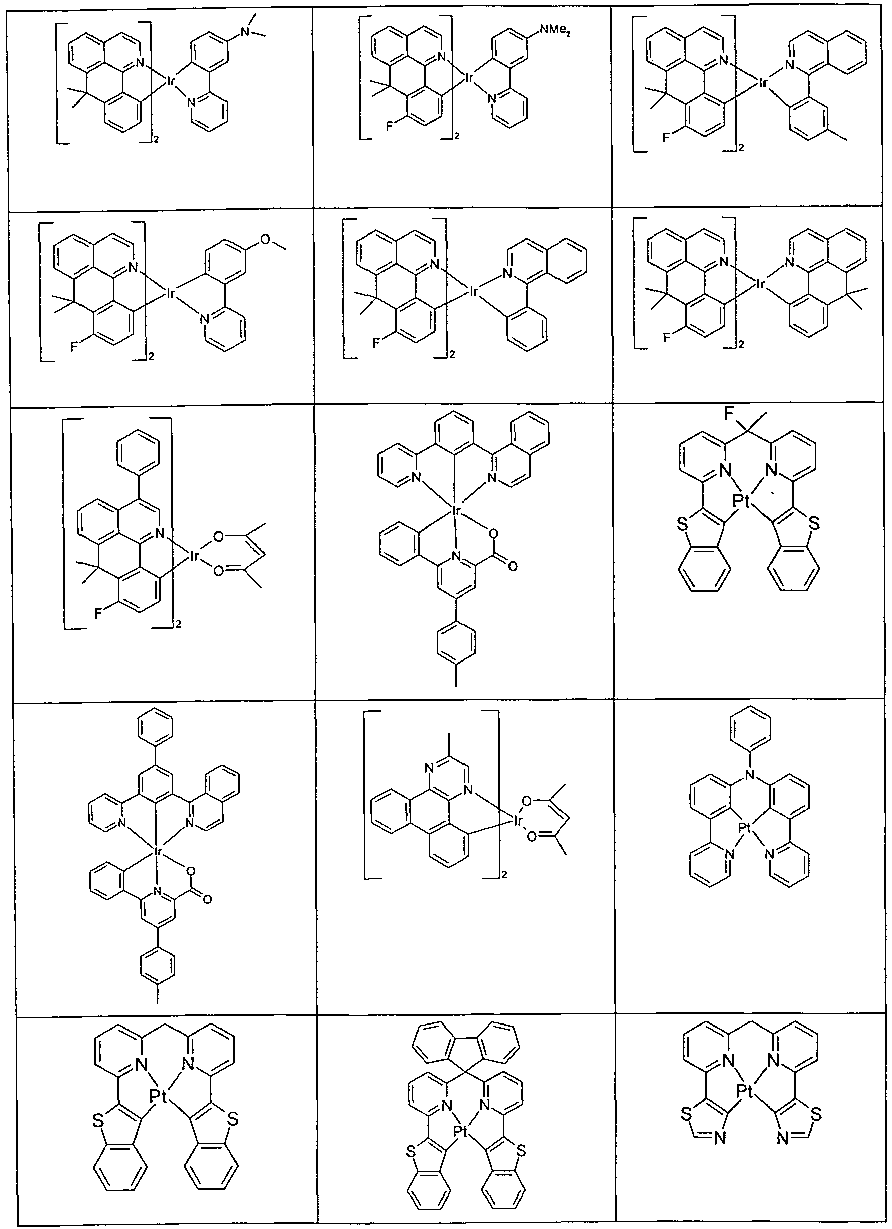

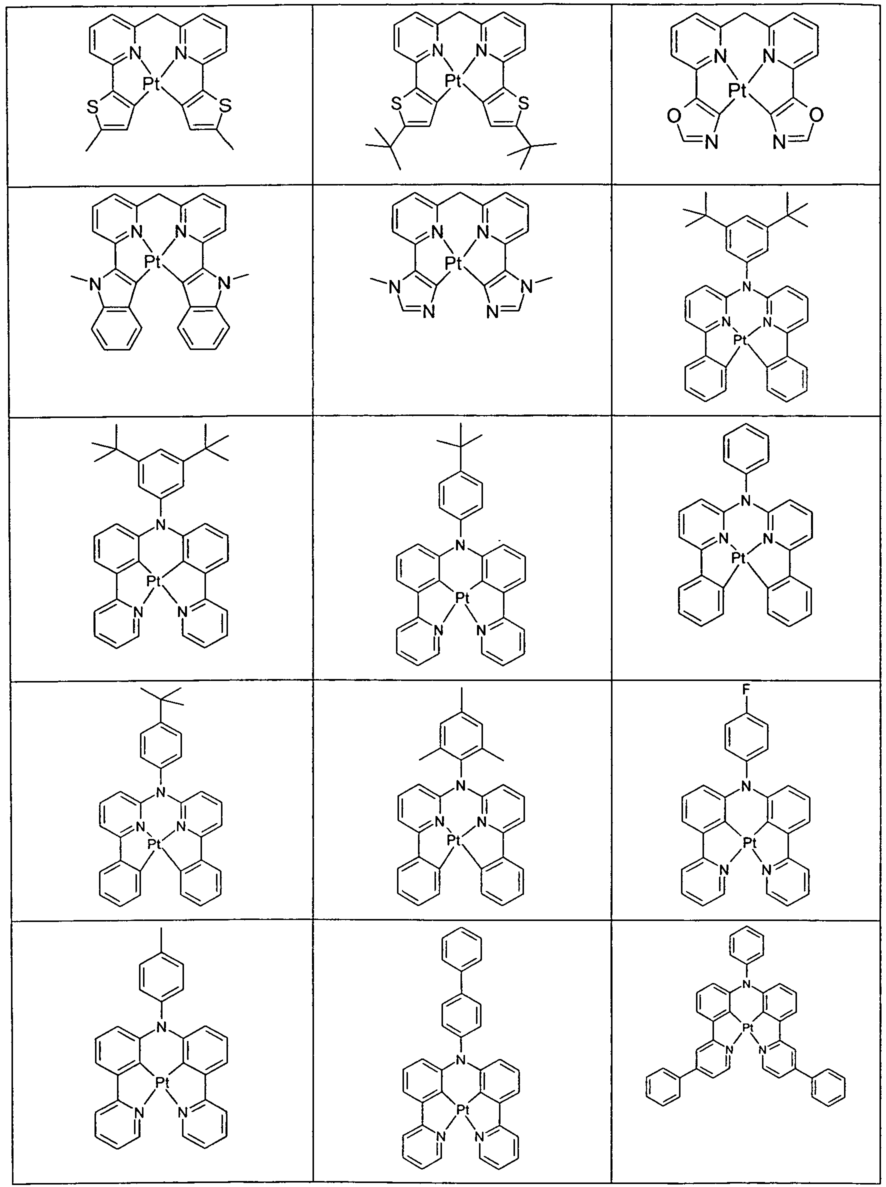

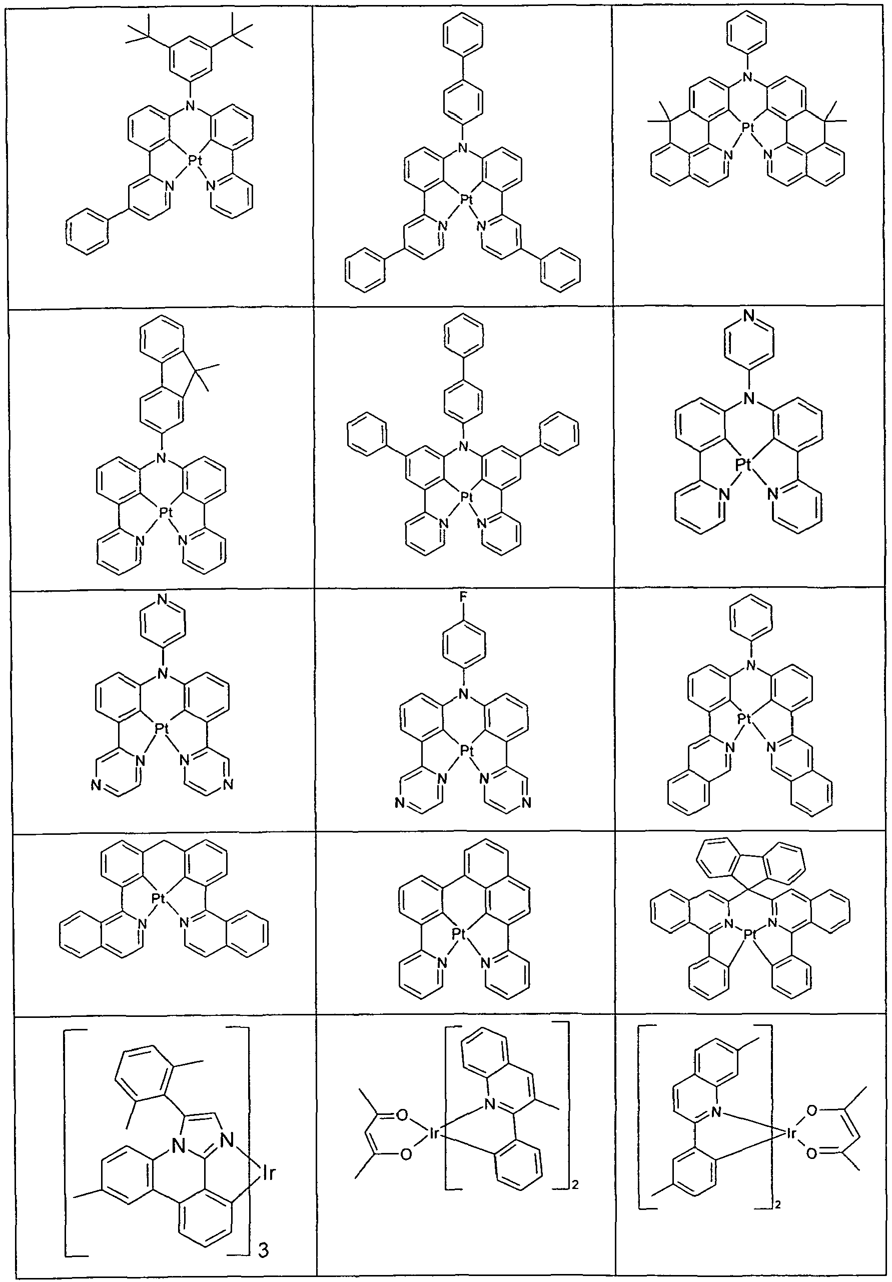

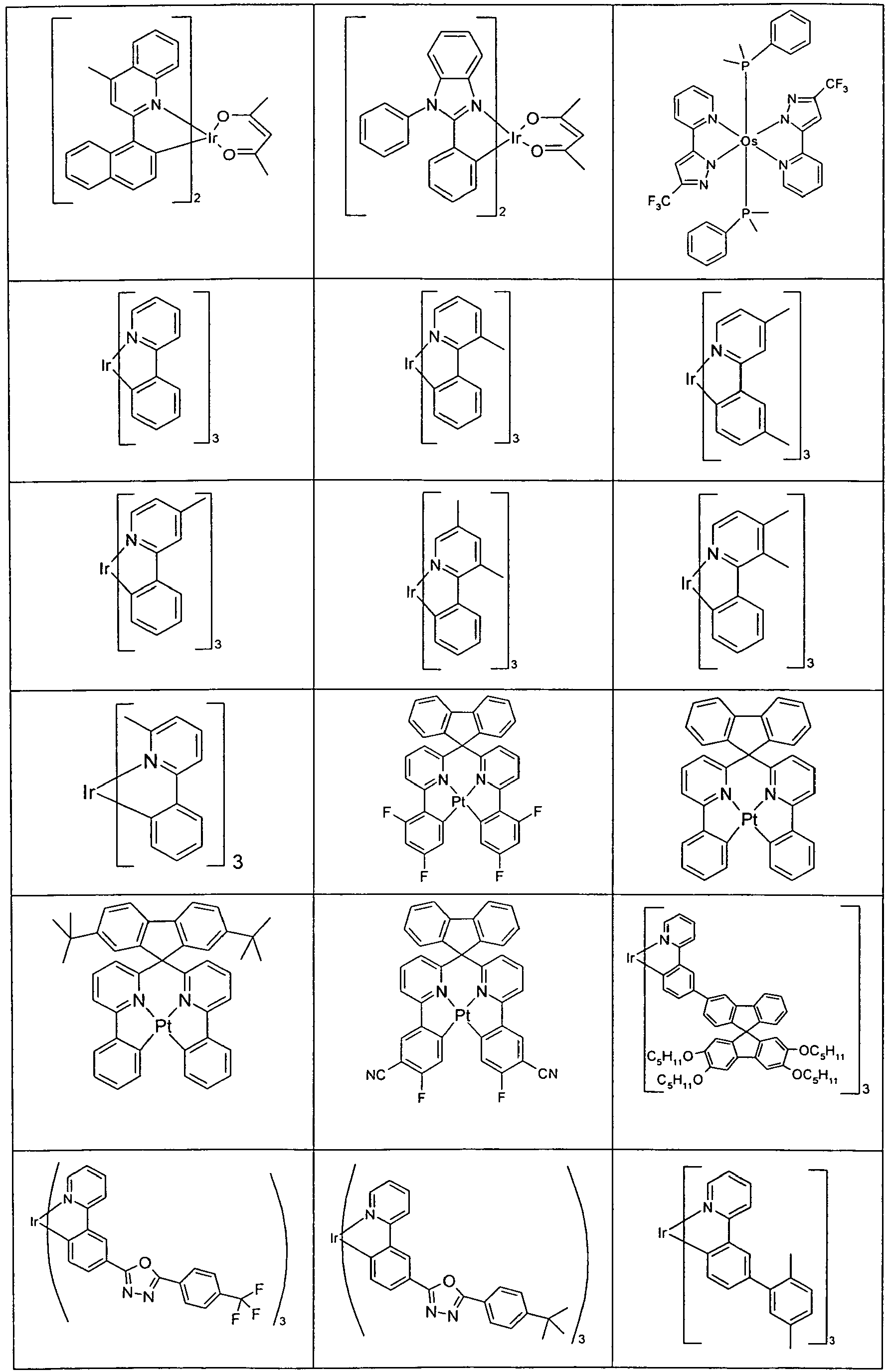

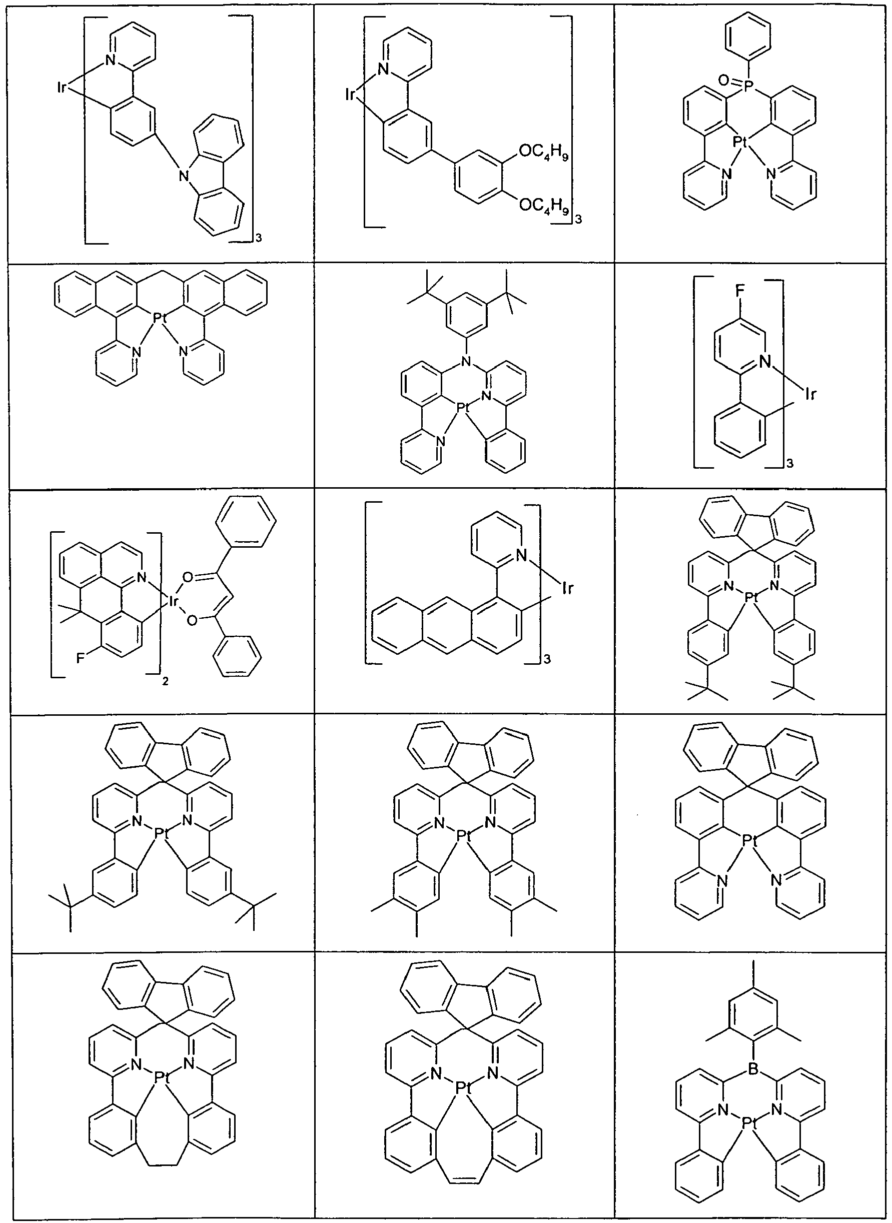

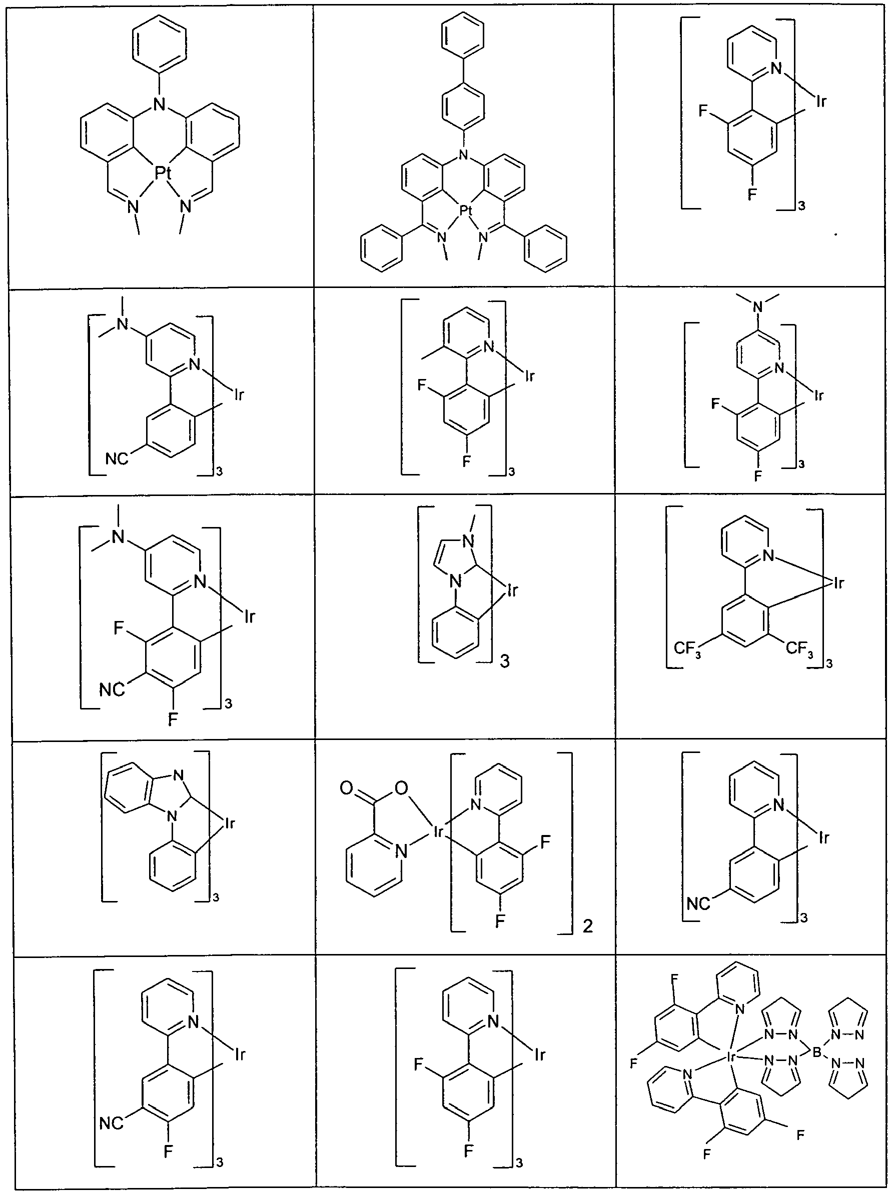

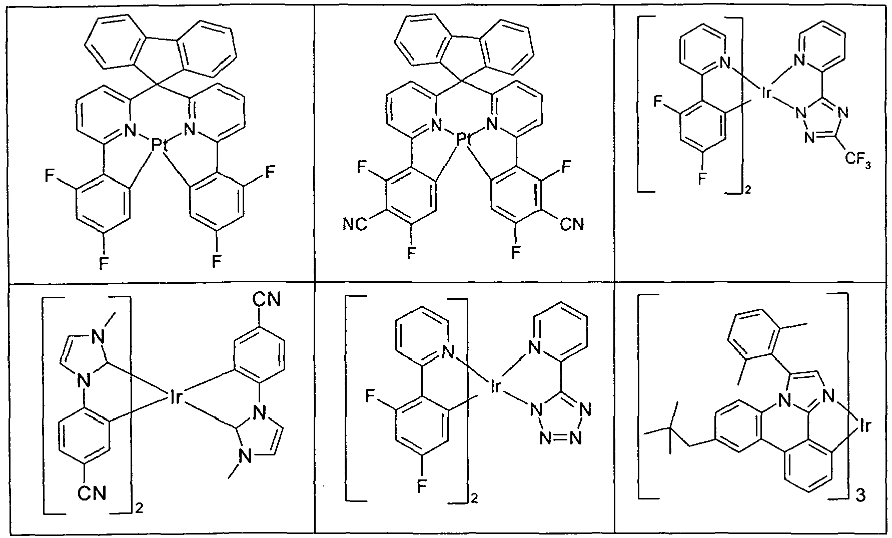

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte fluoreszierende Dotanden sind gewählt aus Indenofluorenaminen bzw. -diaminen, beispielsweise gemäß WO 2006/122630, Benzoindenofluorenaminen bzw. -diaminen, beispielsweise gemäß WO 2008/006449, und Dibenzoindenofluorenaminen bzw. -diaminen, beispielsweise gemäß WO 2007/140847. Beispiele für fluoreszierende Dotanden aus der Klasse der Styrylamine sind substituierte oder unsubstituierte Tristilbenamine oder die fluoreszierenden Dotanden, die in WO 2006/000388, WO 2006/058737, WO 2006/000389, WO 2007/065549 und WO 2007/115610 beschrieben sind. Weiterhin bevorzugt sind die in WO 2010/012328 offenbarten kondensierten Kohlenwasserstoffe.

Geeignete fluoreszierende Dotanden sind weiterhin die in JP 2006/001973, WO 2004/047499, WO 2006/098080, WO 2007/065678, US 2005/0260442 und WO 2004/092111 offenbarten Derivate.

Bevorzugte Matrixmaterialien für phosphoreszierende Dotanden sind neben den erfindungsgemäßen Verbindungen Carbazolderivate (z. B. CBP (N,N-Biscarbazolylbiphenyl) oder Verbindungen gemäß WO 2005/039246, US 2005/0069729, JP 2004/288381, EP 1205527 oder WO 2008/086851), Triarylamine, Azacarbazole (z. B. gemäß EP 1617710, EP 1617711, EP 1731584, JP 2005/347160), Indolocarbazolderivate, z. B. gemäß WO 2007/063754 oder WO 2008/056746, Ketone (z. B. gemäß WO 2004/093207 oder WO 2010/006680), Phosphinoxide, Sulfoxide und Sulfone (z. B. gemäß WO 2005/003253), Oligophenylene, aromatische Amine (z. B. gemäß US 2005/0069729), bipolare Matrixmaterialien (z. B. gemäß WO 2007/137725), Silane (z. B. gemäß WO 2005/111172), Azaborole oder Boronester, z. B. gemäß WO 2006/117052, Triazinderivate, z. B. gemäß WO 2010/015306, WO 2007/063754 oder WO 2008/056746, Zinkkomplexe (z. B. gemäß WO 2009/062578) Aluminiumkomplexe (z. B. BAIq), Diazasilol- und Tetraazasilol-Derivate, z. B. gemäß WO 2010/054730, Indenocarbazolderivate, z. B. gemäß WO 2010/136109 und WO 2011/000455 oder Diazaphosphole, z. B. gemäß WO 2010/054730.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Ba/Ag oder Mg/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Die organischen Elektrolumineszenzvorrichtungen enthaltend eine oder mehrere erfindungsgemäße Verbindungen können in Displays, als Lichtquellen in Beleuchtungsanwendungen sowie als Lichtquellen in medizinischen und/oder kosmetischen Anwendungen (z.B. Lichttherapie) eingesetzt werden.

Die erfindungsgemäßen Verbindungen und die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen eignen sich sehr gut für den Einsatz in einer Elektronentransportschicht einer organischen Elektrolumineszenzvorrichtu ng.
2. Die erfindungsgemäßen Verbindungen, eingesetzt als Matrixmaterial für fluoreszierende oder phosphoreszierende Emitter, führen zu sehr hohen Effizienzen sowie zu langen Lebensdauern. Dies gilt insbesondere, wenn die Verbindungen als Matrixmaterial für einen phosphoreszierenden Emitter eingesetzt werden.
3. Die erfindungsgemäßen Verbindungen eignen sich nicht nur als Matrix für rot und grün phosphoreszierende Verbindungen, sondern auch für blau phosphoreszierende Verbindungen.
4. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatz- und Betriebsspannungen.
5. Die erfindungsgemäßen Verbindungen weisen eine hohe Temperaturstabilität sowie eine hohe Oxidationsstabilität in Lösung auf und sind somit gut prozessierbar.

Die Erfindung wird durch die nachfolgenden Anwendungsbeispiele näher erläutert, wobei die Erfindung nicht auf den Umfang der Beispiele beschränkt ist.

### Ausführungsbeispiele

### A) Synthesebeispiele

Die nachfolgenden Synthesen werden, sofern nicht anders angegeben, unter einer Schutzgasatmosphäre in getrockneten Lösungsmitteln durchgeführt. Die erfindungsgemäßen Verbindungen können mittels dem Fachmann bekannten Syntheseverfahren dargestellt werden.

Als Ausgangsmaterialien können beispielsweise 3-(Bromphenyl)-1-phenyl-2-propen-1-on (Chemistry & Biodiversity, 2005, 2(12), 1656-1664), 2-(3-Bromphenyl)-4,6-diphenyl-pyrimidin, 4-(3-Bromphenyl)-4,6-diphenyl-pyrimidin (WO 2005/085387) und 9,10-Dihydro-9,9'-dimethylacridin (Chem. Ber. 1980,113(1), 358-384) dienen.

### I) Synthese des Zwischenprodukts 1a und der analogen Verbindungen 2a-9a

### 1. Stufe:

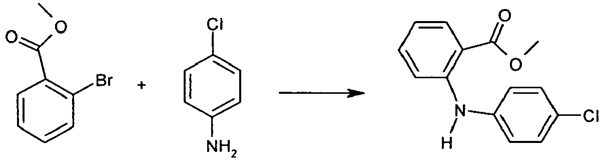

149 g (687 mmol) 2-Brombenzoesäuremethylester und 100 g (784 mmol) 4-Chloranilin werden in 1500 mL Toluol gelöst und durch Inertgaseinleitung entgast. Es wird anschließend mit entgasten 271 g (825 mmol) Cs₂CO₃, 15.4 g (69 mmol) Pd(OAc)₂ und 13.9 g (23 mmol) 4,5-Bis(diphenylphosphino)-9,9-dimethylxanthen versetzt. Die Reaktionsmischung wird anschließend für 6 h bei 82 °C gerührt und über Alox (basisch, Aktivitätsstufe 1) filtriert. Das Produkt wird via Säulenchromatographie an Kieselgel mit Heptan/Toluol (1:49) gereinigt. Man erhält 78.7 g (300 mmol, 43%) des Produkts als hellgelben Feststoff.

### 2. Stufe:

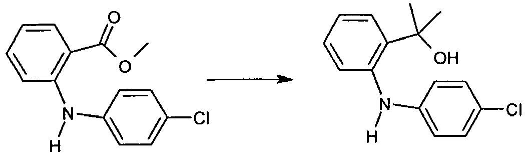

89.5 g (341 mmol) 2-(4-Chlor-phenylamino)-carboxymethylester werden in 1000 mL trockenem THF vorgelegt, bei -78°C mit 1367 mL einer 2M Lösung von MeLi in Et₂O (503 mmol) versetzt und dann innerhalb von 3h auf -40°C erwärmt. Nach vollständigem Umsatz wird bei -30°C langsam und vorsichtig mit 300 mL MeOH zum Quenchen des überschüssigen MeLi versetzt. Man lässt auf Raumtemperatur kommen, engt auf 1/3 ein, versetzt mit Essigsäureethylester und wäscht die organische Phase mit Wasser. Anschließend wird die organische Phase über MgSO₄ getrocknet und eingeengt. Man erhält 72 g (276 mmol, 80 %) des Produkts als Feststoff.

### 3. Stufe:

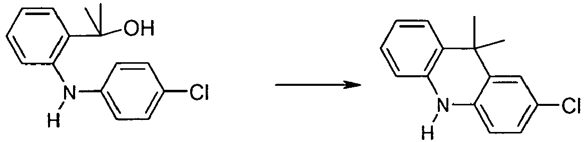

72.3 g (276 mmol) 2-[2-(4-Chlor-phenylamino)-phenyl]-propan-2-ol werden in 1000 mL Dichloromethan gelöst und durch Inertgaseinleitung entgast. Bei RT wird mit einem Gemisch aus 178 g Polyphosphorsäure und 118 mL Methansulfonsäure tropfenweise versetzt und auf 50 °C erhitzt. Nach vollständiger Abreaktion (ca. 30 min) wird die Reaktionslösung unter gutem Kühlen vorsichtig mit 20%iger NaOH-Lösung versetzt, bis sich ein pH-Wert von 8 eingestellt hat. Die organische Phase wird abgetrennt und die Wasserphase mit Toluol extrahiert. Anschließend wird die vereinigte organische Phase über MgSO₄ getrocknet und eingeengt. Man erhält 62 g (270 mmol) des Produkts als Feststoff (98%).

### 4. Stufe:

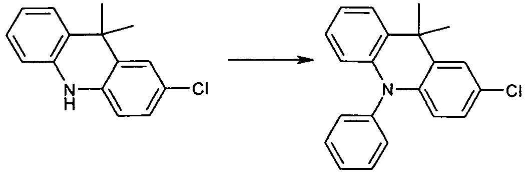

46 g (188.7 mmol) 2-Chlor-9,9-dimethyl-9,10-dihydroacridin und 46 g (226 mmol) Iodbenzol werden in Toluol gelöst und via Schutzgaseinleitung entgast. Anschließend wird mit 13 mL (13 mmol / 1 M Lösung in Toluol) Tri-tert-butylphosphin, 2.1g (9.4 mmol) Pd(OAc)₂ und 27 g (283 mmol) NaOtBu versetzt. Die Feststoffe werden zuvor entgast, die Reaktionsmischung wird nachentgast und anschließend unter Rückfluss für 5 h gerührt. Die warme Reaktionslösung wird über Alox B (Aktivitätsstufe 1) filtriert, mit Wasser gewaschen, getrocknet und eingeengt. Nach Kristallisation aus Toluol erhält man 51 g (166 mmol, 85%) der Verbindung **1a** als weißen Feststoff.

Analog werden die Verbindungen **2a-9a** erhalten:

| **Bsp.** | **Edukt** 1 | **Produkt** | **Ausbeute** |
|---|---|---|---|
| **2a** | 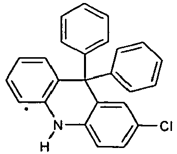 | 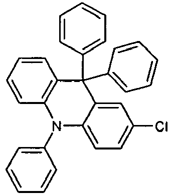 | 88% |
| | **21971-81-3** | | |
| **3a** | 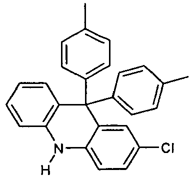 | 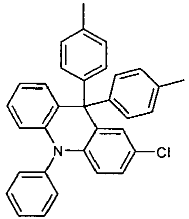 | 69% |
| | **22074-59-5** | | |
| **4a** | 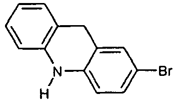 | 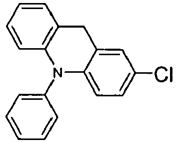 | 63% |
| | **1200798-49-7** | | |
| **5a** | 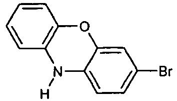 | 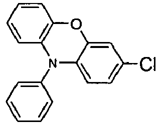 | 87% |
| | **832734-15-3** | | |
| **6a** | 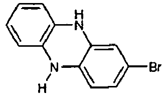 | 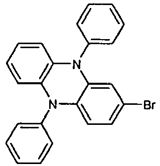 | 73% |
| | **496269-44-4** | | |
| **7a** | 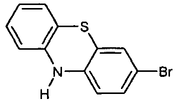 | 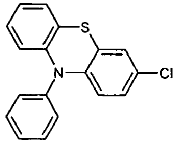 | 63% |
| | **63245-51-2** | | |
| **8a** | 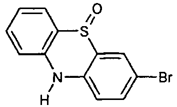 | 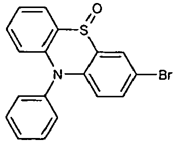 | 69% |
| | **197861-19-1** | | |
| **9a** | 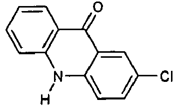 | 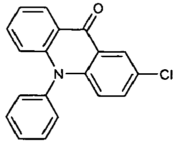 | 59% |
| | **7497-52-1** | | |

### II) Synthese des Zwischenprodukts 1b und der analogen Verbindungen 2b-9b

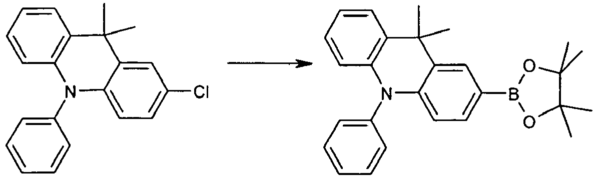

51 g (161 mmol) 2-Chlor-9,9-dimethyl-10-phenyl-9,10-dihydro-acridin (Verbindung 1a), 53.3 g (210 mmol) Bis(pinacolato)diboran und 26 g (274 mmol) Kaliumacetat werden in 800 ml Dioxan suspendiert. Zu dieser Suspension werden 11.9 g (16.1 mmol) 1,1-Bis-(diphenylphosphino)ferrocen-dichloropalladium(II)-Komplex mit Dichlormethan gegeben. Die Reaktionsmischung wird 16 h unter Rückfluss erhitzt. Nach Erkalten wird die organische Phase abgetrennt, dreimal mit 150 mL Wasser gewaschen und anschließend zur Trockne eingeengt. Der Rückstand wird aus Toluol umkristallisiert. Die Ausbeute beträgt 48 g (72 mmol, 90 %) an Verbindung **1b.**

Analog werden die Verbindungen **2b-8b** erhalten:

| **Bsp.** | **Edukt 1** | **Produkt** | **Ausbeute** |
|---|---|---|---|
| **2b** | 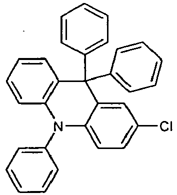 | 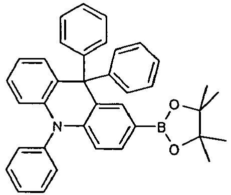 | 88 % |
| | **2a** | | |
| **3b** | 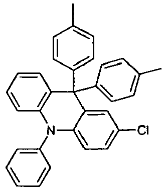 | 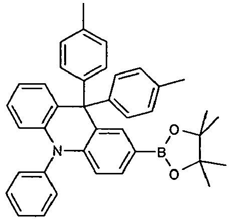 | 69% |
| | **3a** | | |
| **4b** | 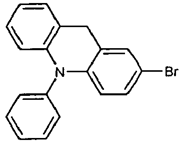 | 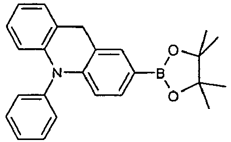 | 63% |
| | **4a** | | |
| **5b** | 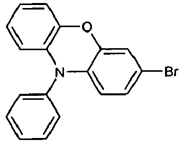 | 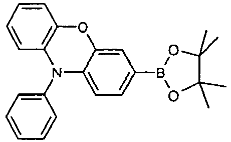 | 87% |
| | **5a** | | |
| **6b** | 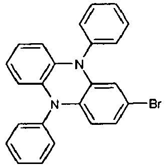 | 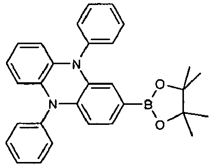 | 85% |
| | **6a** | | |
| **7b** | 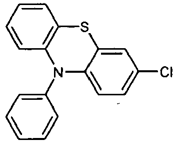 | 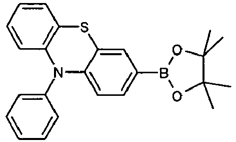 | 78% |
| | **7a** | | |
| **8b** | 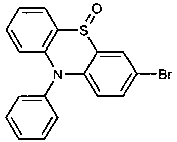 | 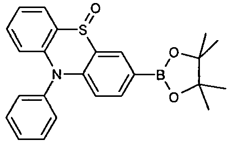 | 69% |
| | **8a** | | |
| **9b** | 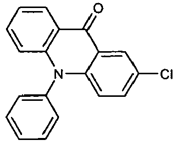 | 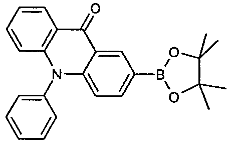 | 77% |
| | **9a** | | |

### III) Synthese der Zwischenprodukte A-C

### Zwischenprodukt A: 4-(2-Brom-phenyl)-2,6-diphenyl-pyrimidin

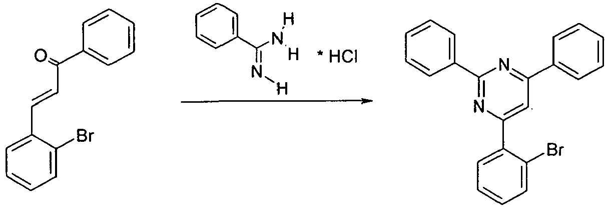

23 g (409 mmol) Kaliumhydroxid werden in 500 mL Ethanol gelöst, bei Raumtemperatur mit 40 g (255 mmol) Benzamid-hydrochlorid und 129 g (452 mmol) (3-(Bromphenyl)-1-phenyl-2-propen-1-on, gelöst in 500 ml Ethanol, versetzt und 3 h unter Rückfluss gerührt. Nach Kühlung auf Raumtemperatur wird der ausgefallene Feststoff abgesaugt, mit etwas Ethanol gewaschen und getrocknet. Es verbleiben 55 g (129 mmol), 50 % des Produkts in Form farbloser Kristalle.

### Zwischenprodukt B: (3'-Brom-biphenyl-3-yl)-phenyl-methanon

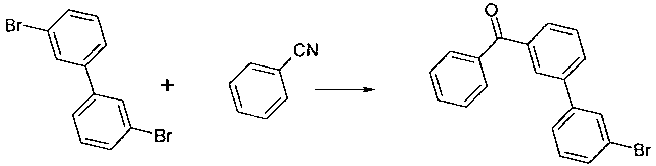

Aus einer Lösung von 31.5 g (101 mmol) 3,3'-Dibrom-biphenyl und 1 ml 1,2-Dichlorethan in 30 ml 1,2-Dimethoxyethan und 300 ml THF und 2.8 g (115 mmol) Magnesium wird in der Siedehitze das entsprechende Grignard-Reagenz hergestellt. Zu dieser Grignard-Lösung wird bei 0-5 °C eine Lösung von 10.4 g (101 mmol) Benzonitril in einer Mischung aus 130 ml THF und 130 ml Toluol während 20 min zugetropft. Anschließend wird die Mischung 16 h unter Rückfluss erhitzt. Nach Abkühlen wird die Reaktionsmischung bis zur Trockne eingeengt. Der Feststoff wird in 1000 ml NMP aufgenommen und mit 40 ml Wasser und 2 ml Eisessigsäure 12 h auf Rückfluss erhitzt. Es wird eine Mischung aus 600 ml Methanol und 600 ml 1N Salzsäure zugesetzt und der ausfallende Feststoff über Filtration abgetrennt und getrocknet. Das Rohprodukt wird aus Toluol/ Heptan umkristallisiert. Die Ausbeute bei einer Reinheit >98 % nach HPLC beträgt 27.1 g (80.5 mmol), entsprechend 80 % der Theorie.

### Zwischenprodukt C: Bis-(3'-brom-biphenyl-3-yl)-methanon

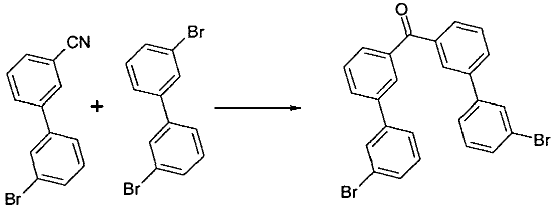

Aus einer Lösung von 31.5 g (101 mmol) 3,3'-Dibrom-biphenyl, 1 ml 1,2-Dichlorethan und 30 ml 1,2-Dimethoxyethan in 300 ml THF und 2.8 g (115 mmol) Magnesium wird in der Siedehitze das entsprechende Grignard-Reagenz hergestellt. Zu dieser Grignard-Lösung wird bei 0-5 °C eine Lösung von 26.06 g (101 mmol) 3-Brom-3'-cyano-biphenyl in einer Mischung aus 130 ml THF und 130 ml Toluol während 20 min zugetropft. Anschließend wird die Mischung 16 h unter Rückfluss erhitzt. Nach Abkühlen wird die Reaktionsmischung bis zur Trockene eingeengt. Der Feststoff wird in 1100 ml NMP aufgenommen und mit 40 ml Wasser und 5 ml Eisessig 24 h unter Rückfluss erhitzt. Es wird eine Mischung aus 600 ml Methanol und 600 ml 1 N Salzsäure zugesetzt und der ausfallende Feststoff über Filtration abgetrennt und getrocknet. Das Rohprodukt wird dreimal aus Toluol/Heptan umkristallisiert. Die Ausbeute bei einer Reinheit > 97 % nach HPLC beträgt 34.8 g (70.7 mmol) entsprechend 70.1 % der Theorie.

### IV) Synthese der Beispielverbindung 1 und der analogen Verbindungen 2-12

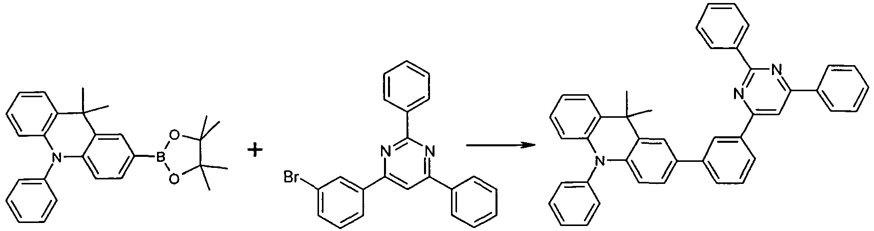

16 g (43.3 mmol) 2-(3-Bromphenyl)-4,6-diphenyl-pyrimidin ([864377-28-6]) und 19.3 g (48 mmol) 7-Boronsäure-12,12-dimethyl-10-phenyl-10,12-dihydro-10-aza-indeno[2,1-b]fluoren (Verbindung **1b**) werden in 80 mL Toluol gelöst und entgast. Es wird mit 281 mL einer entgasten 2M K₂CO₃-Lösung und mit 2.5 g (2.2 mmol) Pd(OAc)₂ versetzt. Die Reaktionsmischung wird anschließend bei 80 °C für 48 h unter Schutzgasatmosphäre gerührt. Die abgekühlte Lösung wird mit Toluol verdünnt, mehrmals mit Wasser gewaschen, getrocknet und eingeengt. Das Produkt wird via Säulenchromatographie an Kieselgel mit Toluol/Heptan (1:2) gereinigt. Die Reinheit beträgt 99.9 %. Ausbeute: 22 g (31 mmol, 77 %) der Theorie.

Analog werden die Verbindungen **2-12** erhalten:

| **Bsp.** | **Edukt 1** | **Edukt 2** | **Produkt** | **Ausbeute** |
|---|---|---|---|---|
| **2** | 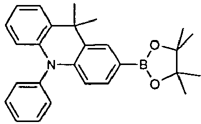 | 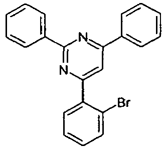 | 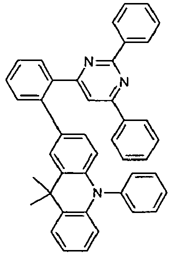 | 69% |
| | **1b** | **A** | | |
| **3** | 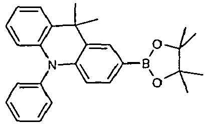 | 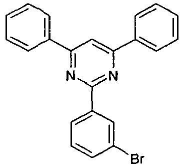 | 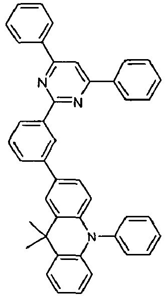 | 79% |
| | **1b** | | | |
| **4** | 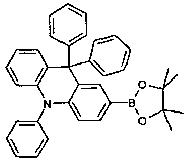 | 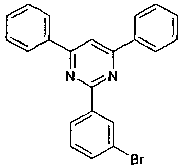 | 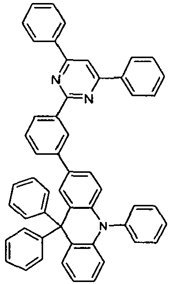 | 77% |
| | **2b** | | | |
| **5** | 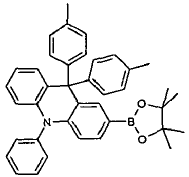 | 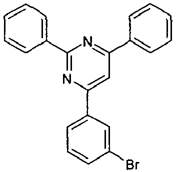 | 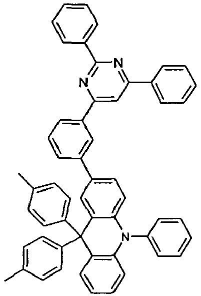 | 78% |
| | **3b** | **864377-28-6** | | |
| **6** | 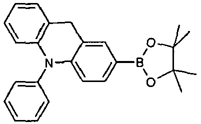 | 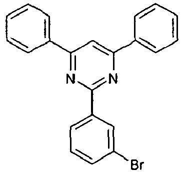 | 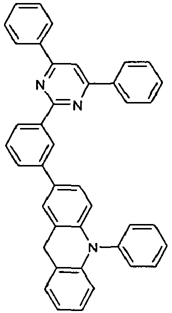 | 85% |
| | **4b** | | | |
| **7** | 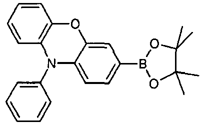 | 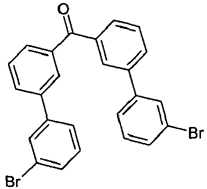 | 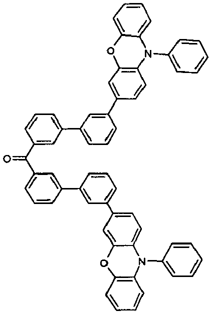 | 65% |
| | **5b** | C | | |
| **8** | 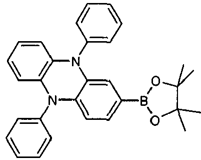 | 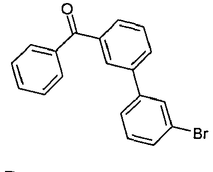 | 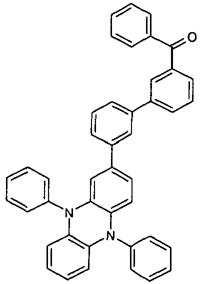 | 91% |
| | **6b** | **B** | | |
| **9*** | 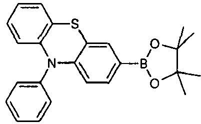 | 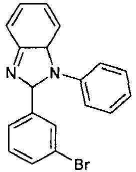 | 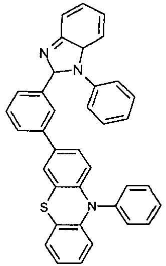 | 88% |
| | **7b** | **760212-40-6** | | |
| **10*** | 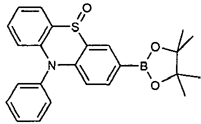 | 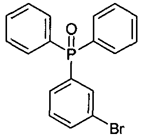 | 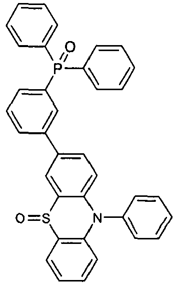 | 74% |
| | **8b** | **10212-04-1** | | |
| **11*** | 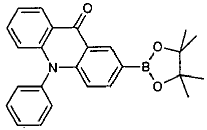 | 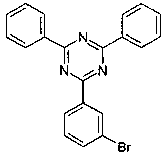 | 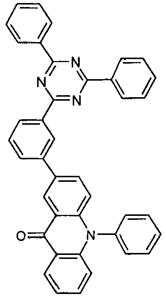 | 83% |
| | **9b** | **864377-31-1** | | |
| **12** | 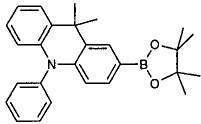 | 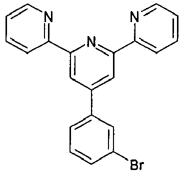 | 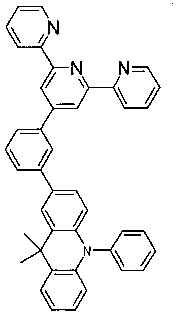 | 68% |
| | **1b** | **879879-65-9** | | |

| | | | | |
|---|---|---|---|---|
| **Nicht gemäß der Erfindung* | | | | |

### V) Synthese der Beispielverbindung 13 und der analogen Verbindungen 14-24

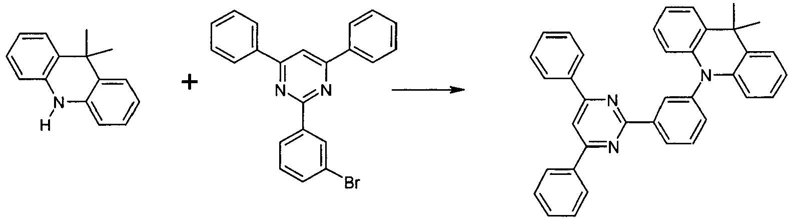

Unter Schutzgas werden 16.7 g (79.8 mmol) 9,10-Dihydro-9,9'-dimethyl-acridin, 34 g (87 mmol) 2-(3-Bromo-phenyl)-4,6-diphenyl-pyrimidin, 15.9 ml (15.9 mmol) 1 mol/l Tri-tert-butylphosphin und 1.79 g (7.9 mmol) Palladiumacetat in 120 ml p-Xylol suspendiert. Die Reaktionsmischung wird 16 h unter Rückfluss erhitzt. Nach Erkalten wird die organische Phase abgetrennt, dreimal mit 200 mL Wasser gewaschen und anschließend zur Trockne eingeengt. Der Rückstand wird mit Toluol heiß extrahiert, aus Toluol umkristallisiert und abschließend im Hochvakuum sublimiert. Die Reinheit beträgt 99.9 %. Ausbeute: 34 g (66 mmol, 83 % der Theorie).

Analog werden die Verbindungen **14-24** erhalten:

| **Bsp.** | **Edukt 1** | **Edukt 2** | **Produkt** | **Ausbeute** |
|---|---|---|---|---|
| **14** | 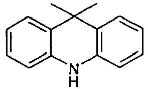 | 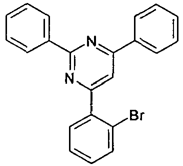 | 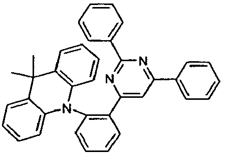 | 66% |
| | | **A** | | |
| **15** | 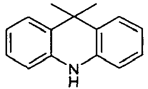 | 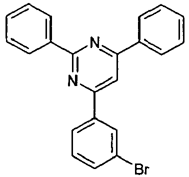 | 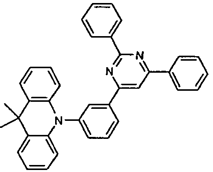 | 71% |
| | | **864377-28-6** | | |
| **16** | 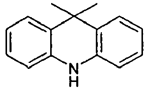 | 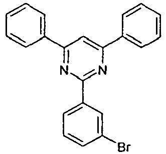 | 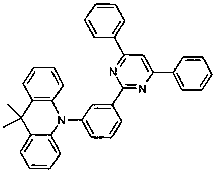 | 73% |
| **17** | 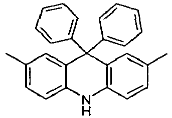 | 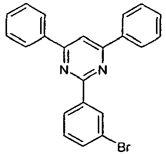 | 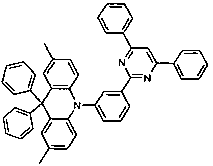 | 78% |
| | **92638-87-4** | | | |
| **18** | 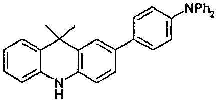 | 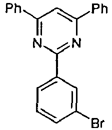 | 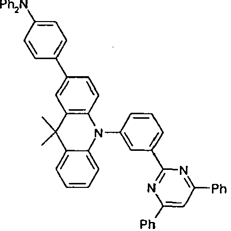 | 82% |
| | **1236106-99-2** | | | |
| **19*** | 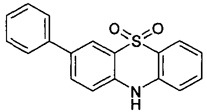 | 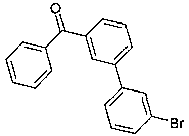 | 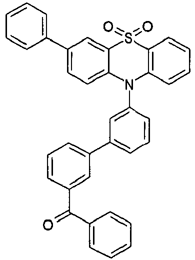 | 69% |
| | **888008-94-4** | **B** | | |
| **20*** | 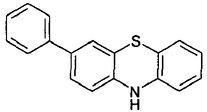 | 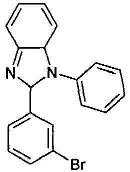 | 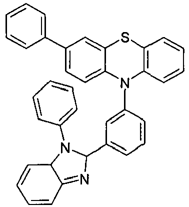 | 77% |
| | **4018-68-2** | **760212-40-6** | | |
| **21*** | 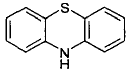 | 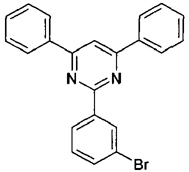 | 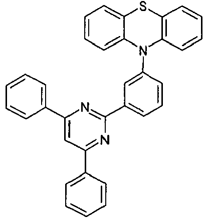 | 67% |
| | **92-84-2** | | | |
| 22 | 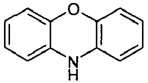 | 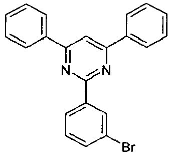 | 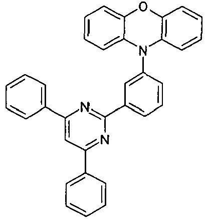 | 82% |
| | **135-67-1** | | | |
| **23** | 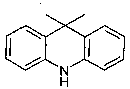 | 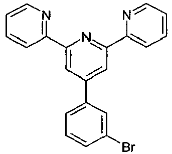 | 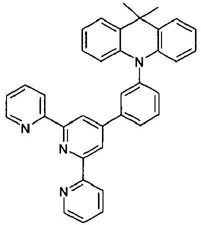 | 79% |
| | | **879879-65-9** | | |
| **24** | 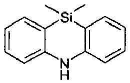 | 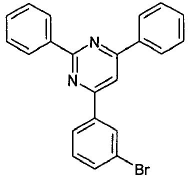 | 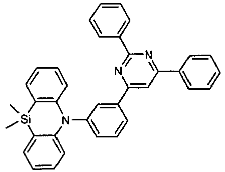 | 67% |
| | **89590-57-8** | **864377-28-6** | | |

| | | | | |
|---|---|---|---|---|
| **Nicht gemäß der Erfindung* | | | | |

### B) Devicebeispiele

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen V1 bis E14 (siehe Tabellen 1 und 2) werden die Daten verschiedener OLEDs vorgestellt. Glasplättchen, die mit strukturiertem ITO (Indium Zinn Oxid) der Dicke 150 nm beschichtet sind, werden zur verbesserten Prozessierung mit 20 nm PEDOT beschichtet (Poly(3,4-ethylendioxy-2,5-thiophen), aus Wasser aufgeschleudert; bezogen von H. C. Starck, Goslar, Deutschland). Diese beschichteten Glasplättchen bilden die Substrate, auf weiche die OLEDs aufgebracht werden. Die OLEDs haben prinzipiell folgenden Schichtaufbau: Substrat / Lochtransportschicht (HTL) / Optionale Zwischenschicht (IL) / Elektronenblockerschicht (EBL) / Emissionsschicht (EML) / Optionale Lochblockierschicht (HBL) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie ST1:H15:TEG1 (75%:15%:10%) bedeutet hierbei, dass das Material ST1 in einem Volumenanteil von 75%, H15 in einem Anteil von 15% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst. Die Beispiele V1 und V2 sind Vergleichsbeispiele mit Materialien gemäßt dem Stand der Technik, E1-E14 zeigen Daten von OLEDs mit erfindungsgemäßen Materialien.

Im folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt. Wie sich der Tabelle entnehmen lässt, werden auch bei Verwendung der nicht näher ausgeführten erfindungsgemäßen Verbindungen gute bis sehr gute Leistungsdaten erzielt.

### Verwendung von erfindungsgemäßen Verbindungen als Matrixmaterialien in phosphoreszierenden OLEDs

Die erfindungsgemäßen Materialien eignen sich vor allem zum Einsatz als Matrixmaterialien in phosphoreszierenden OLEDs, entweder als Einzelmaterial (Beispiele E1, E2, E8-E13) bzw. als Komponente in einem Mixed-Matrix System (Beispiele E3-E7). Es sind hierbei sehr gute Leistungsdaten erreichbar. Z.B. erhält man mit Verbindung H6 in Kombination mit dem Dotanden TEG1 eine Spannung von nur 3.7 V, eine externe Quanteneffizienz von 15.5% und eine sehr gute Leistungseffizienz von 48 Im/W (Beispiel E11). Betreibt man die entsprechende OLED mit konstanter Stromdichte, so fällt die anfängliche Leuchtdichte von 8000 cd/m² innerhalb 180 h auf 6400 cd/m² ab, was einem guten Wert entspricht.

### Vergleich mit dem Stand der Technik

Im Vergleich zur Verbindung H1 aus dem Stand der Technik erhält man bei Verwendung der Verbindungen H9 und H10 eine um bis zu 20% erhöhte Quanteneffizienz. Weiterhin ergibt sich ein deutlicher Vorteil bezüglich der Lebensdauer: Während man bei Betrieb mit konstanter Stromdichte für H1 einen Abfall der Leuchtdichte von 8000 cd/m² auf 6400 cd/m² innerhalb etwa 105 h beobachtet, wird dieser Wert für H9 erst nach ca. 125 h, für H10 nach ca. 140 h erreicht (Beispiele V1, E1 und E2). Verglichen mit H2 (Stand der Technik) zeigt sich eine deutliche Verbesserung in der Effizienz um etwas mehr als 25% mit dem erfindungsgemäßen Material H12 (Beispiele V2, E4). Die Lebensdauer mit H12 ist etwa 15% höher.

Die erfindungsgemäßen Materialien ergeben bei Einsatz als Matrixmaterialien in phosphoreszierenden OLEDs somit wesentliche Verbesserungen gegenüber dem Stand der Technik, vor allem bezüglich Lebensdauer und Effizienz.

### Verwendung von erfindungsgemäßen Verbindungen als Elektronentransportmaterialien

Weiterhin lassen sich die erfindungsgemäßen Verbindungen in der Elektronentransportschicht einsetzen. Z.B. werden mit der Verbindung H7 und einer 3 nm dicken Schicht LiQ als Elektroneninjektionsmaterial eine Spannung von 3.9 V und eine Quanteneffizienz von 15.3% erhalten (Beispiel E14). Für dieses Beispiel wird weiterhin eine sehr gute Lebensdauer gemessen: Bei Betrieb mit konstanter Stromdichte fällt die Leuchtdichte innerhalb von etwa 220 h von 8000 cd/m² auf 6400 cd/m² ab.

**Tabelle 1: Aufbau der OLEDs**

| Bsp. | HTL Dicke | IL Dicke | EBL Dicke | EML Dicke | HBL Dicke | ETL Dicke | EIL Dicke |
|---|---|---|---|---|---|---|---|
| V1 | SpA1 | HATCN | BPA1 | H1:TEG1 (90%:10%) | ST1 | ST2:LiQ (50%:50%) | --- |
| | 70nm | 5nm | 90nm | 30nm | 10nm | 30nm | |
| V2 | SpA1 | HATCN | BPA1 | ST1:H2:TEG1 | ST1 | ST2:LiQ (50%:50%) | --- |
| | 70nm | 5nm | 90nm | (75%:15%:10%) 30nm | 10nm | 30nm | |
| E1 | SpA1 | HATCN | BPA1 | H9:TEG1 (90%:10%) | ST1 | ST2:LiQ (50%:50%) | --- |
| | 70nm | 5nm | 90nm | 30nm | 10nm | 30nm | |
| E2 | SpA1 | HATCN | BPA1 | H10:TEG1 (90%:10%) | ST1 | ST2:LiQ (50%:50%) | --- |
| | 70nm | 5nm | 90nm | 30nm | 10nm | 30nm | |
| E3 | SpA1 | --- | NPB | ST1:H11:TER1 | ST1 | Alq₃ | LiF |
| | 20nm | | 20nm | (70%:20%:10%) 30nm | 30nm | 20nm | 1nm |
| E4 | SpA1 | HATCN | BPA1 | ST1:H12:TEG1 | ST1 | ST2:LiQ (50%:50%) | --- |
| | 70nm | 5nm | 90nm | (75%:15%:10%) 30nm | 10nm | 30nm | |
| E5 | SpA1 | --- | NPB | ST1:H13:TER1 | ST1 | Alq₃ | LiF |
| | 20nm | | 20nm | (75%:15%:10%) 30nm | 30nm | 20nm | 1nm |
| E6 | SpA1 | --- | NPB | ST1:H14:TER1 | ST1 | Alq₃ | LiF |
| | 20nm | | 20nm | (75%:15%:10%) 30nm | 30nm | 20nm | 1nm |
| E7 | SpA1 | HATCN | BPA1 | ST1:H15:TEG1 | IC1 | ST2:LiQ (50%:50%) | --- |
| | 70nm | 5nm | 90nm | (75%:15%:10%) 30nm | 10n | 30nm | |
| E8 | SpA1 | HATCN | BPA1 | H3:TEG1 (90%:10%) | IC1 | ST2:LiQ (50%:50%) | --- |
| | 70nm | 5nm | 90nm | 30nm | 10nm | 30nm | |
| E9 | SpA1 | HATCN | BPA1 | H4:TEG1 (90%:10%) | IC1 | ST2:LiQ (50%:50%) | --- |
| | 70nm | 5nm | 90nm | 30nm | 10nm | 30nm | |
| E10 | SpA1 | HATCN | BPA1 | H5:TEG1(90%:10%) | IC1 | ST2:LiQ (50%:50%) | --- |
| | 70nm | 5nm | 90nm | 30nm | 10nm | 30nm | |
| E11 | SpA1 | HATCN | BPA1 | H6:TEG1 (90%:10%) | IC1 | ST2:LiQ (50%:50%) | --- |
| | 70nm | 5nm | 90nm | 30nm | 10n | 30nm | |
| E12 | SpA1 | HATCN | BPA1 | H7:TEG1 (90%:10%) | IC1 | ST2:LiQ (50%:50%) | --- |
| | 70nm | 5nm | 90nm | 30nm | 10nm | 30nm | |

| E13 | SpA1 70nm | HATCN 5nm | BPA1 90nm | H8:TEG1 (90%:10%) 30nm | IC1 10nm | ST2:LiQ (50%:50%) 30nm | --- |
|---|---|---|---|---|---|---|---|
| E14 | SpA1 | HATCN | BPA1 | IC1:TEG1 (90%:10%) | --- | H7 | LiQ |
| | 70nm | 5nm | 90nm | 30nm | | 40nm | 3nm |

**Tabelle 2: Daten der OLEDs**

| Bsp. | U1000 (V) | SE1000 (cd/A) | LE1000 (Im/W) | EQE 1000 | CIE x/y bei 1000 cd/m² |
|---|---|---|---|---|---|
| V1 | 3.8 | 44 | 36 | 12.1% | 0.36/0.61 |
| V2 | 3.6 | 36 | 32 | 10.3% | 0.35/0.59 |
| E1 | 3.9 | 50 | 41 | 14.0% | 0.36/0.61 |
| E2 | 3.7 | 53 | 45 | 14.7% | 0.36/0.61 |
| E3 | 4.3 | 9.1 | 6.6 | 12.8% | 0.68/0.32 |
| E4 | 3.7 | 47 | 40 | 13.1% | 0.36/0.61 |
| E5 | 4.7 | 8.2 | 5.5 | 11.6% | 0.68/0.32 |
| E6 | 4.2 | 9.3 | 6.9 | 13.1% | 0.68/0.32 |
| E7 | 4.0 | 53 | 41 | 14.6% | 0.37/0.60 |
| E8 | 3.5 | 47 | 43 | 13.2% | 0.36/0.61 |
| E9 | 3.3 | 57 | 54 | 15.8% | 0.36/0.61 |
| E10 | 4.1 | 54 | 41 | 15.0% | 0.36/0.60 |
| E11 | 3.7 | 56 | 48 | 15.5% | 0.36/0.61 |
| E12 | 4.2 | 45 | 34 | 12.5% | 0.36/0.61 |
| E13 | 3.9 | 51 | 41 | 14.3% | 0.36/0.61 |
| E14 | 3.9 | 55 | 44 | 15.3% | 0.37/0.60 |

**Tabelle 3: Strukturformeln der Materialien für die OLEDs**

| | |
|---|---|
| 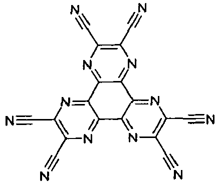 | 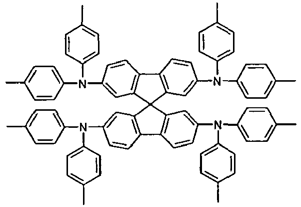 |
| HATCN | SpA1 |
| 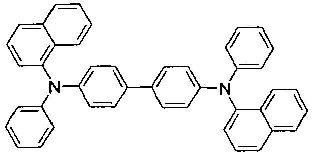 | 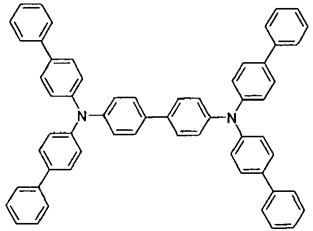 |
| NPB | BPA1 |
| 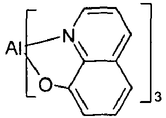 | 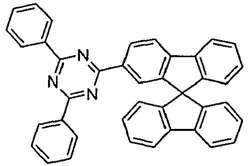 |
| Alq₃ | ST1 |
| 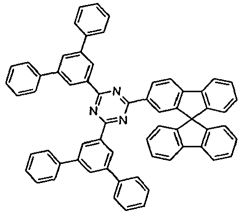 | 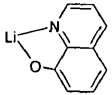 |
| ST2 | LiQ |
| 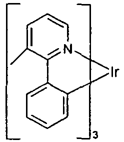 | 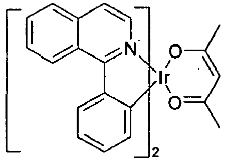 |
| TEG1 | TER1 |
| 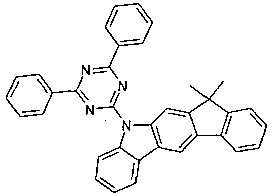 | 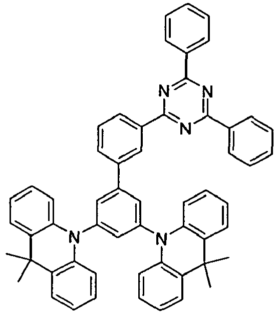 |
| IC1 | H1 (Stand der Technik) |
| 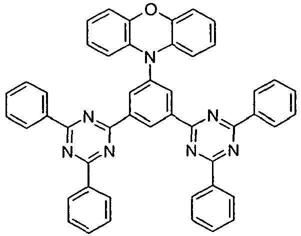 | 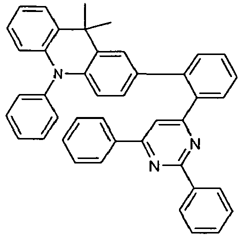 |
| H2 (Stand der Technik) | H3 (Beispielverbindung 2) |
| 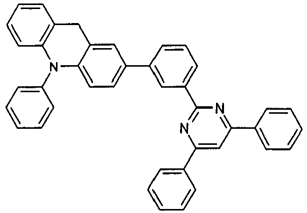 | 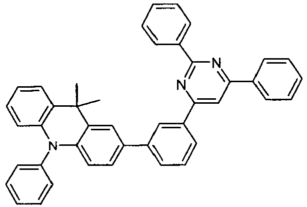 |
| H4 (Beispielverbindung 6) | H5 (Beispielverbindung 1) |
| 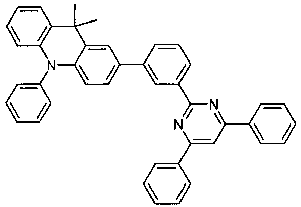 | 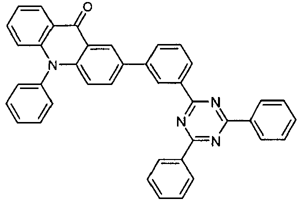 |
| H6 (Beispielverbindung 3) | H7 (Beispielverbindung 11)* |
| 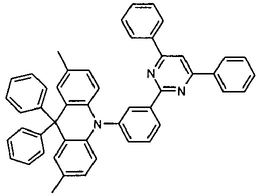 | 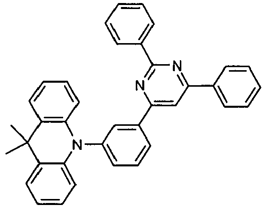 |
| H8 (Beispielverbindung 17) | H9 (Beispielverbindung 15) |
| 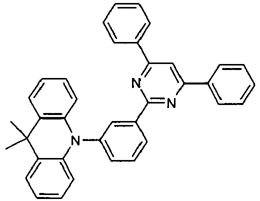 | 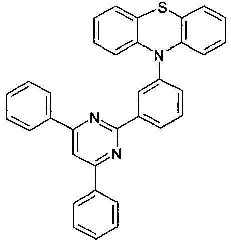 |
| H10 (Beispielverbindung 16) | H11 (Beispielverbindung 21)* |
| 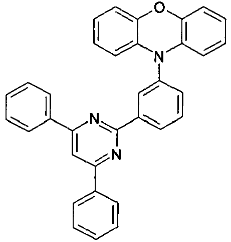 | 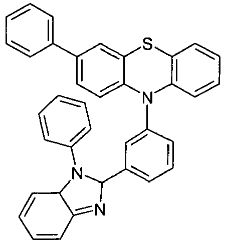 |
| H12 (Beispielverbindung 22) | H13 (Beispielverbindung 20)* |
| 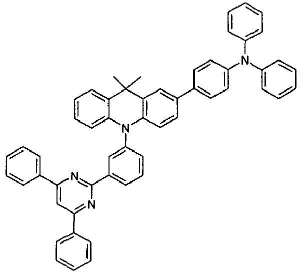 | 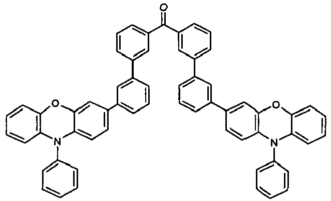 |
| H14 (Beispielverbindung 18) | H15 (Beispielverbindung 7) |

| | |
|---|---|
| **Nicht gemäß der Erfindung* | |

## Patentansprüche

1. Verbindung einer Formel (I) 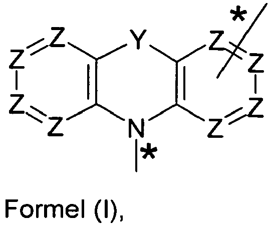 wobei über mindestens eine der mit * gekennzeichneten Bindungen eine Gruppe der Formel (II) gebunden ist 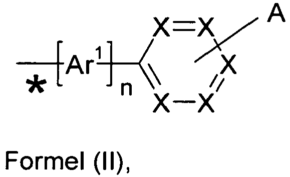 wobei * in Formel (II) wiederum die Bindung zur Einheit gemäß Formel (I) kennzeichnet, und
wobei in Formel (I) für den Fall, dass an der mit * gekennzeichneten Bindung keine Gruppe der Formel (II) gebunden ist, dort ein Rest R² gebunden ist,
und wobei für die auftretenden Symbole und Indices gilt:
A ist bei jedem Auftreten gleich oder verschieden
- eine Heteroarylgruppe mit 5 bis 20 aromatischen Ringatomen, welche mit einem oder mehreren Resten R¹ substituiert sein kann und welche mindestens einen heteroaromatischen Fünfring mit zwei oder mehr Heteroatomen ausgewählt aus N, O und S enthält oder mindestens einen heteroaromatischen Sechsring mit einem oder mehr Heteroatomen ausgewählt aus N, O und S enthält, oder
- eine Ketogruppe, welche mit einem oder mehreren Resten R¹ substituiert sein kann, oder
- eine Phosphoroxidgruppe, welche mit einem oder mehreren Resten R¹ substituiert sein kann, oder
- eine Schwefeloxidgruppe, welche mit einem oder mehreren Resten R¹ substituiert sein kann;
Y ist eine divalente Gruppe ausgewählt aus C(R²)₂, Si(R²)₂, NR² und O;
X ist bei jedem Auftreten gleich oder verschieden CR¹ oder N, bzw. ist C mit daran gebundener Gruppe A;
Z ist bei jedem Auftreten gleich oder verschieden CR² oder N;
Ar¹ ist ein aromatisches oder heteroaromatisches Ringsystem mit 5 bis 30 aromatischen Ringatomen, welches mit einem oder mehreren Resten R¹ substituiert sein kann;
R¹ ist bei jedem Auftreten gleich oder verschieden H, D, F, Cl, Br, I, B(OR³)₂, CHO, C(=O)R³, CR³=C(R³)₂, CN, C(=O)OR³, C(=O)N(R³)₂, Si(R³)₃, NO₂, P(=O)(R³)₂, OSO₂R³, OR³, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C=C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R³ substituiert sein kann, oder eine Aryloxygruppe mit 6 bis 30 aromatischen Ringatomen, die durch einen oder mehrere Reste R³ substituiert sein kann, wobei zwei oder mehr Reste R¹ miteinander verknüpft sein können und einen Ring bilden können;
R² ist bei jedem Auftreten gleich oder verschieden H, D, F, Cl, Br, I, B(OR³)₂, CHO, C(=O)R₃, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C=C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 60 aromatischen Ringatomen, die durch einen oder mehrere Reste R³ substituiert sein kann, wobei zwei oder mehr Reste R² miteinander verknüpft sein können und einen Ring bilden können;
R³ ist bei jedem Auftreten gleich oder verschieden H, D, F, Cl, Br, I, B(OR⁴)₂, CHO, C(=O)R⁴, CR⁴=C(R⁴)₂, CN, C(=O)OR⁴, C(=O)N(R⁴)₂, Si(R⁴)₃, N(R⁴)₂, NO₂, P(=O)(R⁴)₂, OSO₂R⁴, OR⁴, S(=O)R⁴, S(=O)₂R⁴, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C=C-, Si(R⁴)₂, C=O, C=S, C=Se, C=NR⁴, -C(=O)O-, -C(=O)NR⁴-, NR⁴, P(=O)(R⁴), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R⁴ substituiert sein kann, oder eine Aryloxy- oder Heteroaryloxygruppe mit 5 bis 60 aromatischen Ringatomen, die durch einen oder mehrere Reste R⁴ substituiert sein kann, wobei zwei oder mehr Reste R³ miteinander verknüpft sein können und einen Ring bilden können;
R⁴ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁴ miteinander verknüpft sein und einen Ring bilden;
n ist bei jedem Auftreten gleich oder verschieden 0, 1, 2 oder 3;
wobei Reste R² als Bestandteile von Gruppen Z keine an das Ringsystem von Formel (I) ankondensierten Ringe bilden dürfen; und
wobei weiterhin die Gruppe A in meta- oder in ortho-Position an den aromatischen Sechsring gebunden ist, wenn die Gruppe der Formel (II) an das Stickstoffatom in Formel (I) gebunden ist;
und wobei weiterhin die folgenden Verbindung ausgenommen ist:

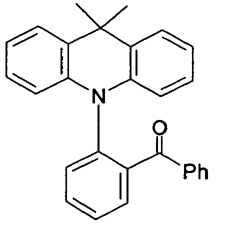

2. Verbindung nach Anspruch 1, **dadurch gekennzeichnet, dass** sie zusätzlich zu der in Formel (I) gezeigten Aminogruppe keine weitere Aminogruppe enthält.

3. Verbindung nach Anspruch 1 oder 2, **dadurch gekennzeichnet, dass** die Gruppe Ar¹ ein aromatisches Ringsystem mit 6 bis 18 aromatischen Ringatomen oder ein heteroaromatisches Ringsystem mit 5 bis 18 aromatischen Ringatomen darstellt, welche jeweils mit einem oder mehreren Resten R¹ substituiert sein können.

4. Verbindung nach einem oder mehreren der Ansprüche 1 bis 3, **dadurch gekennzeichnet, dass** nicht mehr als eine Gruppe X pro Gruppe der Formel (II) gleich N ist.

5. Verbindung nach einem oder mehreren der Ansprüche 1 bis 4, **dadurch gekennzeichnet, dass** nicht mehr als eine Gruppe Z pro aromatischem Sechsring gleich N ist.

6. Verbindung nach einem oder mehreren der Ansprüche 1 bis 5, **dadurch gekennzeichnet, dass** R¹ bei jedem Auftreten gleich oder verschieden ausgewählt ist aus H, D, F, CN, Si(R³)₃ oder einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, wobei die oben genannten Gruppen jeweils mit einem oder mehreren Resten R³ substituiert sein können und wobei in den oben genannten Gruppen eine oder mehrere CH₂-Gruppen durch -C≡C-, -R³C=CR³-, Si(R³)₂, C=O, C=NR³, -NR³-, -O-, -S-, -C(=O)O- oder -C(=O)NR³- ersetzt sein können, oder einem aromatischen Ringsystem mit 6 bis 24 aromatischen Ringatomen, welches mit einem oder mehreren Resten R³ substituiert sein kann, wobei zwei oder mehr Reste R¹ miteinander verknüpft sein können und einen Ring bilden können.

7. Verbindung nach einem oder mehreren der Ansprüche 1 bis 6, **dadurch gekennzeichnet, dass** diejenigen Gruppen R¹, welche nicht an eine Gruppe A binden, gleich H sind.

8. Verbindung nach einem oder mehreren der Ansprüche 1 bis 7, **dadurch gekennzeichnet, dass** die Gruppe A eine Gruppe der Formel (A-1) bis (A-9) darstellt: 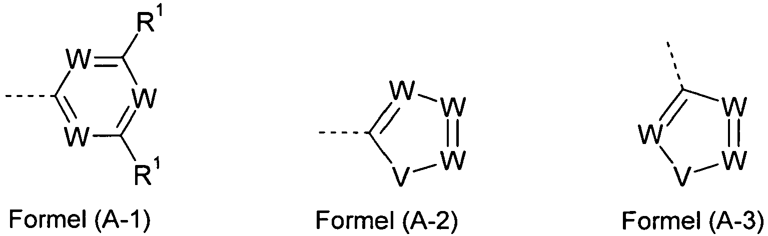 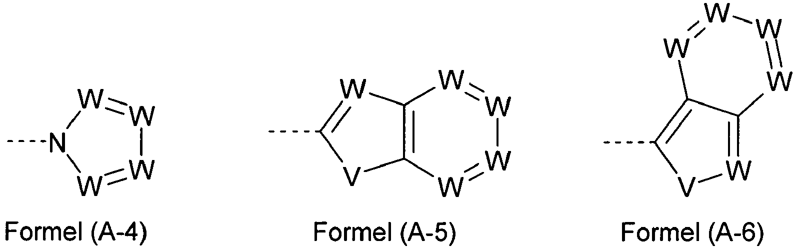 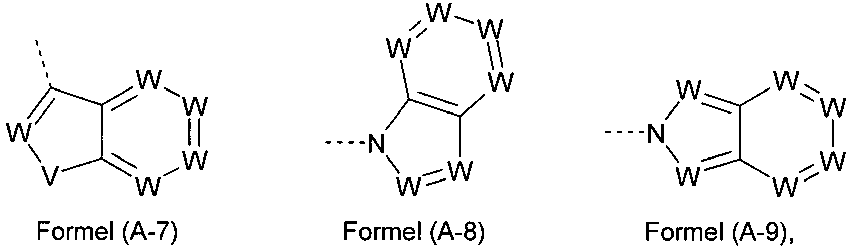 wobei die gestrichelte Bindung die Bindung an die Einheit gemäß Formel (II) darstellt, R¹ wie in Anspruch 1 definiert ist und
W bei jedem Auftreten gleich oder verschieden CR¹ oder N darstellt, und
V NR¹, O oder S darstellt, und
wobei mindestens eine Gruppe W gleich N ist.

9. Verbindung nach einem oder mehreren der Ansprüche 1 bis 8, **dadurch gekennzeichnet, dass** die Verbindung der Formel (I) bevorzugt eine Struktur gemäß einer der folgenden Formeln (I-1) bis (I-10) aufweist: 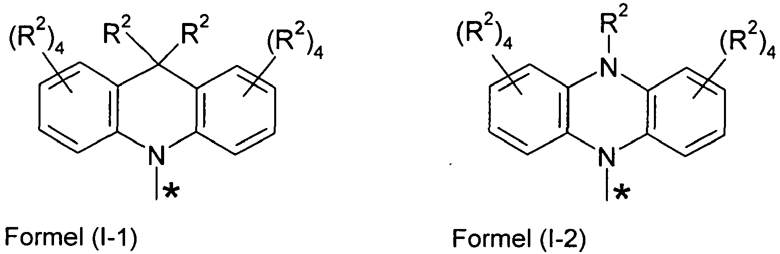 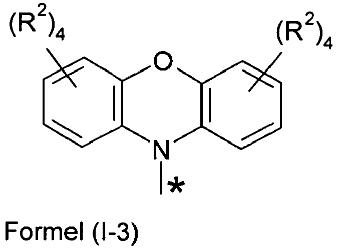 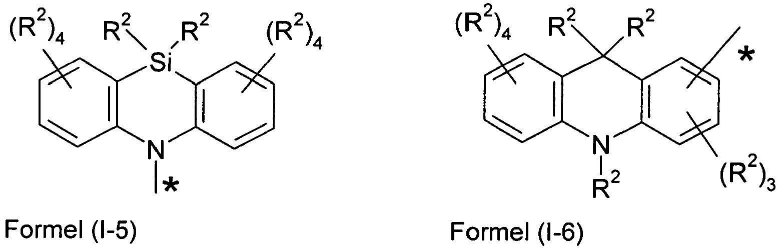 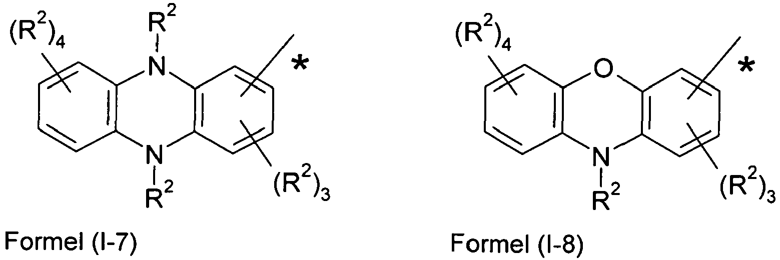 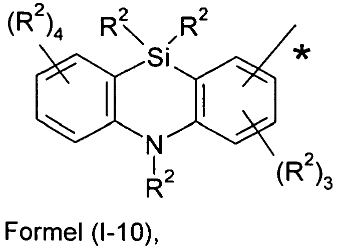 wobei über die mit * gekennzeichnete Bindung eine Gruppe der Formel (II) gebunden ist, und wobei weiterhin R² wie in Anspruch 1 definiert ist.

10. Oligomer, Polymer oder Dendrimer enthaltend eine oder mehrere Verbindungen nach einem oder mehreren der Ansprüche 1 bis 9, wobei die Bindung(en) zum Polymer, Oligomer oder Dendrimer an beliebigen, in Formel (I) mit R¹, R² oder R³ substituierten Positionen lokalisiert sein können.

11. Formulierung enthaltend mindestens eine Verbindung nach einem oder mehreren der Ansprüche 1 bis 9 oder mindestens ein Polymer, Oligomer oder Dendrimer nach Anspruch 10 sowie mindestens ein Lösungsmittel.

12. Verfahren zur Herstellung einer Verbindung nach einem oder mehreren der Ansprüche 1 bis 9, **dadurch gekennzeichnet, dass** mindestens eine Gruppe umfassend eine Struktur gemäß Formel (II) nach Anspruch 1 in einer metallkatalysierten Kupplungsreaktion eingeführt wird.

13. Elektronische Vorrichtung, enthaltend mindestens eine Verbindung nach einem oder mehreren der Ansprüche 1 bis 9 oder mindestens ein Polymer, Dendrimer oder Oligomer nach Anspruch 10, bevorzugt ausgewählt aus der Gruppe bestehend aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und organischen Elektrolumineszenzvorrichtungen (OLEDs).

14. Organische Elektrolumineszenzvorrichtung nach Anspruch 13, **dadurch gekennzeichnet, dass** die Verbindung nach einem oder mehreren der Ansprüche 1 bis 9 oder das Polymer, Dendrimer oder Oligomer nach Anspruch 10 als Matrixmaterial in einer emittierenden Schicht und/oder als Elektronentransportmaterial in einer Elektronentransport- oder Elektroneninjektionsschicht und/oder als Lochblockiermaterial in einer Lochblockierschicht vorhanden ist.

## Claims

1. Compound of a formula (I) 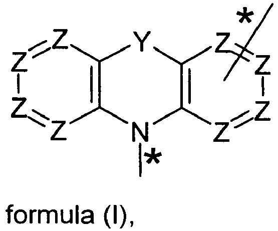 where a group of the formula (II) 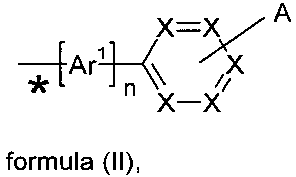 is bonded via at least one of the bonds denoted by *, where * in formula (II) in turn denotes the bond to the unit of the formula (I), and
where, in formula (I), for the case where no group of the formula (II) is bonded at the bond denoted by *, a radical R² is bonded there,
and where the following applies to the symbols and indices occurring:
A is on each occurrence, identically or differently,
- a heteroaryl group having 5 to 20 aromatic ring atoms, which may be substituted by one or more radicals R¹ and which contains at least one heteroaromatic five-membered ring having two or more heteroatoms selected from N, O and S or contains at least one hetero-aromatic six-membered ring having one or more hetero-atoms selected from N, O and S, or
- a keto group, which may be substituted by one or more radicals R¹, or
- a phosphorus oxide group, which may be substituted by one r or more radicals R¹, or
- a sulfur oxide group, which may be substituted by one or more radicals R¹;
Y is a divalent group selected from C(R²)₂, Si(R²)₂, NR² and O;
X is on each occurrence, identically or differently, CR¹ or N, or is C with group A bonded thereto;
Z is on each occurrence, identically or differently, CR² or N;
Ar¹ is an aromatic or heteroaromatic ring system having 5 to 30 aromatic ring atoms, which may be substituted by one or more radicals R¹;
R¹ is on each occurrence, identically or differently, H, D, F, Cl, Br, I, (OR³)₂, CHO, C(=O)R³, CR³=C(R³)₂, CN, C(=O)OR³, C(=O)N(R³)₂, Si(R³)₃, NO₂, P(=O)(R³)₂, OSO₂R³, OR³, S(=O)R³, S(=O)₂R³, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C=C-, Si(R³)₂, C=O, C=S, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I, CN or NO₂, or an aromatic ring system having 6 to 30 aromatic ring atoms, which may in each case be substituted by one or more radicals R³, or an aryloxy group having 6 to 30 aromatic ring atoms, which may be substituted by one or more radicals R³, where two or more radicals R¹ may be linked to one another and may form a ring;
R² is on each occurrence, identically or differently, H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C=C-, Si(R³)₂, C=O, C=S, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R³, or an aryloxy or heteroaryloxy group having 5 to 60 aromatic ring atoms, which may be substituted by one or more radicals R³, where two or more radicals R² may be linked to one another and may form a ring;
R³ is on each occurrence, identically or differently, H, D, F, Cl, Br, I, B(OR⁴)₂, CHO, C(=O)R⁴, CR⁴=C(R⁴)₂, CN, C(=O)OR⁴, C(=O)N(R⁴)₂, Si(R⁴)₃, N(R⁴)₂, NO₂, P(=O)(R⁴)₂, OSO₂R⁴, OR⁴, S(=O)R⁴, S(=O)₂R⁴, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R⁴ and where one or more CH₂ groups in the above-mentioned groups may be replaced by -R⁴C=CR⁴-, -C=C-, Si(R⁴)₂, C=O, C=S, C=Se, C=NR⁴, -C(=O)O-, -C(=O)NR⁴-, NR⁴, P(=O)(R⁴), -O-, -S-, SO or SO₂ and where one or more H atoms in the above-mentioned groups may be replaced by D, F, Cl, Br, I, CN or NO₂, or an aromatic or heteroaromatic ring system having 5 to 60 aromatic ring atoms, which may in each case be substituted by one or more radicals R⁴, or an aryloxy or heteroaryloxy group having 5 to 60 aromatic ring atoms, which may be substituted by one or more radicals R⁴, where two or more radicals R³ may be linked to one another and may form a ring;
R⁴ is on each occurrence, identically or differently, H, D, F or an aliphatic, aromatic or heteroaromatic organic radical having 1 to 20 C atoms, in which, in addition, one or more H atoms may be replaced by D or F; two or more substituents R⁴ here may be linked to one another and may form a ring;
n is on each occurrence, identically or differently, 0, 1, 2 or 3;
where radicals R² as constituents of groups Z cannot form any rings which are condensed onto the ring system of formula (I); and
where furthermore the group A is bonded to the aromatic six-membered ring in the meta or ortho position if the group of formula (II) is bonded to the nitrogen atom in formula (I);
and where furthermore the following compound is excluded:

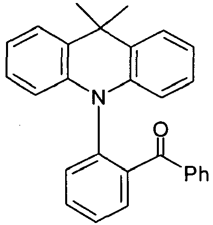

2. Compound according to Claim 1, **characterised in that** it contains no further amino group in addition to the amino group shown in formula (I).

3. Compound according to Claim 1 or 2, **characterised in that** the group Ar¹ represents an aromatic ring system having 6 to 18 aromatic ring atoms or a heteroaromatic ring system having 5 to 18 aromatic ring atoms, which may in each case be substituted by one or more radicals R¹.

4. Compound according to one or more of Claims 1 to 3, **characterised in that** not more than one group X per group of the formula (II) is equal to N.

5. Compound according to one or more of Claims 1 to 4, **characterised in that** not more than one group Z per aromatic six-membered ring is equal to N.

6. Compound according to one or more of Claims 1 to 5, **characterised in that** R¹ is selected on each occurrence, identically or differently, from H, D, F, CN, Si(R³)₃ or a straight-chain alkyl or alkoxy group having 1 to 10 C atoms or a branched or cyclic alkyl or alkoxy group having 3 to 10 C atoms, where the above-mentioned groups may each be substituted by one or more radicals R³ and where one or more CH₂ groups in the above-mentioned groups may be replaced by -C≡C-, -R³C=CR³-, Si(R³)₂, C=O, C=NR³, -NR³-, -O-, -S-, -C(=O)O- or -C(=O)NR³-, or an aromatic ring system having 6 to 24 aromatic ring atoms, which may be substituted by one or more radicals R³, where two or more radicals R¹ may be linked to one another and may form a ring.

7. Compound according to one or more of Claims 1 to 6, **characterised in that** the groups R¹ which are not bonded to a group A are equal to H.

8. Compound according to one or more of Claims 1 to 7, **characterised in that** the group A represents a group of the formula (A-1) to (A-9): 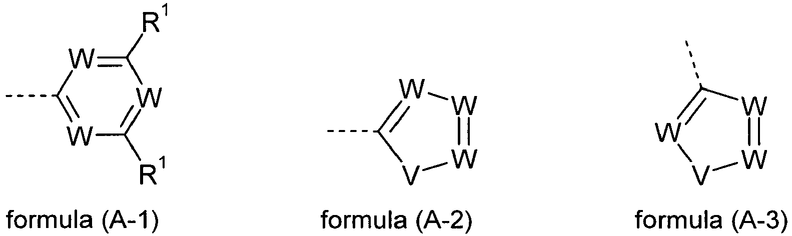 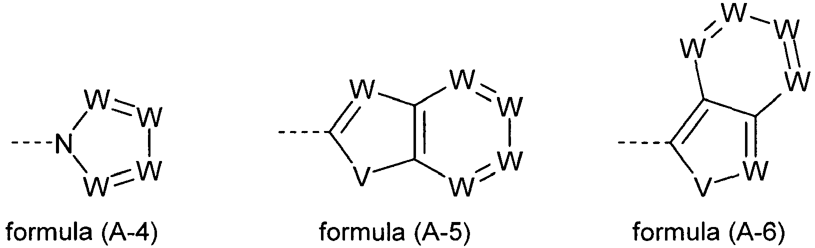 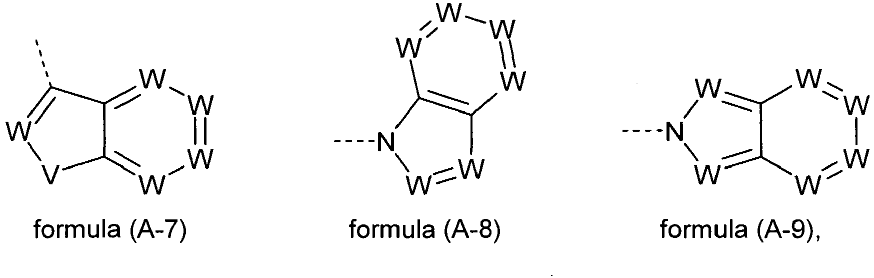 where the dashed bond represents the bond to the unit of the formula (II), R¹ is as defined in Claim 1 and
W represents on each occurrence, identically or differently, CR¹ or N, and
V represents NR¹, O or S, and
where at least one group W is equal to N.

9. Compound according to one or more of Claims 1 to 8, **characterised in that** the compound of the formula (I) preferably has a structure of one of the following formulae (I-1) to (I-10): 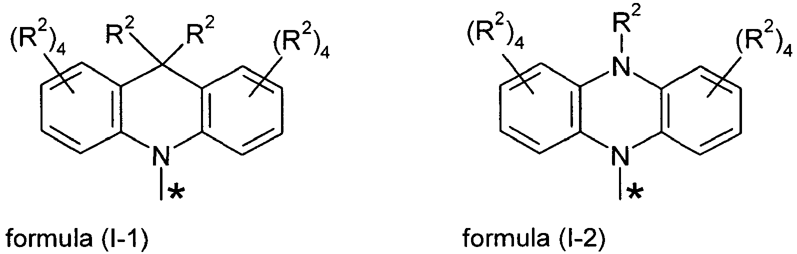 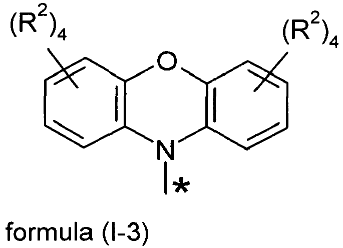 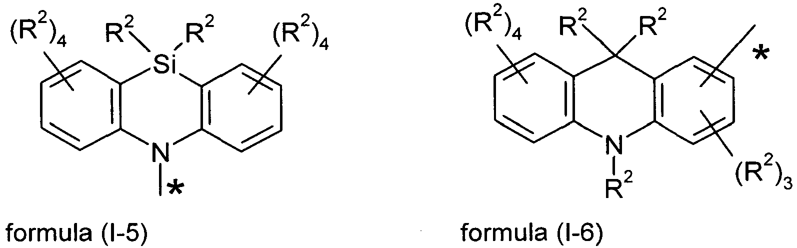 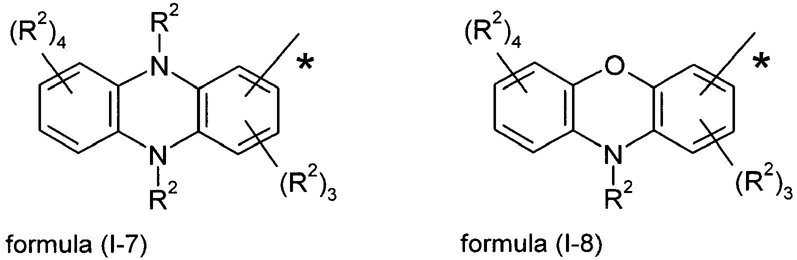 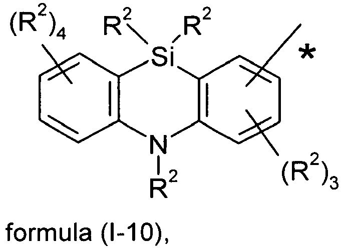 where a group of the formula (II) is bonded via the bond denoted by *, and where furthermore R² is as defined in Claim 1.

10. Oligomer, polymer or dendrimer containing one or more compounds according to one or more of Claims 1 to 9, where the bond(s) to the polymer, oligomer or dendrimer may be localised at any desired positions in formula (I) that are substituted by R¹, R² or R³.

11. Formulation comprising at least one compound according to one or more of Claims 1 to 9 or at least one polymer, oligomer or dendrimer according to Claim 10 and at least one solvent.

12. Process for the preparation of a compound according to one or more of Claims 1 to 9, **characterised in that** at least one group containing a structure of the formula (II) according to Claim 1 is introduced in a metal-catalysed coupling reaction.

13. Electronic device containing at least one compound according to one or more of Claims 1 to 9 or at least one polymer, dendrimer or oligomer according to Claim 10, preferably selected from the group consisting of organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and organic electroluminescent devices (OLEDs).

14. Organic electroluminescent device according to Claim 13, **characterised in that** the compound according to one or more of Claims 1 to 9 or the polymer, dendrimer or oligomer according to Claim 10 is present as matrix material in an emitting layer and/or as electron-transport material in an electron-transport or electron-injection layer and/or as hole-blocking material in a hole-blocking layer.

## Revendications

1. Composé de la formule (I) : 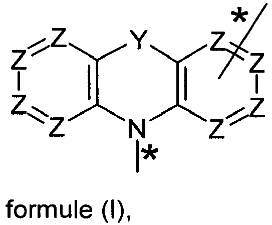 dans laquelle un groupe de la formule (II) : 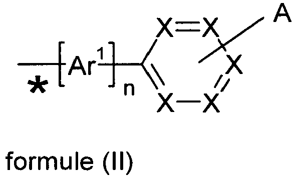 est lié via au moins l'une des liaisons qui sont représentées au moyen de *, où * dans la formule (II) représente à son tour la liaison sur l'unité de la formule (I), et
où, dans la formule (I), dans le cas dans lequel aucun groupe de la formule (II) n'est lié au niveau de la liaison qui est représentée au moyen de *, un radical R² est lié à ce niveau,
et où ce qui suit s'applique aux symboles et indices rencontrés :
A est, pour chaque occurrence, de manière identique ou différente,
- un groupe hétéroaryle qui comporte 5 à 20 atomes de cycle aromatique, lequel peut être substitué par un radical ou plusieurs radicaux R¹ et lequel contient au moins un cycle à cinq éléments hétéroaromatique qui comporte deux hétéroatomes ou plus qui sont sélectionnés parmi N, O et S ou lequel contient au moins un cycle à six éléments hétéroaromatique qui comporte un ou plusieurs hétéroatome(s) qui est/sont sélectionné(s) parmi N, O et S, ou
- un groupe céto, lequel peut être substitué par un radical ou plusieurs radicaux R¹, ou
- un groupe oxyde de phosphore, lequel peut être substitué par un radical ou plusieurs radicaux R¹, ou
- un groupe oxyde de soufre, lequel peut être substitué par un radical ou plusieurs radicaux R¹;
Y est un groupe divalent qui est sélectionné parmi C(R²)₂, Si(R²)₂, NR² et O ;
X est, pour chaque occurrence, de manière identique ou différente, CR¹ ou N, ou est C avec un groupe A qui lui est lié ;
Z est, pour chaque occurrence, de manière identique ou différente, CR² ou N ;
Ar¹ est un système de cycle aromatique ou hétéroaromatique qui comporte 5 à 30 atomes de cycle aromatique, lequel peut être substitué par un radical ou plusieurs radicaux R¹ ;
R¹ est, pour chaque occurrence, de manière identique ou différente, H, D, F, Cl, Br, I, B(OR³)₂, CHO, C(=O)R³, CR³=C(R³)₂, CN, C(=O)OR³, C(=O)N(R³)₂, Si(R³)₃, NO₂, P(=O)(R₃)₂, OSO₂R³, OR³, S(=O)R³, S(=O)₂R³, un groupe alkyle, alcoxy ou thioalkyle en chaîne droite qui comporte 1 à 20 atome(s) de C ou un groupe alkyle, alcoxy ou thioalkyle ramifié ou cyclique qui comporte 3 à 20 atomes de C ou un groupe alkényle ou alkynyle qui comporte 2 à 20 atomes de C, où les groupes qui ont été mentionnés ci-avant peuvent chacun être substitués par un radical ou plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=S, C=NR³, -C(=O)O-, , -C(=O)NR³-, NR³, P(=O)(R³), -O-, -S-, SO ou SO₂ et où un ou plusieurs atome(s) de H dans les groupes qui ont été mentionnés ci-avant peut/peuvent être remplacé(s) par D, F, CI, Br, I, CN ou NO₂, ou un système de cycle aromatique qui comporte 6 à 30 atomes de cycle aromatique, lequel peut, dans chaque cas, être substitué par un radical ou plusieurs radicaux R³, ou un groupe aryloxy qui comporte 6 à 30 atomes de cycle aromatique, lequel peut être substitué par un radical ou plusieurs radicaux R³, où deux radicaux R¹ ou plus peuvent être liés l'un à l'autre ou les uns aux autres et peuvent former un cycle ;
R² est, pour chaque occurrence, de manière identique ou différente, H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, un groupe alkyle, alcoxy ou thioalkyle en chaîne droite qui comporte 1 à 20 atome(s) de C ou un groupe alkyle, alcoxy ou thioalkyle ramifié ou cyclique qui comporte 3 à 20 atomes de C ou un groupe alkényle ou alkynyle qui comporte 2 à 20 atomes de C, où les groupes qui ont été mentionnés ci-avant peuvent chacun être substitués par un radical ou plusieurs radicaux R³ et où un ou plusieurs groupe(s) CH₂ dans les groupes qui ont été mentionnés ci-avant peut/peuvent être remplacé(s) par -R³C=CR³-, -C=C-, Si(R³)₂, C=O, C=S, C=NR³, -C(=O)O-, -C(=O)NR³-, NR³, P(=O)(R³), -O-, -S-, SO ou SO₂ et où un ou plusieurs atome(s) de H dans les groupes qui ont été mentionnés ci-avant peut/peuvent être remplacé(s) par D, F, Cl, Br, I, CN ou NO₂, ou un système de cycle aromatique ou hétéroaromatique qui comporte 5 à 30 atomes de cycle aromatique, lequel peut, dans chaque cas, être substitué par un radical ou plusieurs radicaux R³, ou un groupe aryloxy ou hétéroaryloxy qui comporte 5 à 60 atomes de cycle aromatique, lequel peut être substitué par un radical ou plusieurs radicaux R³, où deux radicaux R² ou plus peuvent être liés l'un à l'autre ou les uns aux autres et peuvent former un cycle ;
R³ est, pour chaque occurrence, de manière identique ou différente, H, D, F, Cl, Br, I, B(OR⁴)₂, CHO, C(=O)R⁴, CR⁴=C(R⁴)₂, CN, C(=O)OR⁴, C(=O)N(R⁴)₂, Si(R⁴)₃, N(R⁴)₂, NO₂, P(=O)(R⁴)₂, OSO₂R⁴, OR⁴, S(=O)R⁴, S(=O)₂R⁴, un groupe alkyle, alcoxy ou thioalkyle en chaîne droite qui comporte 1 à 20 atome(s) de C ou un groupe alkyle, alcoxy ou thioalkyle ramifié ou cyclique qui comporte 3 à 20 atomes de C ou un groupe alkényle ou alkynyle qui comporte 2 à 20 atomes de C, où les groupes qui ont été mentionnés ci-avant peuvent chacun être substitués par un radical ou plusieurs radicaux R⁴ et où un ou plusieurs groupe(s) CH₂ dans les groupes qui ont été mentionnés ci-avant peut/peuvent être remplacé(s) par -R⁴C=CR⁴-, -C=C-, Si(R⁴)₂, C=O, C=S, C=Se, C=NR⁴, -C(=O)O-, -C(=O)NR⁴-, NR⁴, P(=O)(R⁴), -O-, -S-, SO ou SO₂ et où un ou plusieurs atome(s) de H dans les groupes qui ont été mentionnés ci-avant peut/peuvent être remplacé(s) par D, F, Cl, Br, I, CN ou NO₂, ou un système de cycle aromatique ou hétéroaromatique qui comporte 5 à 60 atomes de cycle aromatique, lequel peut, dans chaque cas, être substitué par un radical ou plusieurs radicaux R⁴, ou un groupe aryloxy ou hétéroaryloxy qui comporte 5 à 60 atomes de cycle aromatique, lequel peut être substitué par un radical ou plusieurs radicaux R⁴, où deux radicaux R³ ou plus peuvent être liés l'un à l'autre ou les uns aux autres et peuvent former un cycle ;
R⁴ est, pour chaque occurrence, de manière identique ou différente, H, D, F ou un radical organique aliphatique, aromatique ou hétéroaromatique qui comporte 1 à 20 atome(s) de C, où, en outre, un ou plusieurs atome(s) de H peut/peuvent être remplacé(s) par D ou F ; deux substituants R⁴ ou plus peuvent ici être liés l'un à l'autre ou les uns aux autres et peuvent former un cycle ;
n est, pour chaque occurrence, de manière identique ou différente, 0, 1, 2 ou 3 ;
où des radicaux R² en tant que constituants de groupes Z ne peuvent pas former de quelconques cycles qui sont condensés sur le système de cycle de la formule (I) ; et
où, qui plus est, le groupe A est lié au cycle à six éléments aromatique à la position méta ou ortho si le groupe de la formule (II) est lié à l'atome d'azote dans la formule (I) ;
et où, qui plus est, le composé qui suit est exclus :

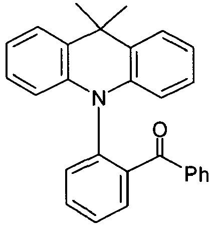

2. Composé selon la revendication 1, **caractérisé en ce qu'**il ne contient pas d'autre groupe amino en plus du groupe amino qui est représenté dans la formule (I).

3. Composé selon la revendication 1 ou 2, **caractérisé en ce que** le groupe Ar¹ représente un système de cycle aromatique qui comporte 6 à 18 atomes de cycle aromatique ou un système de cycle hétéroaromatique qui comporte 5 à 18 atomes de cycle aromatique, lequel peut, dans chaque cas, être substitué par un radical ou plusieurs radicaux R¹.

4. Composé selon une ou plusieurs des revendications 1 à 3, **caractérisé en ce que** pas plus d'un groupe X par groupe de la formule (II) est égal à N.

5. Composé selon une ou plusieurs des revendications 1 à 4, **caractérisé en ce que** pas plus d'un groupe Z par cycle à six éléments aromatique est égal à N.

6. Composé selon une ou plusieurs des revendications 1 à 5, **caractérisé en ce que** R¹ est sélectionné, pour chaque occurrence, de manière identique ou différente, parmi H, D, F, CN, Si(R³)₃ ou un groupe alkyle ou alcoxy en chaîne droite qui comporte 1 à 10 atome(s) de C ou un groupe alkyle ou alcoxy ramifié ou cyclique qui comporte 3 à 10 atomes de C, où les groupes qui ont été mentionnés ci-avant peuvent chacun être substitués par un radical ou plusieurs radicaux R³ et où un ou plusieurs groupe(s) CH₂ dans les groupes qui ont été mentionnés ci-avant peut/peuvent être remplacé(s) par -C≡C-, -R³C=CR³-, Si(R³)₂, C=O, C=NR³, -NR³-, -O-, -S-, -C(=O)O- ou -C(=O)NR³-, ou un système de cycle aromatique qui comporte 6 à 24 atomes de cycle aromatique, lequel peut être substitué par un radical ou plusieurs radicaux R³, où deux radicaux R¹ ou plus peuvent être liés l'un à l'autre ou les uns aux autres et peuvent former un cycle.

7. Composé selon une ou plusieurs des revendications 1 à 6, **caractérisé en ce que** les groupes R¹ qui ne sont pas liés à un groupe A sont égaux à H.

8. Composé selon une ou plusieurs des revendications 1 à 7, **caractérisé en ce que** le groupe A représente un groupe des formules (A-1) à (A-9) : 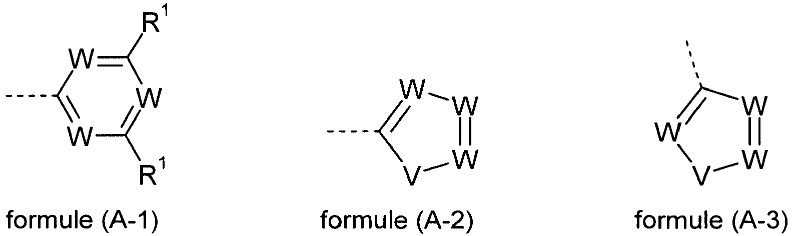 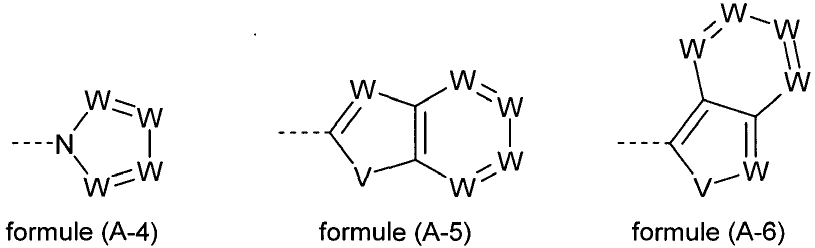 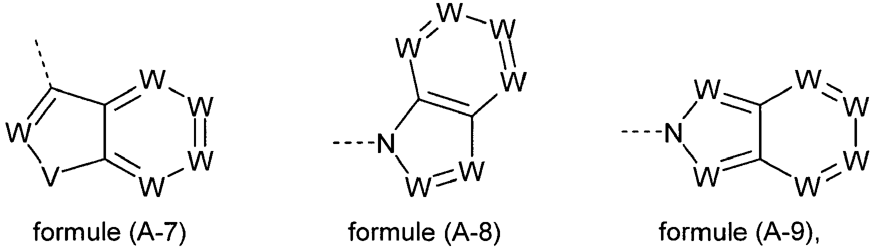 dans lesquelles la liaison en pointillés représente la liaison sur l'unité de la formule (II), R¹ est comme défini selon la revendication 1 et
W représente, pour chaque occurrence, de manière identique ou différente, CR¹ ou N, et
V représente NR¹, O ou S, et
où au moins un groupe W est égal à N.

9. Composé selon une ou plusieurs des revendications 1 à 8, **caractérisé en ce que** le composé de la formule (I) présente de façon préférable une structure de l'une des formules qui suivent (I-1) à (I-10) : 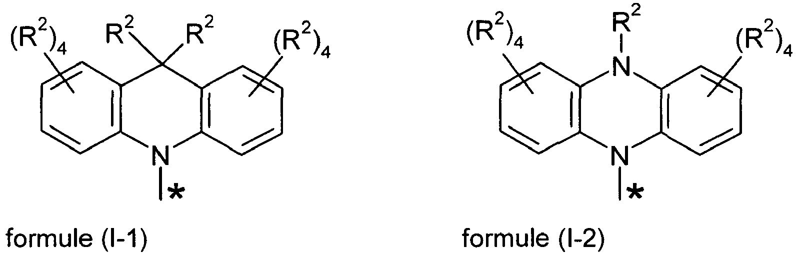 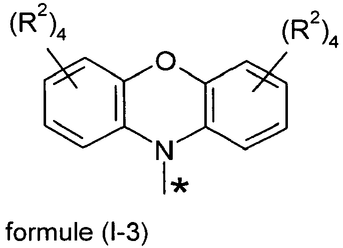 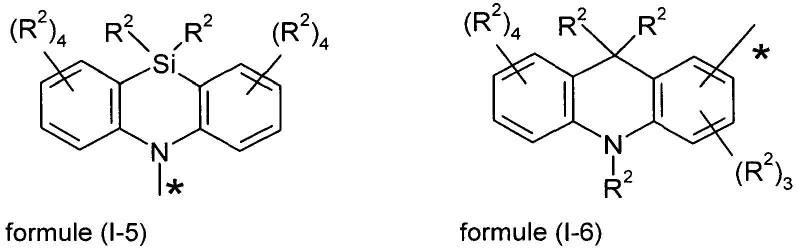 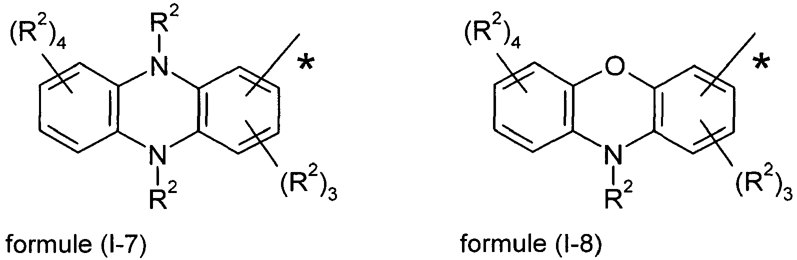 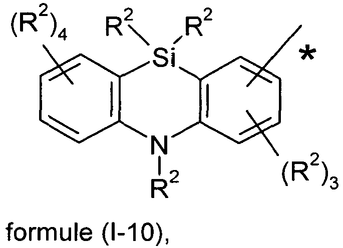 où un groupe de la formule (II) est lié via la liaison qui est représentée par *, et où, qui plus est, R² est comme défini selon la revendication 1.

10. Oligomère, polymère ou dendrimère contenant un ou plusieurs composé(s) selon une ou plusieurs des revendications 1 à 9, où la/les liaison(s) sur le polymère, l'oligomère ou le dendrimère peut/peuvent être localisée(s) au niveau de n'importe quelle(s) position(s) souhaitée(s) dans la formule (I) qui est/sont substituée(s) par R¹, R² ou R³.

11. Formulation comprenant au moins un composé selon une ou plusieurs des revendications 1 à 9 ou au moins un polymère, un oligomère ou un dendrimère selon la revendication 10 et au moins un solvant.

12. Procédé pour la préparation d'un composé selon une ou plusieurs des revendications 1 à 9, **caractérisé en ce qu'**au moins un groupe qui contient une structure de la formule (II) selon la revendication 1 est introduit dans une réaction de couplage catalysée par métal.

13. Dispositif électronique qui contient au moins un composé selon une ou plusieurs des revendications 1 à 9 ou au moins un polymère, un dendrimère ou un oligomère selon la revendication 10, de façon préférable sélectionné parmi le groupe qui est constitué par les circuits intégrés organiques (O-IC), les transistors à film mince organiques (O-FET), les transistors à effet de champ organiques (O-TFT), les transistors à émission de lumière organiques (O-LET), les cellules solaires organiques (O-SC), les détecteurs optiques organiques, les photorécepteurs organiques, les dispositifs à extinction de champ organiques (O-FQD), les cellules électrochimiques à émission de lumière (LEC), les diodes laser organiques (O-laser) et les dispositifs électroluminescents organiques (OLED).

14. Dispositif électroluminescent organique selon la revendication 13, **caractérisé en ce que** le composé selon une ou plusieurs des revendications 1 à 9 ou le polymère, le dendrimère ou l'oligomère selon la revendication 10 est présent en tant que matériau de matrice dans une couche d'émission et/ou en tant que matériau de transport d'électrons dans une couche de transport d'électrons ou d'injection d'électrons et/ou en tant que matériau de blocage de trous dans une couche de blocage de trous.
